(19) Europäisches Patentamt
European Patent Office
Office européen des brevets

(11) EP 1 189 634 B1

(12) **EUROPEAN PATENT SPECIFICATION**

(45) Date of publication and mention
of the grant of the patent:
**28.02.2007 Bulletin 2007/09**

(51) Int Cl.:
*A61K 39/395* (2006.01)   *A61P 35/00* (2006.01)

(21) Application number: **00939992.4**

(22) Date of filing: **23.06.2000**

(86) International application number:
**PCT/US2000/017423**

(87) International publication number:
**WO 2001/000238 (04.01.2001 Gazette 2001/01)**

(54) **TREATING PROSTATE CANCER WITH ANTI-ERBB2 ANTIBODIES**

BEHANDLUNG VON PROSTATA-KREBS MIT ANTI-ERBB2 ANTIKÖRPERN

TRAITEMENT DU CANCER DE LA PROSTATE A L'AIDE DES ANTICORPS ANTI-ERBB2

(84) Designated Contracting States:
**AT BE CH CY DE DK ES FI FR GB GR IE IT LI LU MC NL PT SE**

(30) Priority: **25.06.1999 US 141315 P**

(43) Date of publication of application:
**27.03.2002 Bulletin 2002/13**

(73) Proprietor: **GENENTECH, INC.**
**South San Francisco, CA 94080-4990 (US)**

(72) Inventor: **SLIWKOWSKI, Mark, X.**
**San Carlos, CA (US)**

(74) Representative: **Walton, Seán Malcolm et al**
**Mewburn Ellis LLP**
**York House,**
**23 Kingsway**
**London WC2B 6HP (GB)**

(56) References cited:
**WO-A-99/31140**

• MYERS, R. B. (1) ET AL: "Intracellular antibody mediated down-regulation of p185-erbB-2 expression in malignant prostatic cells." PROCEEDINGS OF THE AMERICAN ASSOCIATION FOR CANCER RESEARCH ANNUAL MEETING, (1996) VOL. 37, NO. 0, PP. 342. MEETING INFO.: 87TH ANNUAL MEETING OF THE AMERICAN ASSOCIATION FOR CANCER RESEARCH WASHINGTON, D.C., USA APRIL 20-24, 1996 , XP000918959

• SKREPNIK N ET AL: "Recombinant oncotoxin AR209 (anti-P185erbB-2) diminishes human prostate carcinoma xenografts." JOURNAL OF UROLOGY, (1999 MAR) 161 (3) 984-9. , XP000940699

• JAMES, N. (1) ET AL: "Phase II trial of the bispecific antibody MDX-H210 (anti-Her2/Neu X anti-CD64) combined with GM-CSF in patients with advanced prostate and renal cell carcinoma that express Her2/neu." BRITISH JOURNAL OF CANCER, (1998) VOL. 78, NO. SUPPL. 2, PP. 19. MEETING INFO.: JOINT MEETING OF THE BRITISH ONCOLOGICAL ASSOCIATION, THE ASSOCIATION OF CANCER PHYSICIANS AND THE ROYAL COLLEGE OF RADIOLOGISTS NOTTINGHAM, ENGLAND, UK JULY 5-7, 1998 AS, XP000940750

• AGUS D B ET AL: "RESPONSE OF PROSTATE CANCER TO ANTI-HER-2/NEU ANTIBODY IN ANDROGEN DEPENDENT AND -INDEPENDENT HUMAN XENOGRAFT MODELS" CANCER RESEARCH, AMERICAN ASSOCIATION FOR CANCER RESEARCH, BALTIMORE, MD, US, vol. 59, 1 October 1999 (1999-10-01), pages 4761-4764, XP000919490 ISSN: 0008-5472

• AGUS, DAVID B. (1) ET AL: "Differential anti-tumor effects of targeting distinct epitopes of the Her-2/neu extracellular domain in xenograft models of prostate cancer." PROCEEDINGS OF THE AMERICAN ASSOCIATION FOR CANCER RESEARCH ANNUAL MEETING, (MARCH, 2000) NO. 41, PP. 719. PRINT.. MEETING INFO.: 91ST ANNUAL MEETING OF THE AMERICAN ASSOCIATION FOR CANCER RESEARCH. SAN FRANCISCO, CALIFORNIA, USA APRIL 01-05, 2000 , XP000919491

- **Curnow R.T.: (1997) Cancer Immunol. Immunother. 45:210-215**

Remarks:

The file contains technical information submitted after the application was filed and not included in this specification

Remarks:

## Description

### Field of the Invention

The present invention concerns the treatment of prostate cancer with anti-ErbB2 antibodies.

### Background of the Invention

The ErbB family of receptor tyrosine kinases are important mediators of cell growth, differentiation and survival. The receptor family includes four distinct members including epidermal growth factor receptor (EGFR or ErbB 1), HER2 (ErbB2 or p185$^{neu}$), HER3 (ErbB3) and HER4 (ErbB4 or tyro2).

EGFR, encoded by the erbB1 gene, has been causally implicated in human malignancy. In particular, increased expression of EGFR has been observed in breast, bladder, lung, head, neck and stomach cancer as well as glioblastomas. Increased EGFR receptor expression is often associated with increased production of the EGFR ligand, transforming growth factor alpha (TGF-$\alpha$), by the same tumor cells resulting in receptor activation by an autocrine stimulatory pathway. Baselga and Mendelsohn *Pharmac. Ther.* 64:127-154 (1994). Monoclonal antibodies directed against the EGFR or its ligands, TGF-$\alpha$ and EGF, have been evaluated as therapeutic agents in the treatment of such malignancies. See, *e.g.,* Baselga and Mendelsohn., *supra;* Masui *et al. Cancer Research* 44:1002-1007 (1984); and Wu *et al. J. Clin. Invest.* 95:1897-1905 (1995).

The second member of the ErbB family, p185$^{neu}$, was originally identified as the product of the transforming gene from neuroblastomas of chemically treated rats. The activated form of the *neu* proto-oncogene results from a point mutation (valine to glutamic acid) in the transmembrane region of the encoded protein. Amplification of the human homolog of *neu* is observed in breast and ovarian cancers and correlates with a poor prognosis (Slamon *et al., Science,* 235:177-182 (1987); Slamon *et al., Science,* 244:707-712 (1989); and US Pat No. 4,968,603). To date, no point mutation analogous to that in the *neu* proto-oncogene has been reported for human tumors. Overexpression of ErbB2 (frequently but not uniformly due to gene amplification) has also been observed in other carcinomas including carcinomas of the stomach, endometrium, salivary gland, lung, kidney, colon, thyroid, pancreas and bladder. See, among others, King *et al., Science,* 229:974 (1985); Yokota *et al., Lancet:* 1:765-767 (1986); Fukushigi *et al., Mol Cell Biol.,* 6:955-958 (1986); Geurin *et al., Oncogene Res.,* 3:21-31 (1988); Cohen *et al., Oncogene,* 4:81-88 (1989); Yonemura *et al., Cancer Res.,* 51:1034 (1991); Borst *et al., Gynecol. Oncol.,* 38:364 (1990); Weiner *et al., Cancer Res.,* 50:421-425 (1990); Kern *et al., Cancer Res.,* 50:5184 (1990); Park *et al., Cancer Res.,* 49:6605 (1989); Zhau *et al., Mol. Carcinog.,* 3:354-357 (1990); Aasland *et al. Br. J. Cancer* 57:358-363 (1988); Williams *et al. Pathiobiology* 59:46-52 (1991); and McCann *et al., Cancer,* 65:88-92 (1990). ErbB2 may be overexpressed in prostate cancer (Gu *et al. Cancer Lett.* 99:185-189 (1996); Ross *et al. Hum. Pathol.* 28:827-833 (1997); Ross *et al. Cancer* 79:2162-2170 (1997); and Sadasivan *et al. J. Urol.* 150:126-131 (1993)). Antibodies directed against the rat p185$^{neu}$ and human ErbB2 protein products have been described. Drebin and colleagues have raised antibodies against the rat *neu* gene product, p185$^{neu}$. See, for example. Drebin *et al., Cell* 41:695-706 (1985); Myers *et al., Meth. Enzym.* 198:277-290 (1991); and WO94/22478. Drebin *et al. Oncogene* 2:273-277 (1988) report that mixtures of antibodies reactive with two distinct regions of p185$^{neu}$ result in synergistic anti-tumor effects on *neu*-transformed NIH-3T3 cells implanted into nude mice. See also U.S. Patent 5,824,311 issued October 20, 1998.

Hudziak *et al., Mol. Cell. Biol.* 9(3):1165-1172 (1989) describe the generation of a panel of anti-ErbB2 antibodies which were characterized using the human breast tumor cell line SKBR3. Relative cell proliferation of the SKBR3 cells following exposure to the antibodies was determined by crystal violet staining of the monolayers after 72 hours. Using this assay, maximum inhibition was obtained with the antibody called 4D5 which inhibited cellular proliferation by 56%. Other antibodies in the panel reduced cellular proliferation to a lesser extent in this assay. The antibody 4D5 was further found to sensitize ErbB2-overexpressing breast tumor cell lines to the cytotoxic effects of TNF-$\alpha$. See also U.S. Patent No. 5,677,171 issued October 14. 1997. The anti-ErbB2 antibodies discussed in Hudziak *et al.* are further characterized in Fendly *et al. Cancer Research* 50:1550-1558 (1990): Kous *et al, In Vitro* 26(3):59A (1990); Sarup *et al. Growth Regulation* 1:72-82 (1991); Shepard *et al. J. Clin. Immunol.* 11(3):117-127 (1991); Kumar *et al. Mol. Cell. Biol.* 11(2): 979-986 (1991); Lewis *et al. Cancer Immunol. Immunother.* 37:255-263 (1993): Pietras *et al. Oncogene* 9:1829-1838 (1994); Vitetta *et al. Cancer Research* 54:5301-5309 (1994); Sliwkowski *et al. J. Biol. Chem.* 269(20):14661-14665 (1994); Scott *et al. J. Biol. Chem.* 266:14300-5 (1991); D'souza *et al. Proc. Natl. Acad Sci.* 91:7202-7206 (1994): Lewis *et al. Cancer Research* 56:1457-1465 (1996): and Schaefer *et al. Oncogene* 15:1385-1394 (1997).

A recombinant humanized version of the murine anti-ErbB2 antibody 4D5 (huMAb4D5-8, rhuMAb HER2 or HERCEPTIN®; U.S. Patent No. 5,821,337) is clinically active in patients with ErbB2-overexpressing metastatic breast cancers that have received extensive prior anti-cancer therapy (Baselga *et al., J. Clin. Oncol.* 14:737-744 (1996)). HERCEPTIN® received marketing approval from the Food and Drug Administration September 25, 1998 for the treatment of patients with metastatic breast cancer whose tumors overexpress the ErbB2 protein.

WO 99/31140 concerns the treatment of disorders characterised by the overexpression of ErbB2. More specifically, the treatment of human patients susceptible to or diagnosed with cancer overexpressing ErbB2 with a combination of an anti-ErbB2 antibody and a chemotherapeutic agent other than an anthracycline, *e.g.* doxorubicin or epirubicin.

Other anti-ErbB2 antibodies with various properties have been described in Tagliabue *et al. Int. J. Cancer* 47: 933-937 (1991); MeKenzie *et al. Oncogene* 4:543-548 (1989); Maier *et al. Cancer Res.* 51:5361-5369 (1991); Bacus *et al. Molecular Carcinogenesis* 3:350-362 (1990): Stancovski *et al. PNAS (USA)* 88:8691-8695 (1991); Bacus *et al. Cancer Research* 52:2580-2589 (1992); Xu *et al. Int. J. Cancer* 53:401-408 (1993); WO94/00136; Kasprzyk *et al. Cancer Research* 52:2771-2776 (1992);Hancock *et al. Cancer Res.* 51 :4575-4580 (1991); Shawver *et al. Cancer Res.* 54: 1367-1373 (1994); Arteaga *et al. Cancer Res.* 54:3758-3765 (1994); Harwerth *et al. J. Biol. Chem.* 267:15160-15167 (1992): U.S. Patent No. 5.783.186: and Klapper *et al. Oncogene* 14:2099-2109 (1997).

An intracellular scFv antibody directed against ErbB2 has been used to down-regulate the membrane expression of this receptor in the cell lines DU145, LNCaP and PC3 (Myers *et al. Proceedings of the American Association for Cancer Research Annual Meeting,* 37(0): 342, #2334 (1996)).

In Skrepnik *et al J. Urology* 161: 984-989 (1999) subcutaneous xenographs in mice, grown from cell lines DU145 and PC3, were treated using an oncotoxin. The oncotoxin comprised an anti-erbB2 antibody contained within a single-chain antibody domain coupled to a portion of the Pseudomonas exotoxin A, which inhibited protein synthesis in target cells.

A bispecific antibody (MDX-H210) comprising a recognition site for ErbB2 in combination with GM-CSF has been used in a phase II clinical trial for the treatment of prostate cancer (James *et al. Brit J. Cancer* 78(2): 19, 056 (1998)).

Homology screening has resulted in the identification of two other ErbB receptor family members; ErbB3 (US Pat. Nos. 5,183,884 and 5.480.968 as well as Kraus *et al. PNAS (USA)* 86:9193-9197 (1989)) and ErbB4 (EP Pat Appln No 599.274; Plowman *et al., Proc. Natl. Acad. Sci. USA.* 90:1746-1750 (1993); and Plowman *et al., Nature.* 366:473-475 (1993)). Both of these receptors display increased expression on at least some breast cancer cell lines.

The ErbB receptors are generally found in various combinations in cells and heterodimerization is thought to increase the diversity of cellular responses to a variety of ErbB ligands (Earp *et al. Breast Cancer Research and Treatment* 35: 115-132 (1995)). EGFR is bound by six different ligands; epidermal growth factor (EGF), transforming growth factor alpha (TGF-$\alpha$), amphiregulin, heparin binding epidermal growth factor (HB-EGF), betacellulin and epiregulin (Groenen *et al. Growth Factors* 11:235-257 (1994)). A family of heregulin proteins resulting from alternative splicing of a single gene are ligands for ErbB3 and ErbB4. The heregulin family includes alpha, beta and gamma heregulins (Holmes *et al., Science,* 256:1205-1210 (1992); U.S. Patent No. 5,641,869; and Schaefer *et al. Oncogene* 15:1385-1394 (1997)); neu differentiation factors (NDFs), glial growth factors (GGFs); acetylcholine receptor inducing activity (ARIA); and sensory and motor neuron derived factor (SMDF). For a review, see Groenen *et al. Growth Factors* 11:235-257 (1994); Lemke, G. *Molec. & Cell. Neurosci.* 7:247-262 (1996) and Lee *et al. Pharm. Rev.* 47:51-85 (1995). Recently three additional ErbB ligands were identified; neuregulin-2 (NRG-2) which is reported to bind either ErbB3 or ErbB4 (Chang *et al. Nature* 387 509-512 (1997); arid Carraway *et al Nature* 387:512-516 (1997)); neuregulin-3 which binds ErbB4 (Zhang *et al. PNAS* (USA) 94(18):9562-7 (1997)); and neuregulin-4 which binds ErbB4 (Harari *et al. Oncogene* 18:2681-2689 (1999)) HB-EGF, betacellulin and epiregulin also bind to ErbB4.

While EGF and TGF$\alpha$ do not bind ErbB2. EGF stimulates EGFR and ErbB2 to form a heterodimer, which activates EGFR and results in transphosphorylation of ErbB2 in the heterodimer. Dimerization and/or transphosphorylation appears to activate the ErbB2 tyrosine kinase. See Earp *et al., supra.* Likewise, when ErbB3 is co-expressed with ErbB2, an active signaling complex is formed and antibodies directed against ErbB2 are capable of disrupting this complex (Sliwkowski *et al., J. Biol. Chem.,* 269(20):14661-14665 (1994)). Additionally, the affinity of ErbB3 for heregulin (HRG) is increased to a higher affinity state when co-expressed with ErbB2. See also, Levi *et al., Journal of Neuroscience* 15: 1329-1340 (1995); Morrissey *et al., Proc. Natl. Acad. Sci. USA* 92: 1431-1435 (1995); and Lewis *et al., Cancer Res.,* 56:1457-1465 (1996) with respect to the ErbB2-ErbB3 protein complex. ErbB4, like ErbB3, forms an active signaling complex with ErbB2 (Carraway and Cantley, *Cell* 78:5-8 (1994)).

Summary of the Invention

In a first aspect, the present invention provides use of an antibody which binds ErbB2 and blocks ligand activation of an ErbB receptor 50-100% more effectively than humanized monoclonal antibody huMAb4D5-8 as disclosed in US patent 5,821,337 for the manufacture of a medicament for treating prostate cancer (e.g. androgen independent prostate cancer) in a human. The antibody blocks binding of monoclonal antibody 2C4 to ErbB2 and preferably blocks TGF-$\alpha$ activation of mitogen-activated protein kinase (MAPK).

The invention further provides use of a chemotherapeutic agent (e.g. a taxane) and of an antibody which binds ErbB2 and blocks ligand activation of an ErbB receptor 50-100% more effectively than humanized monoclonal antibody huMAb4D5-8 as disclosed in US patent 5,821,337 for the manufacture of a medicament for treating prostate cancer in a human. The antibody blocks binding of monoclonal antibody 2C4 obtainable from deposit ATCC HB 12697 to ErbB2.

Administration of a therapeutically effective amount of an antibody which binds ErbB2 can be used to treat androgen dependent prostate cancer in a human, and optionally results in an increased prostate specific antigen (PSA) index in the human. The antibody is one, like monoclonal antibody 2C4 (e.g. humanised 2C4), which blocks ligand activation of an ErbB2 receptor. A chemotherapeutic agent, preferably a taxane, may further be administered to the human.

The invention, in a further aspect, provides an article of manufacture comprising a container and a composition contained therein, wherein the composition comprises an antibody which binds ErbB2 and blocks ligand activation of an ErbB receptor 50-100% more effectively than humanized monoclonal antibody huMAb4D5-8 as disclosed in US patent 5,821,337, and further comprising a package insert indicating that the composition can be used to treat prostate cancer, e.g. androgen dependent prostate cancer, wherein the antibody blocks binding of monoclonal antibody 2C4 obtainable from deposit ATCC HB 12697 to ErbB2. The package insert optionally further indicates treating the patient with a chemotherapeutic agent, such as taxane.

## Brief Description of the Drawings

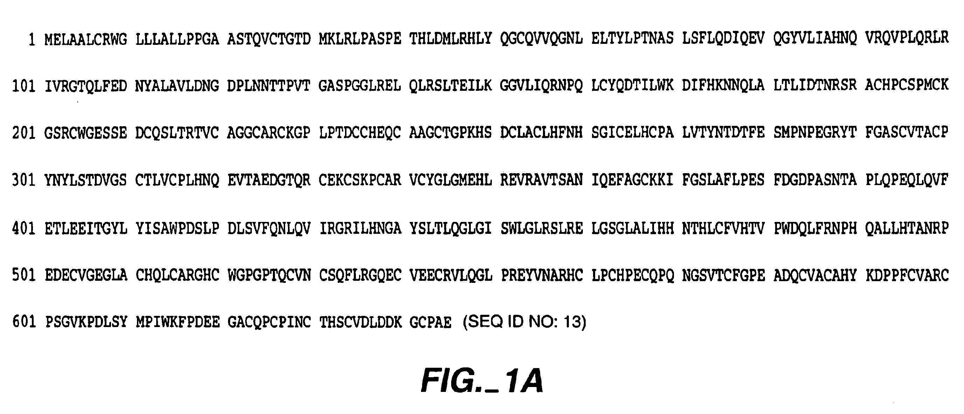

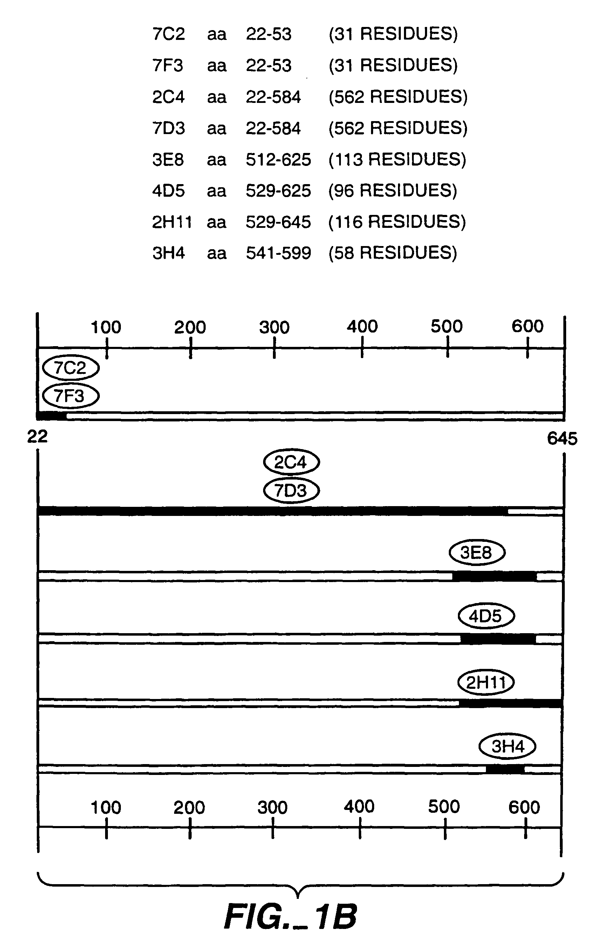

Figures 1A and 1B depict epitope mapping of residues 22-645 within the extracellular domain (ECD) of ErbB2 (amino acid sequence, including signal sequence, shown in Fig. 1A; SEQ ID NO:13) as determined by truncation mutant analysis and site-directed mutagenesis (Nakamura *et al. J. of Virology* 67(10):6179-6191 (1993): and Renz *et al. J. Cell Biol.* 125(6):1395-1406 (1994)). The various ErbB2-ECD truncations or point mutations were prepared from cDNA using polymerase chain reaction technology. The ErbB2 mutants were expressed as gD fusion proteins in a mammalian expression plasmid. This expression plasmid uses the cytomegalovirus promoter/enhancer with SV40 termination and polyadenylation signals located downstream of the inserted cDNA. Plasmid DNA was transfected into 293 cells. One day following transfection, the cells were metabolically labeled overnight in methionine and cysteine-free, low glucose DMEM containing 1% dialyzed fetal bovine serum and 25 $\mu$Ci each of $^{35}$S methionine and $^{35}$S cysteine. Supernatants were harvested and either the anti-ErbB2 monoclonal antibodies or control antibodies were added to the supernatant and incubated 2-4 hours at 4°C. The complexes were precipitated, applied to a 10-20% Tricine SDS gradient gel and electrophoresed at 100 V. The gel was electroblotted onto a membrane and analyzed by autoradiography. As shown in Fig. 1B, the anti-ErbB2 antibodies 7C2. 7F3. 2C4, 7D3, 3E8, 4D5, 2H11 and 3H4 bind various ErbB2 ECD epitopes.

Figures 2A and 2B show the effect of anti-ErbB2 monoclonal antibodies 2C4 and 7F3 on rHRGβ1 activation of MCF7 cells. Fig. 2A shows dose-response curves for 2C4 or 7F3 inhibition of HRG stimulation of tyrosine phosphorylation. Fig. 2B shows dose-response curves for the inhibition of $^{125}$I-labeled rHRGβ1$_{177-244}$ binding to MCF-7 cells by 2C4 or 7F3.

Figure 3 depicts inhibition of specific $^{125}$I-labeled rHRGβ1$_{177-244}$ binding to a panel of human tumor cell lines by the anti-ErbB2 monoclonal antibodies 2C4 or 7F3. Monoclonal antibody-controls are isotype-matched murine monoclonal antibodies that do not block rHRG binding. Nonspecific $^{125}$I-labeled rHRGβ1$_{177-244}$ binding was determined from parallel incubations performed in the presence of 100 nM rHRGβ1. Values for nonspecific $^{125}$I-labeled rHRGβ1$_{177-244}$ binding were less than 1% of the total for all the cell lines tested.

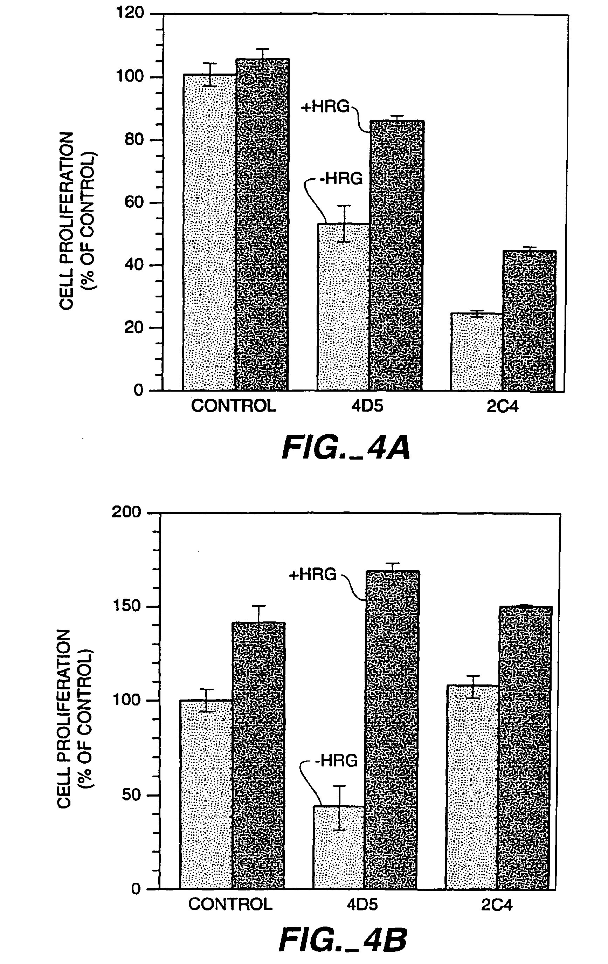

Figures 4A and 4B show the effect of monoclonal antibodies 2C4 and 4D5 on proliferation of MDA-MB-175 (Fig. 4A) and SK-BR-3 (Fig. 4B) cells. MDA-MB-175 and SK-BR-3 cells were seeded in 96 well plates and allowed to adhere for 2 hours. Experiment was carried out in medium containing 1% serum. Anti-ErbB2 antibodies or medium alone were added and the cells were incubated for 2 hours at 37°C. Subsequently rHRGβ1 (1nM) or medium alone were added and the cells were incubated for 4 days. Monolayers were washed and stained/fixed with 0.5% crystal violet. To determine cell proliferation the absorbance was measured at 540 nm.

Figures 5A and 5B show the effect of monoclonal antibody 2C4, HERCEPTIN® antibody or an anti-EGFR antibody on heregulin (HRG) dependent association of ErbB2 with ErbB3 in MCF7 cells expressing low/normal levels of ErbB2 (Fig. 5A) and SK-BR-3 cells expressing high levels of ErbB2 (Fig. 5B); see Example 2 below.

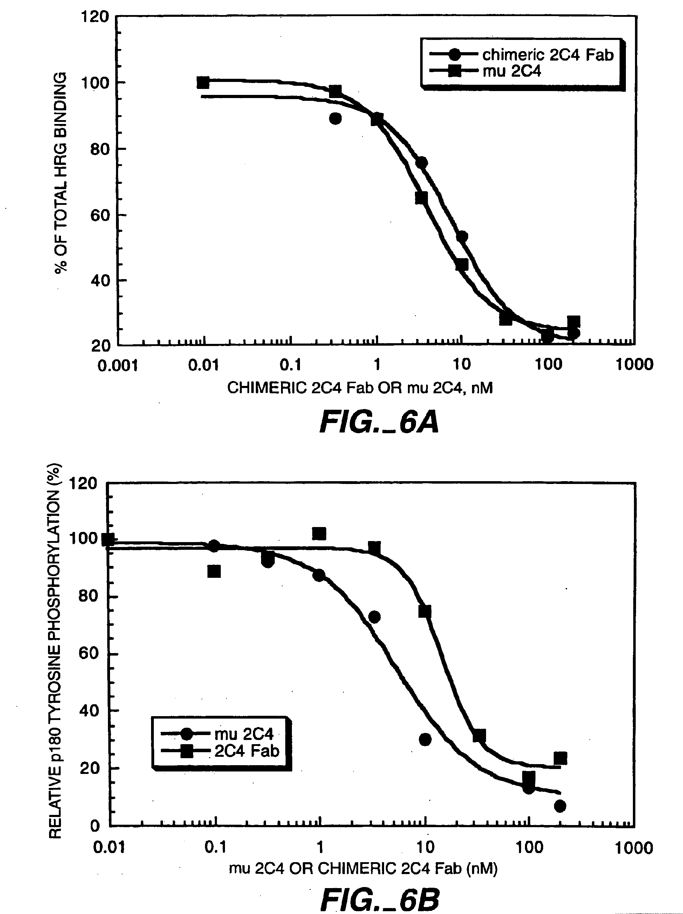

Figures 6A and 6B compare the activities of intact murine monoclonal antibody 2C4 (mu 2C4) and a chimeric 2C4 Fab fragment. Fig. 6A shows inhibition of $^{125}$I-HRG binding to MCF-7 cells by chimeric 2C4 Fab or intact murine monoclonal antibody 2C4. MCF7 cells were seeded in 24-well plates (1 x 10$^5$ cells/well) and grown to about 85% confluency for two days. Binding experiments were conducted as described in Lewis *et al. Cancer Research* 56: 1457-1465 (1996). Fig. 6B depicts inhibition of rHRGβ1 activation of p180 tyrosine phosphorylation in MCF-7 cells performed as described in Lewis *et al. Cancer Research* 56:1457-1465 (1996).

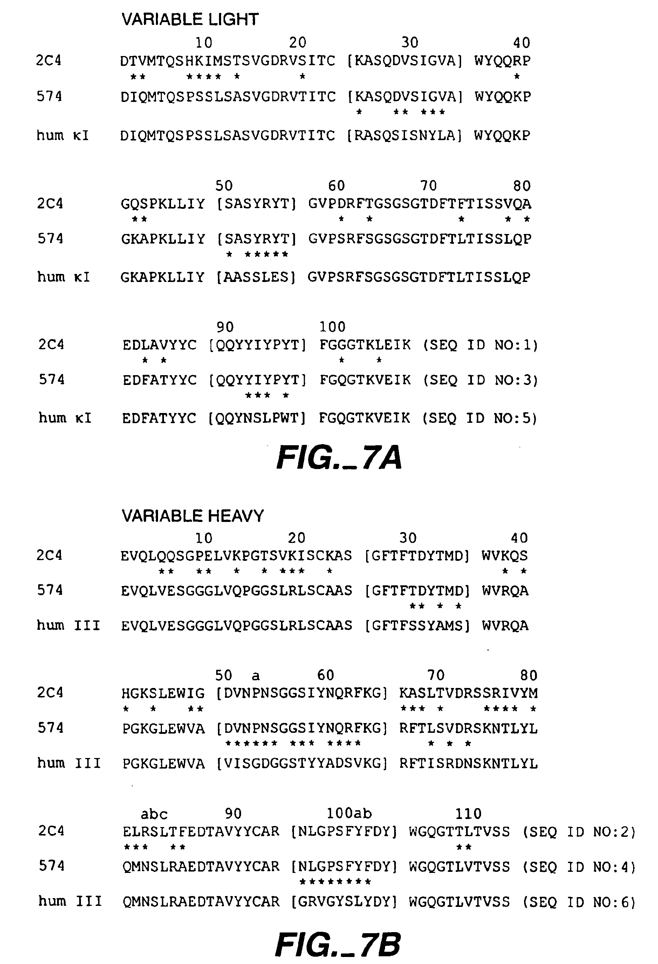

Figures 7A and 7B depict alignments of the amino acid sequences of the variable light (V$_L$) (Fig. 7A) and variable heavy (V$_H$) (Fig. 7B) domains of murine monoclonal antibody 2C4 (SEQ ID Nos. 1 and 2, respectively); V$_L$ and V$_H$ domains of humanized Fab version 574 (SEQ ID Nos. 3 and 4, respectively), and human V$_L$ and V$_H$ consensus frameworks (hum κ1, light kappa subgroup I; humIII, heavy subgroup III) (SEQ ID Nos. 5 and 6, respectively). Asterisks identify differences between humanized Fab version 574 and murine monoclonal antibody 2C4 or between humanized Fab version 574 and the human framework. Complementarity Determining Regions (CDRs) are in brackets.

Figures 8A to C show binding of chimeric Fab 2C4 (Fab.v 1) and several humanized 2C4 variants to ErbB2 extracellular domain (ECD) as determined by ELISA in Example 3.

Figure 9 is a ribbon diagram of the $V_L$ and $V_H$ domains of monoclonal antibody 2C4 with white CDR backbone labeled (L1, L2, L3, H1, H2, H3). $V_H$ side chains evaluated by mutagenesis during humanization (see Example 3, Table 2) are also shown.

Figure 10 depicts the effect of monoclonal antibody 2C4 or HERCEPTIN® on EGF, TGF-α, or HRG-mediated activation of mitogen-activated protein kinase (MAPK).

Figures 11A to H depict response of xenograft tumors to HERCEPTIN® (H,■), control (C, o), TAXOL® (T,▲) and combination HERCEPTIN®/TAXOL® (H/T, ◇) treatment. The response of the androgen independent tumors CWR22R and CWRSA6 (Figs. 11A and B, respectively) and the androgen dependent tumors CWR22 and LNCaP (Figs. 11C and D, respectively) to HERCEPTIN® and control are shown. The response of the tumors to HERCEPTIN®, TAXOL®, HERCEPTIN®fTAXOL® and control are shown in Fig. 11E (CWR22); Fig. 11F (LNCaP); Fig. 11G (CWR22R); and Fig. 11H (CWRSA6). Results are given as mean tumor volume +/- SE.

Figures 12A and 12B depict relative prostate specific antigen (PSA) index response of animals with androgen dependent prostate cancer xenografts treated with HERCEPTIN®. In Fig. 12A, PSA index was measured in the LNCaP xenograft model prior to treatment and at days 9 and 21 after initiating treatment and expressed as relative to pretreatment values. In Fig. 12B, PSA index was measured in the CWR22 xenograft model prior to treatment and at days 9 and 21 after initiating treatment and expressed as relative to pretreatment values. Results are given as mean relative PSA +/- SE.

Figure 13 depicts response of the androgen dependent tumor CWR22 to therapy with control antibody (C, ▲), HERCEPTIN® (H, o) or monoclonal antibody 2C4 (2, ■). Administration of 2C4 designated by *; administration of HERCEPTIN® designated by +.

Figure 14 depicts response of the androgen dependent tumor CWR22 to therapy with TAXOL® alone (T, o), monoclonal antibody 2C4 alone (2, ■) or a combination of monoclonal antibody 2C4 and TAXOL® (2/T, ▲). Administration of 2C4 designated by *; administration of TAXOL® (6.25 mg/kg) designated by +.

Figure 15 depicts response of the androgen independent tumor CWR22R to therapy with control antibody (C, ▲), HERCEPTIN® (H, o) or monoclonal antibody 2C4 (2, ■). Administration of monoclonal antibody 2C4 designated by +; administration of HERCEPTIN® designated by +.

Figure 16 depicts response of the androgen independent tumor CWR22R to therapy with TAXOL® alone (T, o), monoclonal antibody 2C4 alone (2, ■) or a combination of monoclonal antibody 2C4 and TAXOL® (2/T, ▲). Administration of 2C4 designated by *; administration of TAXOL® (6.25 mg/kg) designated by +.

Figure 17 depicts response of the androgen independent tumor CWRSA6 to therapy with control antibody (C. A), HERCEPTIN® (H, o) or monoclonal antibody 2C4 (2, ■). Administration of monoclonal antibody 2C4 designated by +; administration of HERCEPTIN® designated by +.

Figure 18 depicts response of the androgen independent tumor CWRSA6 to therapy with TAXOL® alone (T, o), monoclonal antibody 2C4 alone (2,■) or a combination of monoclonal antibody 2C4 and TAXOL® (2/T, ▲). Administration of 2C4 designated by *; administration of TAXOL® (6.25 mg/kg) designated by +.

Figure 19 depicts relative TGF-α mRNA expression by CWR22R or CWR22 cells as determined by Real Time Quantitative PCR.

Figure 20 depicts relative HB-EGF mRNA expression by CWR22R or CWR22 cells as determined by Real Time Quantitative PCR.

Figure 21 depicts the effect of anti-ErbB2 monoclonal antibody treatment on the growth of prostate cancer xenografts. Tumor growth is normalized to control tumors at the end of each experiment when control animals were sacrificed. The values shown for CWR22 correspond to day 23 after the formation of a palpable tumor; for LNCaP, to day 51; for CWR22R, to day 22; for CWR22SA6, to day 33.

Figure 22 shows the effect of anti-ErbB2 monoclonal antibody treatment on PSA index. PSA index is defined as the amount of serum PSA normalized to tumor volume.

Figure 23 evaluates the activity of recombinant humanized monoclonal antibody (rhuMAb 2C4), a pegylated Fab fragment thereof, and murine 2C4, on the CWR22R androgen independent prostate xenograft.

Figure 24 depicts dose response of rhuMAb 2C4 on the CWR22R androgen independent prostate xenograft.

Figure 25 depicts dose response of rhuMAb 2C4 on the MSKPC6 androgen independent prostate xenograft.

Figure 26 depicts 2C4 and 7C2 dose response in androgen dependent prostate xenograft (CWR22).

Figure 27 depicts tumor volume in CWR22R xenografts treated with TAXOL® and anti-ErbB2 antibodies 2C4 and 7C2.

**Detailed Description of the Preferred Embodiments**

**I. Definitions**

An "ErbB receptor" is a receptor protein tyrosine kinase which belongs to the ErbB receptor family and includes EGFR, ErbB2, ErbB3 and ErbB4 receptors and other members of this family to be identified in the future. The ErbB receptor will generally comprise an extracellular domain, which may bind an ErbB ligand; a lipophilic transmembrane domain; a conserved intracellular tyrosine kinase domain; and a carboxyl-terminal signaling domain harboring several tyrosine residues which can be phosphorylated. The ErbB receptor may be a "native sequence" ErbB receptor or an "amino acid sequence variant" thereof. Preferably the ErbB receptor is native sequence human ErbB receptor.

The terms "ErbB 1", "epidermal growth factor receptor" and "EGFR" are used interchangeably herein and refer to EGFR as disclosed, for example, in Carpenter *et al. Ann. Rev. Biochem.* 56:881-914 (1987), including naturally occurring mutant forms thereof (*e.g.* a deletion mutant EGFR as in Humphrey *et al. PNAS (USA)* 87:4207-421 (1990)). *erb*B1 refers to the gene encoding the EGFR protein product.

The expressions "ErbB2" and "HER2" are used interchangeably herein and refer to human HER2 protein described, for example, in Semba *et al., PNAS (USA)* 82:6497-6501 (1985) and Yamamoto *et al. Nature* 319:230-234 (1986) (Genebank accession number X03363). The term *"erb*B2*"* refers to the gene encoding human ErbB2 and *"neu"* refers to the gene encoding rat p185$^{neu}$. Preferred ErbB2 is native sequence human ErbB2.

"ErbB3 " and "HER3 " refer to the receptor polypeptide as disclosed, for example, in US Pat. Nos. 5,183,884 and 5,480,968 as well as Kraus *et al. PNAS (USA)* 86:9193-9197 (1989).

The terms "ErbB4" and "HER4 " herein refer to the receptor polypeptide as disclosed, for example, in EP Pat Appln No 599,274; Plowman *et al., Proc. Natl. Acad. Sci. USA,* 90:1746-1750 (1993); and Plowman *et al., Nature,* 366: 473-475 (1993), including isoforms thereof, *e.g.,* as disclosed in WO99/19488 published April 22, 1999.

By "ErbB ligand" is meant a polypeptide which binds to and/or activates an ErbB receptor. The ErbB ligand of particular interest herein is a native sequence human ErbB ligand such as epidermal growth factor (EGF) (Savage *et al., J. Biol. Chem.* 247:7612-7621 (1972)); transforming growth factor alpha (TGF-α) (Marquardt *et al., Science* 223: 1079-1082 (1984)); amphiregulin also known as schwanoma or keratinocyte autocrine growth factor (Shoyab *et al. Science* 243:1074-1076 (1989); Kimura *et al. Nature* 348:257-260 (1990); and Cook *et al. Mol. Cell. Biol.* 11:2547-2557 (1991)); betacellulin (Shing *et al., Science* 259:1604-1607 (1993); and Sasada *et al. Biochem. Biophys. Res. Commun.* 190:1173 (1993)); heparin-binding epidermal growth factor (HB-EGF) (Higashiyama *et al., Science* 251:936-939 (1991)); epiregulin (Toyoda *et al., J. Biol. Chem.* 270:7495-7500 (1995); and Komurasaki *et al. Oncogene* 15:2841-2848 (1997)); a heregulin (see below); neuregulin-2 (NRG-2) (Carraway *et al., Nature* 387:512-516 (1997)); neuregulin-3 (NRG-3) (Zhang *et al.. Proc. Natl. Acad. Sci.* 94:9562-9567 (1997)); neuregulin-4 (NRG-4) (Harari *et al. Oncogene* 18: 2681-2689 (1999)); or cripto (CR-1)(Kannan *et al. J. Biol. Chem.* 272(6):3330-3335 (1997)). ErbB ligands which bind EGFR include EGF, TGF-α, amphiregulin, betacellulin, HB-EGF and epiregulin. ErbB ligands which bind ErbB3 include heregulins. ErbB ligands capable of binding ErbB4 include betacellulin, epiregulin, HB-EGF, NRG-2, NRG-3, NRG-4 and heregulins.

"Heregulin" (HRG) when used herein refers to a polypeptide encoded by the heregulin gene product as disclosed in U.S. Patent No. 5,641,869 or Marchionni *et al., Nature,* 362:312-318 (1993). Examples of heregulins include heregulin-α, heregulin-β1, heregulin-β2 and heregulin-β3 (Holmes *et al., Science,* 256:1205-1210 (1992); and U.S. Patent No. 5,641,869); *neu* differentiation factor (NDF) (Peles *et al. Cell* 69: 205-216 (1992)); acetylcholine receptor-inducing activity (ARIA) (Falls *et al. Cell* 72:801-815 (1993)); glial growth factors (GGFs) (Marchionni *et al., Nature,* 362:312-318 (1993)); sensory and motor neuron derived factor (SMDF) (Ho *et al. J. Biol. Chem.* 270:14523-14532 (1995)); γ-heregulin (Schaefer *et al. Oncogene* 15:1385-1394 (1997)). The term includes biologically active fragments and/or amino acid sequence variants of a native sequence HRG polypeptide, such as an EGF-like domain fragment thereof (*e.g.* HRGβ1$_{177-244}$).

An "ErbB hetero-oligomer" herein is a noncovalently associated oligomer comprising at least two different ErbB receptors. Such complexes may form when a cell expressing two or more ErbB receptors is exposed to an ErbB ligand and can be isolated by immunoprecipitation and analyzed by SDS-PAGE as described in Sliwkowski *et al., J. Biol. Chem.,* 269(20):14661-14665 (1994), for example. Examples of such ErbB hetero-oligomers include EGFR-ErbB2, ErbB2-ErbB3 and ErbB3-ErbB4 complexes. Moreover, the ErbB hetero-oligomer may comprise two or more ErbB2 receptors combined with a different ErbB receptor, such as ErbB3, ErbB4 or EGFR. Other proteins, such as a cytokine receptor subunit (*e.g.* gp130) may be included in the hetero-oligomer.

By "ligand activation of an ErbB receptor" is meant signal transduction (*e.g.* that caused by an intracellular kinase domain of an ErbB receptor phosphorylating tyrosine residues in the ErbB receptor or a substrate polypeptide) mediated by ErbB ligand binding to a ErbB hetero-oligomer comprising the ErbB receptor of interest. Generally, this will involve binding of an ErbB ligand to an ErbB hetero-oligomer which activates a kinase domain of one or more of the ErbB receptors in the hetero-oligomer and thereby results in phosphorylation of tyrosine residues in one or more of the ErbB receptors and/or phosphorylation of tyrosine residues in additional substrate polypeptides(s). ErbB receptor activation can be quantified using various tyrosine phosphorylation assays.

A "native sequence" polypeptide is one which has the same amino acid sequence as a polypeptide (*e.g.*, ErbB receptor or ErbB ligand) derived from nature. Such native sequence polypeptides can be isolated from nature or can be produced by recombinant or synthetic means. Thus, a native sequence polypeptide can have the amino acid sequence of naturally occurring human polypeptide, murine polypeptide, or polypeptide from any other mammalian species.

The term "amino acid sequence variant" refers to polypeptides having amino acid sequences that differ to some extent from a native sequence polypeptide. Ordinarily, amino acid sequence variants will possess at least about 70% homology with at least one receptor binding domain of a native ErbB ligand or with at least one ligand binding domain of a native ErbB receptor, and preferably, they will be at least about 80%, more preferably at least about 90% homologous with such receptor or ligand binding domains. The amino acid sequence variants possess substitutions, deletions, and/or insertions at certain positions within the amino acid sequence of the native amino acid sequence.

"Homology" is defined as the percentage of residues in the amino acid sequence variant that are identical after aligning the sequences and introducing gaps, if necessary, to achieve the maximum percent homology. Methods and computer programs for the alignment are well known in the art. One such computer program is "Align 2", authored by Genentech, Inc., which was filed with user documentation in the United States Copyright Office, Washington, DC 20559, on December 10, 1991.

The term "antibody" herein is used in the broadest sense and specifically covers monoclonal antibodies, polyclonal antibodies, multispecific antibodies (*e.g.* bispecific antibodies) formed from at least two intact antibodies, and antibody fragments, so long as they exhibit the desired biological activity.

The term "monoclonal antibody" as used herein refers to an antibody obtained from a population of substantially homogeneous antibodies, *i.e.*, the individual antibodies comprising the population are identical except for possible naturally occurring mutations that may be present in minor amounts. Monoclonal antibodies are highly specific, being directed against a single antigenic site. Furthermore, in contrast to polyclonal antibody preparations which include different antibodies directed against different determinants (epitopes), each monoclonal antibody is directed against a single determinant on the antigen. In addition to their specificity, the monoclonal antibodies are advantageous in that they may be synthesized uncontaminated by other antibodies. The modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by the hybridoma method first described by Kohler *et al., Nature,* 256:495 (1975), or may be made by recombinant DNA methods (see, *e.g.,* U.S. Patent No. 4,816,567). The "monoclonal antibodies" may also be isolated from phage antibody libraries using the techniques described in Clackson *et al., Nature,* 352:624-628 (1991) and Marks *et al., J. Mol. Biol.,* 222:581-597 (1991), for example.

The monoclonal antibodies herein specifically include "chimeric" antibodies in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired biological activity (U.S. Patent No. 4,816,567; and Morrison *et al., Proc. Natl. Acad. Sci. USA,* 81:6851-6855 (1984)). Chimeric antibodies of interest herein include "primatized" antibodies comprising variable domain antigen-binding sequences derived from a non-human primate (*e.g.* Old World Monkey, Ape etc) and human constant region sequences.

"Antibody fragments" comprise a portion of an intact antibody, preferably comprising the antigen-binding or variable region thereof. Examples of antibody fragments include Fab, Fab', F(ab')$_2$, and Fv fragments; diabodies; linear antibodies; single-chain antibody molecules; and multispecific antibodies formed from antibody fragment(s).

An "intact" antibody is one which comprises an antigen-binding variable region as well as a light chain constant domain (C$_L$) and heavy chain constant domains, C$_H$1, C$_H$2 and C$_H$3. The constant domains may be native sequence constant domains (*e.g.* human native sequence constant domains) or amino acid sequence variant thereof. Preferably, the intact antibody has one or more effector functions.

Antibody "effector functions" refer to those biological activities attributable to the Fc region (a native sequence Fc region or amino acid sequence variant Fc region) of an antibody. Examples of antibody effector functions include Clq binding; complement dependent cytotoxicity; Fc receptor binding; antibody-dependent cell-mediated cytotoxicity (ADCC); phagocytosis; down regulation of cell surface receptors (*e.g.* B cell receptor; BCR), etc.

Depending on the amino acid sequence of the constant domain of their heavy chains, intact antibodies can be assigned to different "classes". There are five major classes of intact antibodies: IgA, IgD, IgE, IgG, and IgM, and several of these may be further divided into "subclasses" (isotypes), *e.g.*, IgG1, IgG2, IgG3, IgG4, IgA, and IgA2. The heavy-chain constant domains that correspond to the different classes of antibodies are called α, δ, ε, γ, and μ, respectively. The subunit structures and three-dimensional configurations of different classes of immunoglobulins are well known.

"Antibody-dependent cell-mediated cytotoxicity" and "ADCC" refer to a cell-mediated reaction in which nonspecific cytotoxic cells that express Fc receptors (FcRs) (*e.g.* Natural Killer (NK) cells, neutrophils, and macrophages) recognize bound antibody on a target cell and subsequently cause lysis of the target cell. The primary cells for mediating ADCC, NK cells, express FcγRIII only, whereas monocytes express FcγRI, FcγRII and FcγRIII. FcR expression on hematopoietic cells in summarized is Table 3 on page 464 of Ravetch and Kinet, *Annu. Rev. Immunol* 9:457-92 (1991). To assess ADCC activity of a molecule of interest, an *in vitro* ADCC assay, such as that described in US Patent No. 5,500,362 or 5,821,337 may be performed. Useful effector cells for such assays include peripheral blood mononuclear cells (PBMC) and Natural Killer (NK) cells. Alternatively, or additionally, ADCC activity of the molecule of interest may be *assessed in vivo, e.g.,* in a animal model such as that disclosed in Clynes *et al. PNAS (USA)* 95:652-656 (1998).

"Human effector cells" are leukocytes which express one or more FcRs and perform effector functions. Preferably, the cells express at least FcγRIII and perform ADCC effector function. Examples of human leukocytes which mediate ADCC include peripheral blood mononuclear cells (PBMC), natural killer (NK) cells, monocytes, cytotoxic T cells and neutrophils; with PBMCs and NK cells being preferred. The effector cells may be isolated from a native source thereof, *e.g.* from blood or PBMCs as described herein.

The terms "Fc receptor" or "FcR" are used to describe a receptor that binds to the Fc region of an antibody. The preferred FcR is a native sequence human FcR. Moreover, a preferred FcR is one which binds an IgG antibody (a gamma receptor) and includes receptors of the FcγRI, FcγRII, and Fcγ RIII subclasses, including allelic variants and alternatively spliced forms of these receptors. FcγRII receptors include FcγRIIA (an "activating receptor") and FcγRIIB (an "inhibiting receptor"), which have similar amino acid sequences that differ primarily in the cytoplasmic domains thereof. Activating receptor FcγRIIA contains an immunoreceptor tyrosine-based activation motif (ITAM) in its cytoplasmic domain. Inhibiting receptor FcγRIIB contains an immunoreceptor tyrosine-based inhibition motif (ITIM) in its cytoplasmic domain. (see review M. in Daëron, *Annu. Rev. Immunol.* 15:203-234 (1997)). FcRs are reviewed in Ravetch and Kinet, *Annu. Rev. Immunol* 9:457-92 (1991); Capel *et al., Immunomethods* 4:25-34 (1994); and de Haas *et al., J. Lab. Clin. Med.* 126:330-41 (1995). Other FcRs, including those to be identified in the future, are encompassed by the term "FcR" herein. The term also includes the neonatal receptor, FcRn, which is responsible for the transfer of maternal IgGs to the fetus (Guyer *et al., J. Immunol.* 117:587 (1976) and Kim *et al., J. Immunol.* 24:249 (1994)).

"Complement dependent cytotoxicity" or "CDC" refers to the ability of a molecule to lyse a target in the presence of complement. The complement activation pathway is initiated by the binding of the first component of the complement system (C1q) to a molecule (*e.g.* an antibody) complexed with a cognate antigen. To assess complement activation, a CDC assay, *e.g.* as described in Gazzano-Santoro *et al., J. Immunol. Methods* 202:163 (1996), may be performed.

"Native antibodies" are usually heterotetrameric glycoproteins of about 150,000 daltons, composed of two identical light (L) chains and two identical heavy (H) chains. Each light chain is linked to a heavy chain by one covalent disulfide bond, while the number of disulfide linkages varies among the heavy chains of different immunoglobulin isotypes. Each heavy and light chain also has regularly spaced intrachain disulfide bridges. Each heavy chain has at one end a variable domain ($V_H$) followed by a number of constant domains. Each light chain has a variable domain at one end ($V_L$) and a constant domain at its other end. The constant domain of the light chain is aligned with the first constant domain of the heavy chain, and the light-chain variable domain is aligned with the variable domain of the heavy chain. Particular amino acid residues are believed to form an interface between the light chain and heavy chain variable domains.

The term "variable" refers to the fact that certain portions of the variable domains differ extensively in sequence among antibodies and are used in the binding and specificity of each particular antibody for its particular antigen. However, the variability is not evenly distributed throughout the variable domains of antibodies. It is concentrated in three segments called hypervariable regions both in the light chain and the heavy chain variable domains. The more highly conserved portions of variable domains are called the framework regions (FRs). The variable domains of native heavy and light chains each comprise four FRs, largely adopting a β-sheet configuration, connected by three hypervariable regions, which form loops connecting, and in some cases forming part of, the β-sheet structure. The hypervariable regions in each chain are held together in close proximity by the FRs and, with the hypervariable regions from the other chain, contribute to the formation of the antigen-binding site of antibodies (see Kabat *et al., Sequences of Proteins of Immunological Interest,* 5th Ed. Public Health Service, National Institutes of Health, Bethesda, MD. (1991)). The constant domains are not involved directly in binding an antibody to an antigen, but exhibit various effector functions, such as participation of the antibody in antibody dependent cellular cytotoxicity (ADCC).

The term "hypervariable region" when used herein refers to the amino acid residues of an antibody which are responsible for antigen-binding. The hypervariable region generally comprises amino acid residues from a "complementarity determining region" or "CDR" (*e.g.* residues 24-34 (L1), 50-56 (L2) and 89-97 (L3) in the light chain variable domain and 31-35 (H1), 50-65 (H2) and 95-102 (H3) in the heavy chain variable domain; Kabat *et al., Sequences of Proteins of Immunological Interest,* 5th Ed. Public Health Service, National Institutes of Health, Bethesda, MD. (1991)) and/or those residues from a "hypervariable loop" (*e.g.* residues 26-32 (L1), 50-52 (L2) and 91-96 (L3) in the light chain variable domain and 26-32 (H1), 53-55 (H2) and 96-101 (H3) in the heavy chain variable domain; Chothia and Lesk *J. Mol. Biol.* 196:901-917 (1987)). "Framework Region" or "FR" residues are those variable domain residues other than the hypervariable region residues as herein defined.

Papain digestion of antibodies produces two identical antigen-binding fragments, called "Fab" fragments, each with a single antigen-binding site, and a residual "Fc" fragment, whose name reflects its ability to crystallize readily. Pepsin treatment yields an F(ab')$_2$ fragment that has two antigen-binding sites and is still capable of cross-linking antigen.

"Fv" is the minimum antibody fragment which contains a complete antigen-recognition and antigen-binding site. This region consists of a dimer of one heavy chain and one light chain variable domain in tight, noncovalent association. It is in this configuration that the three hypervariable regions of each variable domain interact to define an antigen-binding site on the surface of the $V_H$-$V_L$ dimer. Collectively, the six hypervariable regions confer antigen-binding specificity to the antibody. However, even a single variable domain (or half of an Fv comprising only three hypervariable regions specific for an antigen) has the ability to recognize and bind antigen, although at a lower affinity than the entire binding site.

The Fab fragment also contains the constant domain of the light chain and the first constant domain (CH1) of the heavy chain. Fab' fragments differ from Fab fragments by the addition of a few residues at the carboxy terminus of the heavy chain CH1 domain including one or more cysteines from the antibody hinge region. Fab'-SH is the designation herein for Fab' in which the cysteine residue(s) of the constant domains bear at least one free thiol group. F(ab')$_2$ antibody fragments originally were produced as pairs of Fab' fragments which have hinge cysteines between them. Other chemical couplings of antibody fragments are also known.

The "light chains" of antibodies from any vertebrate species can be assigned to one of two clearly distinct types, called kappa ($\kappa$) and lambda ($\lambda$), based on the amino acid sequences of their constant domains.

"Single-chain Fv" or "scFv" antibody fragments comprise the $V_H$ and $V_L$ domains of antibody, wherein these domains are present in a single polypeptide chain. Preferably, the Fv polypeptide further comprises a polypeptide linker between the $V_H$ and $V_L$ domains which enables the scFv to form the desired structure for antigen binding. For a review of scFv see Plückthun in *The Pharmacology of Monoclonal Antibodies,* vol. 113, Rosenburg and Moore eds., Springer-Verlag, New York, pp. 269-315 (1994). Anti-ErbB2 antibody scFv fragments are described in WO93/16185; U.S. Patent No. 5,571,894; and U.S. Patent No. 5,587,458.

The term "diabodies" refers to small antibody fragments with two antigen-binding sites, which fragments comprise a variable heavy domain ($V_H$) connected to a variable light domain ($V_L$) in the same polypeptide chain ($V_H$ - $V_L$). By using a linker that is too short to allow pairing between the two domains on the same chain, the domains are forced to pair with the complementary domains of another chain and create two antigen-binding sites. Diabodies are described more fully in, for example, EP 404,097; WO 93/11161; and Hollinger *et al., Proc. Natl. Acad. Sci. USA,* 90:6444-6448 (1993).

"Humanized" forms of non-human (*e.g.*, rodent) antibodies are chimeric antibodies that contain minimal sequence derived from non-human immunoglobulin. For the most part, humanized antibodies are human immunoglobulins (recipient antibody) in which residues from a hypervariable region of the recipient are replaced by residues from a hypervariable region of a non-human species (donor antibody) such as mouse, rat, rabbit or nonhuman primate having the desired specificity, affinity, and capacity. In some instances, framework region (FR) residues of the human immunoglobulin are replaced by corresponding non-human residues. Furthermore, humanized antibodies may comprise residues that are not found in the recipient antibody or in the donor antibody. These modifications are made to further refine antibody performance. In general, the humanized antibody will comprise substantially all of at least one, and typically two, variable domains, in which all or substantially all of the hypervariable loops correspond to those of a non-human immunoglobulin and all or substantially all of the FRs are those of a human immunoglobulin sequence. The humanized antibody optionally also will comprise at least a portion of an immunoglobulin constant region (Fc), typically that of a human immunoglobulin. For further details, see Jones *et al., Nature* 321:522-525 (1986); Riechmann *et al., Nature* 332:323-329 (1988); and Presta, *Curr. Op. Struct. Biol.* 2:593-596 (1992).

Humanized anti-ErbB2 antibodies include huMAb4D5-1, huMAb4D5-2, huMAb4D5-3, huMAb4D5-4, huMAb4D5-5, huMAb4D5-6, huMAb4D5-7 and huMAb4D5-8 (HERCEPTIN®) as described in Table 3 of U.S. Patent 5.821,337 ; humanized 520C9 (WO93/21319) and humanized 2C4 as described hereinbelow.

An "isolated" antibody is one which has been identified and separated and/or recovered from a component of its natural environment. Contaminant components of its natural environment are materials which would interfere with diagnostic or therapeutic uses for the antibody, and may include enzymes, hormones, and other proteinaceous or nonproteinaceous solutes. In preferred embodiments, the antibody will be purified (1) to greater than 95% by weight of antibody as determined by the Lowry method, and most preferably more than 99% by weight, (2) to a degree sufficient to obtain at least 15 residues of N-terminal or internal amino acid sequence by use of a spinning cup sequenator, or (3) to homogeneity by SDS-PAGE under reducing or nonreducing conditions using Coomassie blue or, preferably, silver stain. Isolated antibody includes the antibody *in situ* within recombinant cells since at least one component of the antibody's natural environment will not be present. Ordinarily, however, isolated antibody will be prepared by at least one purification step.

An antibody "which binds" an antigen of interest, *e.g.* ErbB2 antigen, is one capable of binding that antigen with sufficient affinity such that the antibody is useful as a therapeutic agent in targeting a cell expressing the antigen. Where the antibody is one which binds ErbB2, it will usually preferentially bind ErbB2 as opposed to other ErbB receptors, and may be one which does not significantly cross-react with other proteins such as EGFR. ErbB3 or ErbB4. In such embodiments, the extent of binding of the antibody to these non-ErbB2 proteins (*e.g.*, cell surface binding to endogenous receptor) will be less than 10% as determined by fluorescence activated cell sorting (FACS) analysis or radioimmunoprecipitation (RIA). Sometimes, the anti-ErbB2 antibody will not significantly cross-react with the rat *neu* protein, *e.g.*, as described in Schecter *et al. Nature* 312:513 (1984) and Drebin *et al., Nature* 312:545-548 (1984).

An antibody which "blocks" ligand activation of an ErbB receptor is one which reduces or prevents such activation as hereinabove defined, wherein the antibody is able to block ligand activation of the ErbB receptor substantially more effectively than monoclonal antibody 4D5, *e.g.* about as effectively as monoclonal antibodies 7F3 or 2C4 or Fab fragments thereof and preferably about as effectively as monoclonal antibody 2C4 or a Fab fragment thereof. For example, the antibody that blocks ligand activation of an ErbB receptor may be one which is about 50-100% more effective than 4D5 at blocking formation of an ErbB hetero-oligomer. Blocking of ligand activation of an ErbB receptor can occur by any means, *e.g.* by interfering with: ligand binding to an ErbB receptor, ErbB complex formation, tyrosine kinase activity of an ErbB receptor in an ErbB complex and/or phosphorylation of tyrosine kinase residue(s) in or by an ErbB receptor. Examples of antibodies which block ligand activation of an ErbB receptor include monoclonal antibodies 2C4 and 7F3 (which block HRG activation of ErbB2/ErbB3 and ErbB2/ErbB4 hetero-oligomers; and EGF, TGF-α, amphiregulin, HB-EGF and/or epiregulin activation of an EGFR/ErbB2 hetero-oligomer); and L26, L96 and L288 antibodies (Klapper *et al. Oncogene* 14:2099-2109 (1997)), which block EGF and NDF binding to T47D cells which express EGFR, ErbB2, ErbB3 and ErbB4.

An antibody having a "biological characteristic" of a designated antibody, such as the monoclonal antibody designated 2C4, is one which possesses one or more of the biological characteristics of that antibody which distinguish it from other antibodies that bind to the same antigen (*e.g.* ErbB2). For example, an antibody with a biological characteristic of 2C4 may block HRG activation of an ErbB hetero-oligomer comprising ErbB2 and ErbB3 or ErbB4; block EGF, TGF-α, HB-EGF, epiregulin and/or amphiregulin activation of an ErbB receptor comprising EGFR and ErbB2; block EGF, TGF-α and/or HRG mediated activation of MAPK; and/or bind the same epitope in the extracellular domain of ErbB2 as that bound by 2C4 (*e.g.* which blocks binding of monoclonal antibody 2C4 to ErbB2).

Unless indicated otherwise, the expression "monoclonal antibody 2C4" refers to an antibody that has antigen binding residues of, or derived from, the murine 2C4 antibody of the Examples below. For example, the monoclonal antibody 2C4 may be murine monoclonal antibody 2C4 or a variant thereof, such as a humanized 2C4, possessing antigen binding amino acid residues of murine monoclonal antibody 2C4. Examples of humanized 2C4 antibodies are provided in Example 3 below. Unless indicated otherwise, the expression "rhuMAb 2C4" when used herein refers to an antibody comprising the variable light ($V_L$) and variable heavy ($V_H$) sequences of SEQ ID Nos. 3 and 4, respectively, fused to human light and heavy IgG 1 (non-A allotype) constant region sequences optionally expressed by a Chinese Hamster Ovary (CHO) cell.

Unless indicated otherwise, the term "monoclonal antibody 4D5" refers to an antibody that has antigen binding residues of, or derived from, the murine 4D5 antibody (ATCC CRL 10463). For example, the monoclonal antibody 4D5 may be murine monoclonal antibody 4D5 or a variant thereof, such as a humanized 4D5, possessing antigen binding residues of murine monoclonal antibody 4D5. Exemplary humanized 4D5 antibodies include huMAb4D5-1, huMAb4D5-2, huMAb4D5-3, huMAb4D5-4, huMAb4D5-5, huMAb4D5-6, huMAb4D5-7 and huMAb4D5-8 (HERCEPTIN®) as in US Patent No. 5,821,337, with huMAb4D5-8 (HERCEPTIN®) being a preferred humanized 4D5 antibody.

A "growth inhibitory agent" when used herein refers to a compound or composition which inhibits growth of a cell, especially an ErbB expressing cancer cell either *in vitro* or *in vivo.* Thus, the growth inhibitory agent may be one which significantly reduces the percentage of ErbB expressing cells in S phase. Examples of growth inhibitory agents include agents that block cell cycle progression (at a place other than S phase), such as agents that induce G1 arrest and M-phase arrest. Classical M-phase blockers include the vincas (vincristine and vinblastine), taxanes, and topo II inhibitors such as doxorubicin, epirubicin, daunorubicin, etoposide, and bleomycin. Those agents that arrest G1 also spill over into S-phase arrest, for example, DNA alkylating agents such as tamoxifen, prednisone, dacarbazine, mechlorethamine, cisplatin, methotrexate, 5-fluorouracil, and ara-C. Further information can be found in *The Molecular Basis of Cancer,* Mendelsohn and Israel, eds., Chapter 1, entitled "Cell cycle regulation, oncogenes, and antineoplastic drugs" by Murakami *et al.* (WB Saunders: Philadelphia, 1995), especially p. 13.

Examples of "growth inhibitory" antibodies are those which bind to ErbB2 and inhibit the growth of cancer cells overexpressing ErbB2. Preferred growth inhibitory anti-ErbB2 antibodies inhibit growth of SK-BR-3 breast tumor cells in cell culture by greater than 20%, and preferably greater than 50% (*e.g.* from about 50% to about 100%) at an antibody concentration of about 0.5 to 30 μg/ml, where the growth inhibition is determined six days after exposure of the SK-BR-3 cells to the antibody (see U.S. Patent No. 5,677,171 issued October 14, 1997). The SK-BR-3 cell growth inhibition assay is described in more detail in that patent and hereinbelow.

An antibody which "induces cell death" is one which causes a viable cell to become nonviable. The cell is generally one which expresses the ErbB2 receptor, especially where the cell overexpresses the ErbB2 receptor. Preferably, the cell is a cancer cell, *e.g.* a breast, ovarian, stomach, endometrial, salivary gland, lung, kidney, colon, thyroid, pancreatic or bladder cell. *In vitro,* the cell may be a SK-BR-3, BT474, Calu 3, MDA-MB-453, MDA-MB-361 or SKOV3 cell. Cell death *in vitro* may be determined in the absence of complement and immune effector cells to distinguish cell death induced by antibody-dependent cell-mediated cytotoxicity (ADCC) or complement dependent cytotoxicity (CDC). Thus, the assay for cell death may be performed using heat inactivated serum (*i.e.* in the absence of complement) and in the absence of immune effector cells. To determine whether the antibody is able to induce cell death, loss of membrane integrity as evaluated by uptake of propidium iodide (PI), trypan blue (see Moore *er al. Cytotechnology* 17:1-11 (1995)) or 7AAD can be assessed relative to untreated cells. Preferred cell death-inducing antibodies are those which induce PI uptake in the PI uptake assay in BT474 cells (see below).

An antibody which "induces apoptosis" is one which induces programmed cell death as determined by binding of annexin V, fragmentation of DNA, cell shrinkage, dilation of endoplasmic reticulum, cell fragmentation, and/or formation of membrane vesicles (called apoptotic bodies). The cell is usually one which overexpresses the ErbB2 receptor. Preferably the cell is a tumor cell, e.g. a breast, ovarian, stomach, endometrial, salivary gland, lung, kidney, colon, thyroid, pancreatic or bladder cell. *In vitro,* the cell may be a SK-BR-3, BT474, Calu 3 cell, MDA-MB-453, MDA-MB-361 or SKOV3 cell. Various methods are available for evaluating the cellular events associated with apoptosis. For example, phosphatidyl serine (PS) translocation can be measured by annexin binding; DNA fragmentation can be evaluated through DNA laddering; and nuclear/chromatin condensation along with DNA fragmentation can be evaluated by any increase in hypodiploid cells. Preferably, the antibody which induces apoptosis is one which results in about 2 to 50 fold, preferably about 5 to 50 fold, and most preferably about 10 to 50 fold, induction of annexin binding relative to untreated cell in an annexin binding assay using BT474 cells (see below). Sometimes the pro-apoptotic antibody will be one which further blocks ErbB ligand activation of an ErbB receptor (e.g. 7F3 antibody); *i.e.* the antibody shares a biological characteristic with monoclonal antibody 2C4. In other situations, the antibody is one which does not significantly block ErbB ligand activation of an ErbB receptor (*e.g.* 7C2). Further, the antibody may be one like 7C2 which, while inducing apoptosis, does not induce a large reduction in the percent of cells in S phase (*e.g.* one which only induces about 0-10% reduction in the percent of these cells relative to control).

The "epitope 2C4" is the region in the extracellular domain of ErbB2 to which the antibody 2C4 binds. In order to screen for antibodies which bind to the 2C4 epitope, a routine cross-blocking assay such as that described in *Antibodies, A Laboratory Manual,* Cold Spring Harbor Laboratory, Ed Harlow and David Lane (1988), can be performed. Alternatively, epitope mapping can be performed to assess whether the antibody binds to the 2C4 epitope of ErbB2 (*e.g.* any one or more residues in the region from about residue 22 to about residue 584 of ErbB2, inclusive; see Figs. 1A-B).

The "epitope 4D5" is the region in the extracellular domain of ErbB2 to which the antibody 4D5 (ATCC CRL 10463) binds. This epitope is close to the transmembrane domain of ErbB2. To screen for antibodies which bind to the 4D5 epitope, a routine cross-blocking assay such as that described in *Antibodies, A Laboratory Manual,* Cold Spring Harbor Laboratory, Ed Harlow and David Lane (1988), can be performed. Alternatively, epitope mapping can be performed to assess whether the antibody binds to the 4D5 epitope of ErbB2 (*e.g.* any one or more residues in the region from about residue 529 to about residue 625, inclusive; see Figs. 1A-B).

The "epitope 3H4" is the region in the extracellular domain of ErbB2 to which the antibody 3H4 binds. This epitope includes residues from about 541 to about 599, inclusive, in the amino acid sequence of ErbB2 extracellular domain; see Figs. 1A-B.

The "epitope 7C2/7F3" is the region at the N terminus of the extracellular domain of ErbB2 to which the 7C2 and/or 7F3 antibodies (each deposited with the ATCC, see below) bind. To screen for antibodies which bind to the 7C2/7F3 epitope, a routine cross-blocking assay such as that described in *Antibodies, A Laboratory Manual,* Cold Spring Harbor Laboratory, Ed Harlow and David Lane (1988), can be performed. Alternatively, epitope mapping can be performed to establish whether the antibody binds to the 7C2/7F3 epitope on ErbB2 (*e.g.* any one or more of residues in the region from about residue 22 to about residue 53 of ErbB2; see Figs. 1A-B).

"Treatment" refers to both therapeutic treatment and prophylactic or preventative measures. Those in need of treatment include those already with the disorder as well as those in which the disorder is to be prevented. Hence, the mammal to be treated herein may have been diagnosed as having the disorder or may be predisposed or susceptible to the disorder.

"Mammal" for purposes of treatment refers to any animal classified as a mammal, including humans, domestic and farm animals, and zoo, sports, or pet animals, such as dogs, horses, cats, cows, *etc.* Preferably, the mammal is human.

The term "therapeutically effective amount" refers to an amount of a drug effective to treat a disease or disorder in a mammal. In the case of cancer, the therapeutically effective amount of the drug may reduce the number of cancer cells; reduce the tumor size; inhibit (*i.e.,* slow to some extent and preferably stop) cancer cell infiltration into peripheral organs; inhibit (*i.e.,* slow to some extent and preferably stop) tumor metastasis; inhibit, to some extent, tumor growth; and/or relieve to some extent one or more of the symptoms associated with the cancer. To the extent the drug may prevent growth and/or kill existing cancer cells, it may be cytostatic and/or cytotoxic. For cancer therapy, efficacy can, for example, be measured by assessing the time to disease progression (TTP) and/or determining the response rate (RR).

The terms "cancer" and "cancerous" refer to or describe the physiological condition in mammals that is typically characterized by unregulated cell growth. Examples of cancer include, but are not limited to, carcinoma, lymphoma, blastoma, sarcoma, and leukemia or lymphoid malignancies. More particular examples of such cancers include squamous cell cancer (*e.g.* epithelial squamous cell cancer), lung cancer including small-cell lung cancer, non-small cell lung cancer, adenocarcinoma of the lung and squamous carcinoma of the lung, cancer of the peritoneum, hepatocellular cancer, gastric or stomach cancer including gastrointestinal cancer, pancreatic cancer, glioblastoma, cervical cancer, ovarian cancer, liver cancer, bladder cancer, hepatoma, breast cancer, colon cancer, rectal cancer, colorectal cancer, endometrial or uterine carcinoma, salivary gland carcinoma, kidney or renal cancer, prostate cancer, vulval cancer, thyroid cancer, hepatic carcinoma, anal carcinoma, penile carcinoma, as well as head and neck cancer.

An "ErbB-expressing cancer" is one comprising cells which have ErbB protein present at their cell surface. An "ErbB2-expressing cancer" is one which produces sufficient levels of ErbB2 at the surface of cells thereof, such that an anti-ErbB2 antibody can bind thereto and have a therapeutic effect with respect to the cancer.

A cancer "characterized by excessive activation" of an ErbB receptor is one in which the extent of ErbB receptor activation in cancer cells significantly exceeds the level of activation of that receptor in non-cancerous cells of the same tissue type. Such excessive activation may result from overexpression of the ErbB receptor and/or greater than normal levels of an ErbB ligand available for activating the ErbB receptor in the cancer cells. Such excessive activation may cause and/or be caused by the malignant state of a cancer cell. In some embodiments, the cancer will be subjected to a diagnostic or prognostic assay to determine whether amplification and/or overexpression of an ErbB receptor is occurring which results in such excessive activation of the ErbB receptor. Alternatively, or additionally, the cancer may be subjected to a diagnostic or prognostic assay to determine whether amplification and/or overexpression an ErbB ligand is occurring in the cancer which attributes to excessive activation of the receptor. In a subset of such cancers, excessive activation of the receptor may result from an autocrine stimulatory pathway.

In an "autocrine" stimulatory pathway, self stimulation occurs by virtue of the cancer cell producing both an ErbB ligand and its cognate ErbB receptor. For example, the cancer may express or overexpress EGFR and also express or overexpress an EGFR ligand *(e.g.* EGF, TGF-$\alpha$ or HB-EGF). In another embodiment, the cancer may express or overexpress ErbB2 and also express or overexpress a heregulin *(e.g.* $\gamma$-HRG).

A cancer which "overexpresses" an ErbB receptor is one which has significantly higher levels of an ErbB receptor, such as ErbB2, at the cell surface thereof, compared to a noncancerous cell of the same tissue type. Such overexpression may be caused by gene amplification or by increased transcription or translation. ErbB receptor overexpression may be determined in a diagnostic or prognostic assay by evaluating increased levels of the ErbB protein present on the surface of a cell (*e.g.* via an immunohistochemistry assay; IHC). Alternatively, or additionally, one may measure levels of ErbB-encoding nucleic acid in the cell, *e.g.* via fluorescent *in situ* hybridization; (FISH; see WO98/45479 published October, 1998), southern blotting, or polymerase chain reaction (PCR) techniques, such as real time quantitative PCR (RT-PCR). One may also study ErbB receptor overexpression by measuring shed antigen (*e.g.*, ErbB extracellular domain) in a biological fluid such as serum (see, *e.g.,* U.S. Patent No. 4,933,294 issued June 12, 1990; WO91/05264 published April 18, 1991; U.S. Patent 5,401,638 issued March 28, 1995; and Sias *et al. J. Immunol. Methods* 132: 73-80 (1990)). Aside from the above assays, various *in vivo* assays are available to the skilled practitioner. For example, one may expose cells within the body of the patient to an antibody which is optionally labeled with a detectable label, *e.g.* a radioactive isotope, and binding of the antibody to cells in the patient can be evaluated, *e.g.* by external scanning for radioactivity or by analyzing a biopsy taken from a patient previously exposed to the antibody.

Conversely, a cancer which is "not characterized by overexpression of the ErbB2 receptor" is one which, in a diagnostic assay, does not express higher than normal levels of ErbB2 receptor compared to a noncancerous cell of the same tissue type.

A cancer which "overexpresses" an ErbB ligand is one which produces significantly higher levels of that ligand compared to a noncancerous cell of the same tissue type. Such overexpression may be caused by gene amplification or by increased transcription or translation. Overexpression of the ErbB ligand may be determined diagnostically by evaluating levels of the ligand (or nucleic acid encoding it) in the patient, *e.g.* in a tumor biopsy or by various diagnostic assays such as the IHC, FISH, southern blotting, PCR or *in vivo* assays described above.

A "hormone-independent" cancer is one in which proliferation thereof is not dependent on the presence of a hormone which binds to a receptor expressed by cells in the cancer. Such cancers do not undergo clinical regression upon administration of pharmacological or surgical strategies that reduce the hormone concentration in or near the tumor. Examples of hormone-independent cancers include androgen-independent prostate cancer, estrogen-independent breast cancer, endometrial-cancer and ovarian cancer. Such cancers may begin as hormone-dependent tumors and progress from a hormone-sensitive stage to a hormone-refractory tumor following anti-hormonal therapy.

The term "cytotoxic agent" as used herein refers to a substance that inhibits or prevents the function of cells and/or causes destruction of cells. The term is intended to include radioactive isotopes (*e.g.* At$^{211}$, I$^{131}$, I$^{125}$, Y$^{90}$, Re$^{186}$, Re$^{188}$, Sm$^{153}$, Bi$^{212}$, P$^{32}$ and radioactive isotopes of Lu), chemotherapeutic agents, and toxins such as small molecule toxins or enzymatically active toxins of bacterial, fungal, plant or animal origin, including fragments and/or variants thereof.

A "chemotherapeutic agent" is a chemical compound useful in the treatment of cancer. Examples of chemotherapeutic agents include alkylating agents such as thiotepa and cyclosphosphamide (CYTOXAN™); alkyl sulfonates such as busulfan, improsulfan and piposulfan; aziridines such as benzodopa, carboquone, meturedopa, and uredopa; ethylenimines and methylamelamines including altretamine, triethylenemelamine, trietylenephosphoramide, triethylenethiophosphaoramide and trimethylolomelamine; nitrogen mustards such as chlorambucil, chlornaphazine, cholophosphamide, estramustine, ifosfamide, mechlorethamine, mechlorethamine oxide hydrochloride, melphalan, novembichin, phenesterine, prednimustine, trofosfamide, uracil mustard; nitrosureas such as carmustine, chlorozotocin, fotemustine, lomustine, nimustine, ranimustine; antibiotics such as aclacinomysins, actinomycin, authramycin, azaserine, bleomycins, cactinomycin, calicheanucin, carabicin, carminomycin, carzinophilin, chromomycins, dactinomycin, daunorubicin, detorubicin, 6-diazo-5-oxo-L-norleucine, doxorubicin, epirubicin, esorubicin, idarubicin, marcellomycin, mitomycins, mycophenolic acid, nogalamycin, olivomycins, peplomycin, potfiromycin, puromycin, quelamycin, rodorubicin, streptonigrin, streptozocin, tubercidin, ubenimex, zinostatin, zorubicin; anti-metabolites such as methotrexate and 5-fluorouracil (5-FU); folic acid analogues such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine, 5-FU; androgens such as calusterone, dromostanolone propionate, epitiostanol, mepitiostane, testolactone; anti-adrenals such as aminoglutethimide, mitotane, trilostane; folic acid replenisher such as frolinic acid; aceglatone; aldophosphamide glycoside; aminolevulinic acid; amsacrine; bestrabucil; bisantrene; edatraxate; defofamine; demecolcine; diaziquone; elfornithine; elliptinium acetate; etoglucid; gallium nitrate; hydroxyurea; lentinan; lonidamine; mitoguazone; mitoxantrone; mopidamol; nitracrine; pentostatin; phenamet; pirarubicin; podophyllinic acid; 2-ethylhydrazide; procarbazine; PSK®; razoxane; sizofiran; spirogermanium; tenuazonic acid; triaziquone; 2, 2',2''-trichlorotriethylamine; urethan; vindesine; dacarbazine; mannomustine; mitobronitol; mitolactol; pipobroman; gacytosine; arabinoside ("Ara-C"); cyclophosphamide; thiotepa; taxanes, *e.g.* paclitaxel (TAXOL®, Bristol-Myers Squibb Oncology, Princeton, NJ) and docetaxel (TAXOTERE®, Rhône-Poulenc Rorer, Antony, France); chlorambucil; gemcitabine; 6-thioguanine; mercaptopurine; methotrexate; platinum analogs such as cisplatin and carboplatin; vinblastine; platinum; etoposide (VP-16); ifosfamide; mitomycin C; mitoxantrone; vincristine; vinorelbine; navelbine; leucovorin (LV), novantrone; teniposide; daunomycin; aminopterin; xeloda; ibandronate; CPT-11; topoisomerase inhibitor RFS 2000; difluoromethylornithine (DMFO); retinoic acid; esperamicins; capecitabine; and pharmaceutically acceptable salts, acids or derivatives of any of the above. Also included in this definition are anti-hormonal agents that act to regulate or inhibit hormone action on tumors such as anti-estrogens including for example tamoxifen, raloxifene, aromatase inhibiting 4(5)-imidazoles, 4-hydroxytamoxifen, trioxifene, keoxifene, LY117018, onapristone, and toremifene (Fareston); and anti-androgens such as flutamide, nilutamide, bicalutamide, leuprolide, and goserelin; and pharmaceutically acceptable salts, acids or derivatives of any of the above.

The term "cytokine" is a generic term for proteins released by one cell population which act on another cell as intercellular mediators. Examples of such cytokines are lymphokines, monokines, and traditional polypeptide hormones. Included among the cytokines are growth hormone such as human growth hormone, N-methionyl human growth hormone, and bovine growth hormone; parathyroid hormone; thyroxine; insulin; proinsulin; relaxin; prorelaxin; glycoprotein hormones such as follicle stimulating hormone (FSH), thyroid stimulating hormone (TSH), and luteinizing hormone (LH); hepatic growth factor; fibroblast growth factor; prolactin; placental lactogen; tumor necrosis factor-$\alpha$ and -$\beta$; mullerian-inhibiting substance; mouse gonadotropin-associated peptide; inhibin; activin; vascular endothelial growth factor; integrin; thrombopoietin (TPO); nerve growth factors such as NGF-$\beta$; platelet-growth factor; transforming growth factors (TGFs) such as TGF-$\alpha$ and TGF-$\beta$; insulin-like growth factor-I and -II; erythropoietin (EPO); osteoinductive factors; interferons such as interferon-$\alpha$, -$\beta$, and -$\gamma$; colony stimulating factors (CSFs) such as macrophage-CSF (M-CSF); granulocyte-macrophage-CSF (GM-CSF); and granulocyte-CSF (G-CSF); interleukins (ILs) such as IL-1, IL-1$\alpha$, IL-2, IL-3, IL-4, IL-5, IL-6, IL-7, IL-8, IL-9, IL-10, IL-11, IL-12; a tumor necrosis factor such as TNF-$\alpha$ or TNF-$\beta$; and other polypeptide factors including LIF and kit ligand (KL). As used herein, the term cytokine includes proteins from natural sources or from recombinant cell culture and biologically active equivalents of the native sequence cytokines.

As used herein, the term "EGFR-targeted drug" refers to a therapeutic agent that binds to EGFR receptor and, optionally, inhibits EGFR receptor activation. Examples of such agents include antibodies and small molecules that bind to EGFR. Examples of antibodies which bind to EGFR include MAb 579 (ATCC CRL HB 8506), MAb 455 (ATCC CRL HB8507), MAb 225 (ATCC CRL 8508), MAb 528 (ATCC CRL 8509) (see, US Patent No. 4,943, 533, Mendelsohn *et al.*) and variants thereof, such as chimerized 225 (C225) and reshaped human 225 (H225) (see, WO 96/40210, Imclone Systems Inc.); antibodies that bind type II mutant EGFR (US Patent No. 5,212,290); humanized and chimeric antibodies that bind EGFR as described in US Patent No. 5,891,996; and human antibodies that bind EGFR (see WO98/50433, Abgenix). The anti-EGFR antibody may be conjugated with a cyotoxic agent, thus generating an immunoconjugate (see, *e.g.*, EP659,439A2, Merck Patent GmbH). Examples of small molecules that bind to EGFR include ZD1839 (Astra Zeneca), CP-358774 (OSI/Pfizer) and AG1478.

An "anti-angiogenic agent" refers to a compound which blocks, or interferes to some degree, the development of blood vessels. The anti-angiogenic factor may, for instance, be a small molecule or antibody that binds to a growth factor or growth factor receptor involved in promoting angiogenesis. The preferred anti-angiogenic factor herein is an antibody that binds to Vascular Endothelial Growth Factor (VEGF).

The term "prodrug" as used in this application refers to a precursor or derivative form of a pharmaceutically active substance that is less cytotoxic to tumor cells compared to the parent drug and is capable of being enzymatically activated or converted into the more active parent form. See, *e.g.*, Wilman, "Prodrugs in Cancer Chemotherapy" *Biochemical Society Transactions,* 14, pp. 375-382, 615th Meeting Belfast (1986) and Stella *et al.,* "Prodrugs: A Chemical Approach to Targeted Drug Delivery," *Directed Drug Delivery,* Borchardt *et al.,* (ed.), pp. 247-267, Humana Press (1985). The prodrugs of this invention include, but are not limited to, phosphate-containing prodrugs, thiophosphate-containing prodrugs, sulfate-containing prodrugs, peptide-containing prodrugs, D-amino acid-modified prodrugs, glycosylated prodrugs, β-lactam-containing prodrugs, optionally substituted phenoxyacetamide-containing prodrugs or optionally substituted phenylacetamide-containing prodrugs, 5-fluorocytosine and other 5-fluorouridine prodrugs which can be converted into the more active cytotoxic free drug. Examples of cytotoxic drugs that can be derivatized into a prodrug form for use in this invention include, but are not limited to, those chemotherapeutic agents described above.

A "liposome" is a small vesicle composed of various types of lipids, phospholipids and/or surfactant which is useful for delivery of a drug (such as the anti-ErbB2 antibodies disclosed herein and, optionally, a chemotherapeutic agent) to a mammal. The components of the liposome are commonly arranged in a bilayer formation, similar to the lipid arrangement of biological membranes.

The term "package insert" is used to refer to instructions customarily included in commercial packages of therapeutic products, that contain information about the indications, usage, dosage, administration, contraindications and/or warnings concerning the use of such therapeutic products.

A "cardioprotectant" is a compound or composition which prevents or reduces myocardial dysfunction (*i.e.* cardiomyopathy and/or congestive heart failure) associated with administration of a drug, such as an anthracycline antibiotic and/or an anti-ErbB2 antibody, to a patient. The cardioprotectant may, for example, block or reduce a free-radical-mediated cardiotoxic effect and/or prevent or reduce oxidative-stress injury. Examples of cardioprotectants encompassed by the present definition include the iron-chelating agent dexrazoxane (ICRF-187) (Seifert *et al. The Annals of Pharmacotherapy* 28:1063-1072 (1994)); a lipid-lowering agent and/or anti-oxidant such as probucol (Singal *et al. J. Mol. Cell Cardiol.* 27:1055-1063 (1995)); amifostine (aminothiol 2-[(3-aminopropyl)amino]ethanethiol-dihydrogen phosphate ester, also called WR-2721, and the dephosphorylated cellular uptake form thereof called WR-1065) and S-3-(3-methylaminopropylamino)propylphosphorothioic acid (WR-151327), see Green *et al. Cancer Research* 54:738-741 (1994); digoxin (Bristow, M.R. In: Bristow MR, ed. *Drug-Induced Heart Disease.* New York: Elsevier 191-215 (1980)); beta-blockers such as metoprolol (Hjalmarson *et al. Drugs* 47:Suppl 4:31-9 (1994); and Shaddy *et al. Am. Heart J.* 129: 197-9 (1995)); vitamin E; ascorbic acid (vitamin C); free radical scavengers such as oleanolic acid, ursolic acid and N-acetylcysteine (NAC); spin trapping compounds such as alpha-phenyl-tert-butyl nitrone (PBN); (Paracchini *et al., Anticancer Res.* 13:1607-1612(1993)); selenoorganic compounds such as P251 (Elbesen); and the like.

## II. Production of anti-ErbB2 Antibodies

A description follows as to exemplary techniques for the production of the antibodies used in accordance with the present invention. The ErbB2 antigen to be used for production of antibodies may be, *e.g.*, a soluble form of the extracellular domain of ErbB2 or a portion thereof, containing the desired epitope. Alternatively, cells expressing ErbB2 at their cell surface (*e.g.* NIH-3T3 cells transformed to overexpress ErbB2; or a carcinoma cell line such as SKBR3 cells, see Stancovski *et al. PNAS (USA)* 88:8691-8695 (1991)) can be used to generate antibodies. Other forms of ErbB2 useful for generating antibodies will be apparent to those skilled in the art.

*(i) Polyclonal antibodies*

Polyclonal antibodies are preferably raised in animals by multiple subcutaneous (sc) or intraperitoneal (ip) injections of the relevant antigen and an adjuvant. It may be useful to conjugate the relevant antigen to a protein that is immunogenic in the species to be immunized, *e.g.*, keyhole limpet hemocyanin, serum albumin, bovine thyroglobulin, or soybean trypsin inhibitor using a bifunctional orderivatizing agent, for example, maleimidobenzoyl sulfosuccinimide ester (conjugation through cysteine residues), N-hydroxysuccinimide (through lysine residues), glutaraldehyde, succinic anhydride, $SOCl_2$, or $R^1N=C=NR$, where R and $R^1$ are different alkyl groups.

Animals are immunized against the antigen, immunogenic conjugates, or derivatives by combining, *e.g.*, 100 μg or 5 μg of the protein or conjugate (for rabbits or mice, respectively) with 3 volumes of Freund's complete adjuvant and injecting the solution intradermally at multiple sites. One month later the animals are boosted with 1/5 to 1/10 the original amount of peptide or conjugate in Freund's complete adjuvant by subcutaneous injection at multiple sites. Seven to 14 days later the animals are bled and the serum is assayed for antibody titer. Animals are boosted until the titer plateaus. Preferably, the animal is boosted with the conjugate of the same antigen, but conjugated to a different protein and/or through a different cross-linking reagent. Conjugates also can be made in recombinant cell culture as protein fusions. Also, aggregating agents such as alum are suitably used to enhance the immune response.

(ii) *Monoclonal antibodies*

Monoclonal antibodies are obtained from a population of substantially homogeneous antibodies, *i.e.,* the individual antibodies comprising the population are identical except for possible naturally occurring mutations that may be present in minor amounts. Thus, the modifier "monoclonal" indicates the character of the antibody as not being a mixture of discrete antibodies.

For example, the monoclonal antibodies may be made using the hybridoma method first described by Kohler *et al., Nature,* 256:495 (1975), or may be made by recombinant DNA methods (U.S. Patent No. 4,816,567).

In the hybridoma method, a mouse or other appropriate host animal, such as a hamster, is immunized as hereinabove described to elicit lymphocytes that produce or are capable of producing antibodies that will specifically bind to the protein used for immunization. Alternatively, lymphocytes may be immunized *in vitro.* Lymphocytes then are fused with myeloma cells using a suitable fusing agent, such as polyethylene glycol, to form a hybridoma cell (Goding, *Monoclonal Antibodies: Principles and Practice,* pp.59-103 (Academic Press, 1986)).

The hybridoma cells thus prepared are seeded and grown in a suitable culture medium that preferably contains one or more substances that inhibit the growth or survival of the unfused, parental myeloma cells. For example, if the parental myeloma cells lack the enzyme hypoxanthine guanine phosphoribosyl transferase (HGPRT or HPRT), the culture medium for the hybridomas typically will include hypoxanthine, aminopterin, and thymidine (HAT medium), which substances prevent the growth of HGPRT-deficient cells.

Preferred myeloma cells are those that fuse efficiently, support stable high-level production of antibody by the selected antibody-producing cells, and are sensitive to a medium such as HAT medium. Among these, preferred myeloma cell lines are murine myeloma lines, such as those derived from MOPC-21 and MPC-11 mouse tumors available from the Salk Institute Cell Distribution Center, San Diego, California USA, and SP-2 or X63-Ag8-653 cells available from the American Type Culture Collection, Rockville, Maryland USA. Human myeloma and mouse-human heteromyeloma cell lines also have been described for the production of human monoclonal antibodies (Kozbor, *J. Immunol.,* 133:3001 (1984); and Brodeur *et al., Monoclonal Antibody Production Techniques and Applications,* pp. 51-63 (Marcel Dekker, Inc., New York, 1987)).

Culture medium in which hybridoma cells are growing is assayed for production of monoclonal antibodies directed against the antigen. Preferably, the binding specificity of monoclonal antibodies produced by hybridoma cells is determined by immunoprecipitation or by an *in vitro* binding assay, such as radioimmunoassay (RIA) or enzyme-linked immunoabsorbent assay (ELISA).

The binding affinity of the monoclonal antibody can, for example, be determined by the Scatchard analysis of Munson *et al., Anal. Biochem.,* 107:220 (1980).

After hybridoma cells are identified that produce antibodies of the desired specificity, affinity, and/or activity, the clones may be subcloned by limiting dilution procedures and grown by standard methods (Goding, *Monoclonal Antibodies: Principles and Practice,* pp.59-103 (Academic Press, 1986)). Suitable culture media for this purpose include, for example, D-MEM or RPMI-1640 medium. In addition, the hybridoma cells may be grown *in vivo* as ascites tumors in an animal.

The monoclonal antibodies secreted by the subclones are suitably separated from the culture medium, ascites fluid, or serum by conventional antibody purification procedures such as, for example, protein A-Sepharose, hydroxylapatite chromatography, gel electrophoresis, dialysis, or affinity chromatography.

DNA encoding the monoclonal antibodies is readily isolated and sequenced using conventional procedures (*e.g.*, by using oligonucleotide probes that are capable of binding specifically to genes encoding the heavy and light chains of murine antibodies). The hybridoma cells serve as a preferred source of such DNA. Once isolated, the DNA may be placed into expression vectors, which are then transfected into host cells such as *E. coli* cells, simian COS cells, Chinese Hamster Ovary (CHO) cells, or myeloma cells that do not otherwise produce antibody protein, to obtain the synthesis of monoclonal antibodies in the recombinant host cells. Review articles on recombinant expression in bacteria of DNA encoding the antibody include Skerra *et al., Curr. Opinion in Immunol.,* 5:256-262 (1993) and Plückthun, *Immunol. Revs.,* 130:151-188 (1992).

In a further embodiment, monoclonal antibodies or antibody fragments can be isolated from antibody phage libraries generated using the techniques described in McCafferty *et al., Nature,* 348:552-554 (1990). Clackson *et al., Nature,* 352:624-628 (1991) and Marks *et al., J. Mol. Biol.,* 222:581-597 (1991) describe the isolation of murine and human antibodies, respectively, using phage libraries. Subsequent publications describe the production of high affinity (nM range) human antibodies by chain shuffling (Marks *et al., Bio/Technology,* 10:779-783 (1992)), as well as combinatorial infection and *in vivo* recombination as a strategy for constructing very large phage libraries (Waterhouse *et al., Nuc. Acids. Res.,* 21:2265-2266 (1993)). Thus, these techniques are viable alternatives to traditional monoclonal antibody hybridoma techniques for isolation of monoclonal antibodies.

The DNA also may be modified, for example, by substituting the coding sequence for human heavy chain and light chain constant domains in place of the homologous murine sequences (U.S. Patent No. 4,816,567; and Morrison, *et al., Proc. Natl Acad. Sci. USA,* 81:6851 (1984)), or by covalently joining to the immunoglobulin coding sequence all or part of the coding sequence for a non-immunoglobulin polypeptide.

Typically such non-immunoglobulin polypeptides are substituted for the constant domains of an antibody, or they are substituted for the variable domains of one antigen-combining site of an antibody to create a chimeric bivalent antibody comprising one antigen-combining site having specificity for an antigen and another antigen-combining site having specificity for a different antigen.

*(iii) Humanized antibodies*

Methods for humanizing non-human antibodies have been described in the art. Preferably, a humanized antibody has one or more amino acid residues introduced into it from a source which is non-human. These non-human amino acid residues are often referred to as "import" residues, which are typically taken from an "import" variable domain. Humanization can be essentially performed following the method of Winter and co-workers (Jones *et al., Nature,* 321: 522-525 (1986); Riechmann *et al., Nature,* 332:323-327 (1988); Verhoeyen *et al., Science,* 239:1534-1536 (1988)), by substituting hypervariable region sequences for the corresponding sequences of a human antibody. Accordingly, such "humanized" antibodies are chimeric antibodies (U.S. Patent No. 4,816,567) wherein substantially less than an intact human variable domain has been substituted by the corresponding sequence from a non-human species. In practice, humanized antibodies are typically human antibodies in which some hypervariable region residues and possibly some FR residues are substituted by residues from analogous sites in rodent antibodies.

The choice of human variable domains, both light and heavy, to be used in making the humanized antibodies is very important to reduce antigenicity. According to the so-called "best-fit" method, the sequence of the variable domain of a rodent antibody is screened against the entire library of known human variable-domain sequences. The human sequence which is closest to that of the rodent is then accepted as the human framework region (FR) for the humanized antibody (Sims *et al., J. Immunol.,* 151:2296 (1993); Chothia *et al., J. Mol. Biol.,* 196:901 (1987)). Another method uses a particular framework region derived from the consensus sequence of all human antibodies of a particular subgroup of light or heavy chains. The same framework may be used for several different humanized antibodies (Carter *et al., Proc. Natl. Acad. Sci. USA,* 89:4285 (1992); Presta *et al., J. Immunol.,* 151:2623 (1993)).

It is further important that antibodies be humanized with retention of high affinity for the antigen and other favorable biological properties. To achieve this goal, according to a preferred method, humanized antibodies are prepared by a process of analysis of the parental sequences and various conceptual humanized products using three-dimensional models of the parental and humanized sequences. Three-dimensional immunoglobulin models are commonly available and are familiar to those skilled in the art. Computer programs are available which illustrate and display probable three-dimensional conformational structures of selected candidate immunoglobulin sequences. Inspection of these displays permits analysis of the likely role of the residues in the functioning of the candidate immunoglobulin sequence, *i.e.,* the analysis of residues that influence the ability of the candidate immunoglobulin to bind its antigen. In this way, FR residues can be selected and combined from the recipient and import sequences so that the desired antibody characteristic, such as increased affinity for the target antigen(s), is achieved. In general, the hypervariable region residues are directly and most substantially involved in influencing antigen binding.

Example 3 below describes production of exemplary humanized anti-ErbB2 antibodies which bind ErbB2 and block ligand activation of an ErbB receptor. The humanized antibody of particular interest herein blocks EGF, TGF-α and/or HRG mediated activation of MAPK essentially as effectively as murine monoclonal antibody 2C4 (or a Fab fragment thereof) and/or binds ErbB2 essentially as effectively as murine monoclonal antibody 2C4 (or a Fab fragment thereof). The humanized antibody herein may, for example, comprise nonhuman hypervariable region residues incorporated into a human variable heavy domain and may further comprise a framework region (FR) substitution at a position selected from the group consisting of 69H, 71H, and 73H, utilizing the variable domain numbering system set forth in Kabat *et al., Sequences of Proteins of Immunological Interest,* 5th Ed. Public Health Service, National Institutes of Health, Bethesda, MD (1991). In one embodiment, the humanized antibody comprises FR substitutions at two or all of positions 69H, 71H and 73H.

An exemplary humanized antibody of interest herein comprises variable heavy domain complementarity determining residues GFTFRDYTMX, where X is preferably D or S (SEQ ID NO:7), DVNPNSGGSIYNQRFKG (SEQ ID NO:8); and/or NLGPSFYFDY (SEQ ID NO:9), optionally comprising amino acid modifications of those CDR residues, *e.g.* where the modifications essentially maintain or improve affinity of the antibody. For example, the antibody variant of interest may have from about one to about seven or about five amino acid substitutions in the above variable heavy CDR sequences. Such antibody variants may be prepared by affinity maturation, *e.g.,* as described below. The most preferred humanized antibody comprises the variable heavy domain amino acid sequence in SEQ ID NO:4.

The humanized antibody may comprise variable light domain complementarity determining residues KASQDVSIGVA (SEQ ID NO:10), SASYX$^1$X$^2$X$^3$, where X$^1$ is preferably R or L; X$^2$ is preferably Y or E; and X$^3$ is preferably T or S (SEQ ID NO:11); and QQYYIYPYT (SEQ ID NO:12), *e.g.* in addition to those variable heavy domain CDR residues in the preceding paragraph. Such humanized antibodies optionally comprise amino acid modifications of the above CDR residues, *e.g.* where the modifications essentially maintain or improve affinity of the antibody. For example, the antibody variant of interest may have from about one to about seven or about five amino acid substitutions in the above variable light CDR sequences. Such antibody variants may be prepared by affinity maturation, *e.g.*, as described below. The most preferred humanized antibody comprises the variable light domain amino acid sequence in SEQ ID NO:3.

The present application also contemplates affinity matured antibodies which antibodies which bind ErbB2 and block ligand activation of an ErbB receptor. The parent antibody may be a human antibody or a humanized antibody, *e.g.,* one comprising the variable light and/or heavy sequences of SEQ ID Nos. 3 and 4, respectively (*i.e.* variant 574). The affinity matured antibody preferably binds to ErbB2 receptor with an affinity superior to that of murine 2C4 or variant 574 (*e.g.* from about two or about four fold, to about 100 fold or about 1000 fold improved affinity, *e.g.* as assessed using a ErbB2-extracellular domain (ECD) ELISA) . Exemplary variable heavy CDR residues for substitution include H28, H30, H34, H35, H64, H96, H99, or combinations of two or more (*e.g.* two three, four, five, six or seven of these residues). Examples of variable light CDR residues for alteration include L28, L50, L53, L56, L91, L92, L93, L94, L96, L97 or combinations of two or more (*e.g.* two to three, four, five or up to about ten of these residues).

Various forms of the humanized or affinity matured antibody are contemplated. For example, the humanized or affinity matured antibody may be an antibody fragment, such as a Fab, which is optionally conjugated with one or more cytotoxic agent(s) in order to generate an immunoconjugate. Alternatively, the humanized or affinity matured antibody may be an intact antibody, such as an intact IgG1 antibody.

*(iv) Human antibodies*

As an alternative to humanization, human antibodies can be generated. For example, it is now possible to produce transgenic animals (*e.g.,* mice) that are capable, upon immunization, of producing a full repertoire of human antibodies in the absence of endogenous immunoglobulin production. For example, it has been described that the homozygous deletion of the antibody heavy-chain joining region (J$_H$) gene in chimeric and germ-line mutant mice results in complete inhibition of endogenous antibody production. Transfer of the human germ-line immunoglobulin gene array in such germ-line mutant mice will result in the production of human antibodies upon antigen challenge. See, *e.g.,* Jakobovits *et al., Proc. Natl. Acad. Sci. USA,* 90:2551 (1993); Jakobovits *et al., Nature,* 362:255-258 (1993); Bruggermann *et al., Year in Immuno.,* 7:33 (1993); and U.S. Patent Nos. 5,591,669, 5,589,369 and 5,545, 807.

Alternatively, phage display technology (McCafferty *et al., Nature* 348:552-553 (1990)) can be used to produce human antibodies and antibody fragments *in vitro,* from immunoglobulin variable (V) domain gene repertoires from unimmunized donors. According to this technique, antibody V domain genes are cloned in-frame into either a major or minor coat protein gene of a filamentous bacteriophage, such as M 13 or fd, and displayed as functional antibody fragments on the surface of the phage particle. Because the filamentous particle contains a single-stranded DNA copy of the phage genome, selections based on the functional properties of the antibody also result in selection of the gene encoding the antibody exhibiting those properties. Thus, the phage mimics some of the properties of the B-cell. Phage display can be performed in a variety of formats; for their review see, *e.g.,* Johnson, Kevin S. and Chiswell, David J., *Current Opinion in Structural Biology* 3:564-571 (1993). Several sources of V-gene segments can be used for phage display. Clackson *et al., Nature,* 352:624-628 (1991) isolated a diverse array of anti-oxazolone antibodies from a small random combinatorial library of V genes derived from the spleens of immunized mice. A repertoire of V genes from unimmunized human donors can be constructed and antibodies to a diverse array of antigens (including self-antigens) can be isolated essentially following the techniques described by Marks *et al., J. Mol. Biol.* 222:581-597 (1991), or Griffith *et al., EMBO J.* 12:725-734 (1993). See, also, U.S. Patent Nos. 5,565,332 and 5,573,905.

As discussed above, human antibodies may also be generated by *in vitro* activated B cells (see U.S. Patents 5,567,610 and 5,229,275).

Human anti-ErbB2 antibodies are described in U.S. Patent No. 5,772,997 issued June 30, 1998 and WO 97/00271 published January 3, 1997.

*(v) Antibody fragments*

Various techniques have been developed for the production of antibody fragments. Traditionally, these fragments were derived via proteolytic digestion of intact antibodies (see, *e.g.,* Morimoto *et al., Journal of Biochemical and Biophysical Methods* 24:107-117 (1992); and Brennan *et al., Science,* 229:81 (1985)). However, these fragments can now be produced directly by recombinant host cells. For example, the antibody fragments can be isolated from the antibody phage libraries discussed above. Alternatively, Fab'-SH fragments can be directly recovered from *E. coli* and chemically coupled to form F(ab')$_2$ fragments (Carter *et al.,* Bio/Technology 10:163-167 (1992)). According to another

approach, F(ab')$_2$ fragments can be isolated directly from recombinant host cell culture. Other techniques for the production of antibody fragments will be apparent to the skilled practitioner. In other embodiments, the antibody of choice is a single chain Fv fragment (scFv). See WO 93/16185; U.S. Patent No. 5,571,894; and U.S. Patent No. 5,587,458. The antibody fragment may also be a "linear antibody", *e.g.*, as described in U.S. Patent 5,641,870 for example. Such linear antibody fragments may be monospecific or bispecific.

*(vi) Bispecific antibodies*

Bispecific antibodies are antibodies that have binding specificities for at least two different epitopes. Exemplary bispecific antibodies may bind to two different epitopes of the ErbB2 protein. Other such antibodies may combine an ErbB2 binding site with binding site(s) for EGFR, ErbB3 and/or ErbB4. Alternatively, an anti-ErbB2 arm may be combined with an arm which binds to a triggering molecule on a leukocyte such as a T-cell receptor molecule (*e.g.* CD2 or CD3), or Fc receptors for IgG (FcγR), such as FcγRI (CD64), FcγRII (CD32) and FcγRIII (CD 16) so as to focus cellular defense mechanisms to the ErbB2-expressing cell. Bispecific antibodies may also be used to localize cytotoxic agents to cells which express ErbB2. These antibodies possess an ErbB2-binding arm and an arm which binds the cytotoxic agent (*e.g.* saporin, anti-interferon-α, vinca alkaloid, ricin A chain, methotrexate or radioactive isotope hapten). Bispecific antibodies can be prepared as full length antibodies or antibody fragments (*e.g.* F(ab')$_2$ bispecific antibodies).

WO 96/16673 describes a bispecific anti-ErbB2/anti-FcγRIII antibody and U.S. Patent No. 5,837,234 discloses a bispecific anti-ErbB2/anti-FcγRI antibody. A bispecific anti-ErbB2/Fcα antibody is shown in WO98/02463. U.S. Patent No. 5,821,337 teaches a bispecific anti-ErbB2/anti-CD3 antibody.

Methods for making bispecific antibodies are known in the art. Traditional production of full length bispecific antibodies is based on the coexpression of two immunoglobulin heavy chain-light chain pairs, where the two chains have different specificities (Millstein *et al., Nature,* 305:537-539 (1983)). Because of the random assortment of immunoglobulin heavy and light chains, these hybridomas (quadromas) produce a potential mixture of 10 different antibody molecules, of which only one has the correct bispecific structure. Purification of the correct molecule, which is usually done by affinity chromatography steps, is rather cumbersome, and the product yields are low. Similar procedures are disclosed in WO 93/08829, and in Traunecker *et al., EMBO J.,* 10:3655-3659 (1991).

According to a different approach, antibody variable domains with the desired binding specificities (antibody-antigen combining sites) are fused to immunoglobulin constant domain sequences. The fusion preferably is with an immunoglobulin heavy chain constant domain, comprising at least part of the hinge, CH2, and CH3 regions. It is preferred to have the first heavy-chain constant region (CH1) containing the site necessary for light chain binding, present in at least one of the fusions. DNAs encoding the immunoglobulin heavy chain fusions and, if desired, the immunoglobulin light chain, are inserted into separate expression vectors, and are co-transfected into a suitable host organism. This provides for great flexibility in adjusting the mutual proportions of the three polypeptide fragments in embodiments when unequal ratios of the three polypeptide chains used in the construction provide the optimum yields. It is, however, possible to insert the coding sequences for two or all three polypeptide chains in one expression vector when the expression of at least two polypeptide chains in equal ratios results in high yields or when the ratios are of no particular significance.

In a preferred embodiment of this approach, the bispecific antibodies are composed of a hybrid immunoglobulin heavy chain with a first binding specificity in one arm, and a hybrid immunoglobulin heavy chain-light chain pair (providing a second binding specificity) in the other arm. It was found that this asymmetric structure facilitates the separation of the desired bispecific compound from unwanted immunoglobulin chain combinations, as the presence of an immunoglobulin light chain in only one half of the bispecific molecule provides for a facile way of separation. This approach is disclosed in WO 94/04690. For further details of generating bispecific antibodies see, for example, Suresh *et al., Methods in Enzymology,* 121:210 (1986).

According to another approach described in U.S. Patent No. 5,731,168, the interface between a pair of antibody molecules can be engineered to maximize the percentage of heterodimers which are recovered from recombinant cell culture. The preferred interface comprises at least a part of the $C_H3$ domain of an antibody constant domain. In this method, one or more small amino acid side chains from the interface of the first antibody molecule are replaced with larger side chains (*e.g.* tyrosine or tryptophan). Compensatory "cavities" of identical or similar size to the large side chain(s) are created on the interface of the second antibody molecule by replacing large amino acid side chains with smaller ones (*e.g.* alanine or threonine). This provides a mechanism for increasing the yield of the heterodimer over other unwanted end-products such as homodimers.

Bispecific antibodies include cross-linked or "heteroconjugate" antibodies. For example, one of the antibodies in the heteroconjugate can be coupled to avidin, the other to biotin. Such antibodies have, for example, been proposed to target immune system cells to unwanted cells (U.S. Patent No. 4,676,980), and for treatment of HIV infection (WO 91/00360, WO 92/200373, and EP 03089). Heteroconjugate antibodies may be made using any convenient cross-linking methods. Suitable cross-linking agents are well known in the art, and are disclosed in U.S. Patent No. 4,676,980, along with a number of cross-linking techniques.

Techniques for generating bispecific antibodies from antibody fragments have also been described in the literature. For example, bispecific antibodies can be prepared using chemical linkage. Brennan *et al., Science,* 229: 81 (1985) describe a procedure wherein intact antibodies are proteolytically cleaved to generate F(ab')$_2$ fragments. These fragments are reduced in the presence of the dithiol complexing agent sodium arsenite to stabilize vicinal dithiols and prevent intermolecular disulfide formation. The Fab' fragments generated are then converted to thionitrobenzoate (TNB) derivatives. One of the Fab'-TNB derivatives is then reconverted to the Fab'-thiol by reduction with mercaptoethylamine and is mixed with an equimolar amount of the other Fab'-TNB derivative to form the bispecific antibody. The bispecific antibodies produced can be used as agents for the selective immobilization of enzymes.

Recent progress has facilitated the direct recovery of Fab'-SH fragments from *E. coli,* which can be chemically coupled to form bispecific antibodies. Shalaby *et al., J. Exp. Med.,* 175: 217-225 (1992) describe the production of a fully humanized bispecific antibody F(ab)$_2$ molecule. Each Fab' fragment was separately secreted from *E. coli* and subjected to directed chemical coupling *in vitro* to form the bispecific antibody. The bispecific antibody thus formed was able to bind to cells overexpressing the ErbB2 receptor and normal human T cells, as well as trigger the lytic activity of human cytotoxic lymphocytes against human breast tumor targets.

Various techniques for making and isolating bispecific antibody fragments directly from recombinant cell culture have also been described. For example, bispecific antibodies have been produced using leucine zippers. Kostelny *et al., J. Immunol.,* 148(5):1547-1553 (1992). The leucine zipper peptides from the Fos and Jun proteins were linked to the Fab' portions of two different antibodies by gene fusion. The antibody homodimers were reduced at the hinge region to form monomers and then re-oxidized to form the antibody heterodimers. This method can also be utilized for the production of antibody homodimers. The "diabody" technology described by Hollinger *et al., Proc. Natl. Acad. Sci. USA,* 90:6444-6448 (1993) has provided an alternative mechanism for making bispecific antibody fragments. The fragments comprise a heavy-chain variable domain ($V_H$) connected to a light-chain variable domain ($V_L$) by a linker which is too short to allow pairing between the two domains on the same chain. Accordingly, the $V_H$ and $V_L$ domains of one fragment are forced to pair with the complementary $V_L$ and $V_H$ domains of another fragment, thereby forming two antigen-binding sites. Another strategy for making bispecific antibody fragments by the use of single-chain Fv (sFv) dimers has also been reported. See Gruber *et al., J. Immunol.,* 152:5368 (1994).

Antibodies with more than two valencies are contemplated. For example, trispecific antibodies can be prepared. Tutt *et al. J. Immunol.* 147: 60 (1991).

*(vii) Other amino acid sequence modifications*

Amino acid sequence modification(s) of the anti-ErbB2 antibodies described herein are contemplated. For example, it may be desirable to improve the binding affinity and/or other biological properties of the antibody. Amino acid sequence variants of the anti-ErbB2 antibody are prepared by introducing appropriate nucleotide changes into the anti-ErbB2 antibody nucleic acid, or by peptide synthesis. Such modifications include, for example, deletions from, and/or insertions into and/or substitutions of, residues within the amino acid sequences of the anti-ErbB2 antibody. Any combination of deletion, insertion, and substitution is made to arrive at the final construct, provided that the final construct possesses the desired characteristics. The amino acid changes also may alter post-translational processes of the anti-ErbB2 antibody, such as changing the number or position of glycosylation sites.

A useful method for identification of certain residues or regions of the anti-ErbB2 antibody that are preferred locations for mutagenesis is called "alanine scanning mutagenesis" as described by Cunningham and Wells *Science,* 244:1081-1085 (1989). Here, a residue or group of target residues are identified (*e.g.,* charged residues such as arg, asp, his, lys, and glu) and replaced by a neutral or negatively charged amino acid (most preferably alanine or polyalanine) to affect the interaction of the amino acids with ErbB2 antigen. Those amino acid locations demonstrating functional sensitivity to the substitutions then are refined by introducing further or other variants at, or for, the sites of substitution. Thus, while the site for introducing an amino acid sequence variation is predetermined, the nature of the mutation *per se* need not be predetermined. For example, to analyze the performance of a mutation at a given site, ala scanning or random mutagenesis is conducted at the target codon or region and the expressed anti-ErbB2 antibody variants are screened for the desired activity.

Amino acid sequence insertions include amino- and/or carboxyl-terminal fusions ranging in length from one residue to polypeptides containing a hundred or more residues, as well as intrasequence insertions of single or multiple amino acid residues. Examples of terminal insertions include an anti-ErbB2 antibody with an N-terminal methionyl residue or the antibody fused to a cytotoxic polypeptide. Other insertional variants of the anti-ErbB2 antibody molecule include the fusion to the N- or C-terminus of the anti-ErbB2 antibody to an enzyme (e.g. for ADEPT) or a polypeptide which increases the serum half-life of the antibody.

Another type of variant is an amino acid substitution variant. These variants have at least one amino acid residue in the anti-ErbB2 antibody molecule replaced by a different residue. The sites of greatest interest for substitutional mutagenesis include the hypervariable regions, but FR alterations are also contemplated. Conservative substitutions are shown in Table I under the heading of "preferred substitutions". If such substitutions result in a change in biological activity, then more substantial changes, denominated "exemplary substitutions" in Table 1, or as further described below in reference to amino acid classes, may be introduced and the products screened.

**Table 1**

| Original Residue | Exemplary Substitutions | Preferred Substitutions |
|---|---|---|
| Ala (A) | val; leu; ile | val |
| Arg (R) | lys; gln; asn | lys |
| Asn (N) | gln; his; asp, lys; arg | gln |
| Asp (D) | glu; asn | glu |
| Cys (C) | ser; ala | ser |
| Gln (Q) | asn; glu | asn |
| Glu (E) | asp; gln | asp |
| Gly (G) | ala | ala |
| His (H) | asn; gin; lys; arg | arg |
| Ile (I) | leu; val; met; ala; phe; norleucine | leu |
| Leu (L) | norleucine; ile; val; met; ala; phe | ile |
| Lys (K) | arg; gin; asn | arg |
| Met (M) | leu; phe; ile | leu |
| Phe (F) | leu; val; ile; ala; tyr | tyr |
| Pro (P) | ala | ala |
| Ser (S) | thr | thr |
| Thr(T) | ser | ser |
| Trp (W) | tyr; phe | tyr |
| Tyr (Y) | trp; phe; thr; ser | phe |
| Val (V) | ile; leu; met; phe; ala; norleucine | leu |

Substantial modifications in the biological properties of the antibody are accomplished by selecting substitutions that differ significantly in their effect on maintaining (a) the structure of the polypeptide backbone in the area of the substitution, for example, as a sheet or helical conformation, (b) the charge or hydrophobicity of the molecule at the target site, or (c) the bulk of the side chain. Naturally occurring residues are divided into groups based on common side-chain properties:

(1) hydrophobic: norleucine, met, ala, val, leu, ile;
(2) neutral hydrophilic: cys, ser, thr;
(3) acidic: asp, glu;
(4) basic: asn, gln, his, lys, arg;
(5) residues that influence chain orientation: gly, pro; and
(6) aromatic: trp, tyr, phe.

Non-conservative substitutions will entail exchanging a member of one of these classes for another class.

Any cysteine residue not involved in maintaining the proper conformation of the anti-ErbB2 antibody also may be substituted, generally with serine, to improve the oxidative stability of the molecule and prevent aberrant crosslinking. Conversely, cysteine bond(s) may be added to the antibody to improve its stability (particularly where the antibody is an antibody fragment such as an Fv fragment).

A particularly preferred type of substitutional variant involves substituting one or more hypervariable region residues of a parent antibody (*e.g.* a humanized or human antibody). Generally, the resulting variant(s) selected for further development will have improved biological properties relative to the parent antibody from which they are generated.

A convenient way for generating such substitutional variants involves affinity maturation using phage display. Briefly, several hypervariable region sites (*e.g.* 6-7 sites) are mutated to generate all possible amino substitutions at each site. The antibody variants thus generated are displayed in a monovalent fashion from filamentous phage particles as fusions to the gene III product of M 13 packaged within each particle. The phage-displayed variants are then screened for their biological activity (*e.g.* binding affinity) as herein disclosed. In order to identify candidate hypervariable region sites for modification, alanine scanning mutagenesis can be performed to identify hypervariable region residues contributing significantly to antigen binding. Alternatively, or additionally, it may be beneficial to analyze a crystal structure of the antigen-antibody complex to identify contact points between the antibody and human ErbB2. Such contact residues and neighboring residues are candidates for substitution according to the techniques elaborated herein. Once such variants are generated, the panel of variants is subjected to screening as described herein and antibodies with superior properties in one or more relevant assays may be selected for further development.

Another type of amino acid variant of the antibody alters the original glycosylation pattern of the antibody. By altering is meant deleting one or more carbohydrate moieties found in the antibody, and/or adding one or more glycosylation sites that are not present in the antibody.

Glycosylation of antibodies is typically either N-linked or O-linked. N-linked refers to the attachment of the carbohydrate moiety to the side chain of an asparagine residue. The tripeptide sequences asparagine-X-serine and asparagine-X-threonine, where X is any amino acid except proline, are the recognition sequences for enzymatic attachment of the carbohydrate moiety to the asparagine side chain. Thus, the presence of either of these tripeptide sequences in a polypeptide creates a potential glycosylation site. O-linked glycosylation refers to the attachment of one of the sugars N-aceylgalactosamine, galactose, or xylose to a hydroxyamino acid, most commonly serine or threonine, although 5-hydroxyproline or 5-hydroxylysine may also be used.

Addition of glycosylation sites to the antibody is conveniently accomplished by altering the amino acid sequence such that it contains one or more of the above-described tripeptide sequences (for N-linked glycosylation sites). The alteration may also be made by the addition of, or substitution by, one or more serine or threonine residues to the sequence of the original antibody (for O-linked glycosylation sites).

Nucleic acid molecules encoding amino acid sequence variants of the anti-ErbB2 antibody are prepared by a variety of methods known in the art. These methods include, but are not limited to, isolation from a natural source (in the case of naturally occurring amino acid sequence variants) or preparation by oligonucleotide-mediated (or site-directed) mutagenesis, PCR mutagenesis, and cassette mutagenesis of an earlier prepared variant or a non-variant version of the anti-ErbB2 antibody.

It may be desirable to modify the antibody of the invention with respect to effector function, e.g. so as to enhance antigen-dependent cell-mediated cyotoxicity (ADCC) and/or complement dependent cytotoxicity (CDC) of the antibody. This may be achieved by introducing one or more amino acid substitutions in an Fc region of the antibody. Alternatively or additionally, cysteine residue(s) may be introduced in the Fc region, thereby allowing interchain disulfide bond formation in this region. The homodimeric antibody thus generated may have improved internalization capability and/or increased complement-mediated cell killing and antibody-dependent cellular cytotoxicity (ADCC). See Caron *et al., J. Exp Med.* 176:1191-1195 (1992) and Shopes, B. *J. Immunol.* 148:2918-2922 (1992). Homodimeric antibodies with enhanced anti-tumor activity may also be prepared using heterobifunctional cross-linkers as described in Wolff *et al. Cancer Research* 53:2560-2565 (1993). Alternatively, an antibody can be engineered which has dual Fc regions and may thereby have enhanced complement lysis and ADCC capabilities. See Stevenson *et al. Anti-Cancer Drug Design* 3:219-230 (1989).

To increase the serum half life of the antibody, one may incorporate a salvage receptor binding epitope into the antibody (especially an antibody fragment) as described in U.S. Patent 5,739,277, for example. As used herein, the term "salvage receptor binding epitope" refers to an epitope of the Fc region of an IgG molecule (*e.g.*, $IgG_1$, $IgG_2$, $IgG_3$, or $IgG_4$) that is responsible for increasing the *in vivo* serum half-life of the IgG molecule.

*(viii) Screening for antibodies with the desired properties*

Techniques for generating antibodies have been described above. One may further select antibodies with certain biological characteristics, as desired.

To identify an antibody which blocks ligand activation of an ErbB receptor, the ability of the antibody to block ErbB ligand binding to cells expressing the ErbB receptor (*e.g.* in conjugation with another ErbB receptor with which the ErbB receptor of interest forms an ErbB hetero-oligomer) may be determined. For example, cells naturally expressing, or transfected to express, ErbB receptors of the ErbB hetero-oligomer may be incubated with the antibody and then exposed to labeled ErbB ligand. The ability of the anti-ErbB2 antibody to block ligand binding to the ErbB receptor in the ErbB hetero-oligomer may then be evaluated.

For example, inhibition of HRG binding to MCF7 breast tumor cell lines by anti-ErbB2 antibodies may be performed using monolayer MCF7 cultures on ice in a 24-well-plate format essentially as described in Example 1 below. Anti-ErbB2 monoclonal antibodies may be added to each well and incubated for 30 minutes. $^{125}$I-labeled $rHRG\beta1_{177-224}$

(25 pm) may then be added, and the incubation may be continued for 4 to 16 hours. Dose response curves may be prepared and an $IC_{50}$ value may be calculated for the antibody of interest. In one embodiment, the antibody which blocks ligand activation of an ErbB receptor will have an $IC_{50}$ for inhibiting HRG binding to MCF7 cells in this assay of about 50nM or less, more preferably 10nM or less. Where the antibody is an antibody fragment such as a Fab fragment, the $IC_{50}$ for inhibiting HRG binding to MCF7 cells in this assay may, for example, be about 100nM or less, more preferably 50nM or less.

Alternatively, or additionally, the ability of the anti-ErbB2 antibody to block ErbB ligand-stimulated tyrosine phosphorylation of an ErbB receptor present in an ErbB hetero-oligomer may be assessed. For example, cells endogenously expressing the ErbB receptors or transfected to expressed them may be incubated with the antibody and then assayed for ErbB ligand-dependent tyrosine phosphorylation activity using an antiphosphotyrosine monoclonal (which is optionally conjugated with a detectable label). The kinase receptor activation assay described in U.S. Patent No. 5,766,863 is also available for determining ErbB receptor activation and blocking of that activity by an antibody.

In one embodiment, one may screen for an antibody which inhibits HRG stimulation of p180 tyrosine phosphorylation in MCF7 cells essentially as described in Example 1 below. For example, the MCF7 cells may be plated in 24-well plates and monoclonal antibodies to ErbB2 may be added to each well and incubated for 30 minutes at room temperature; then $rHRG\beta1_{177\text{-}244}$ may be added to each well to a final concentration of 0.2 nM, and the incubation may be continued for 8 minutes. Media may be aspirated from each well, and reactions may be stopped by the addition of 100 µl of SDS sample buffer (5% SDS, 25 mM DTT, and 25 mM Tris-HCl, pH 6.8). Each sample (25 µl) may be electrophoresed on a 4-12% gradient gel (Novex) and then electrophoretically transferred to polyvinylidene difluoride membrane. Antiphosphotyrosine (at 1 µg/ml) immunoblots may be developed, and the intensity of the predominant reactive band at $M_r \sim 180,000$ may be quantified by reflectance densitometry. The antibody selected will preferably significantly inhibit stimulation of p180 tyrosime phosphorylation to about 0-35% of control in this assay. A dose-response curve for inhibition of HRG stimulation of p180 tyrosine phosphorylation as determined by reflectance densitometry may be prepared and an $IC_{50}$ for the antibody of interest may be calculated. In one embodiment, the antibody which blocks ligand activation of an ErbB receptor will have an $IC_{50}$ for inhibiting HRG stimulation of p180 tyrosine phosphorylation in this assay of about 50nM or less, more preferably 10nM or less. Where the antibody is an antibody fragment such as a Fab fragment, the $IC_{50}$ for inhibiting HRG stimulation of p180 tyrosine phosphorylation in this assay may, for example, be about 100nM or less, more preferably 50nM or less.

One may also assess the growth inhibitory effects of the antibody on MDA-MB-175 cells, *e.g,* essentially as described in Schaefer *et al. Oncogene* 15:1385-1394 (1997). According to this assay, MDA-MB-175 cells may treated with an anti-ErbB2 monoclonal antibody (10µg/mL) for 4 days and stained with crystal violet. Incubation with an anti-ErbB2 antibody may show a growth inhibitory effect on this cell line similar to that displayed by monoclonal antibody 2C4. In a further embodiment, exogenous HRG will not significantly reverse this inhibition. Preferably, the antibody will be able to inhibit cell proliferation of MDA-MB-175 cells to a greater extent than monoclonal antibody 4D5 (and optionally to a greater extent than monoclonal antibody 7F3), both in the presence and absence of exogenous HRG.

In one embodiment, the anti-ErbB2 antibody of interest may block heregulin dependent association of ErbB2 with ErbB3 in both MCF7 and SK-BR-3 cells as determined in a co-immunoprecipitation experiment such as that described in Example 2 substantially more effectively than monoclonal antibody 4D5 and, optionally, substantially more effectively than monoclonal antibody 7F3.

Alternatively, or additionally, one may determine the ability of the antibody to block EGF, TGF-$\alpha$ and/or HRG mediated activation of mitogen-activated protein kinase (MAPK), *e.g.,* as shown in Example 4 below. An antibody which blocks EGF, TGF-$\alpha$ and/or HRG mediated activation of mitogen-activated protein kinase (MAPK) to a greater extent than HERCEPTIN® or monoclonal antibody 4D5 may be selected. Moreover, the antibody of interest may block EGF, TGF-$\alpha$ and/or HRG mediated activation of mitogen-activated protein kinase (MAPK) to a greater extent than monoclonal antibody 7F3.

To identify growth inhibitory anti-ErbB2 antibodies, one may screen for antibodies which inhibit the growth of cancer cells which overexpress ErbB2. In one embodiment, the growth inhibitory antibody of choice is able to inhibit growth of SK-BR-3 cells in cell culture by about 20-100% and preferably by about 50-100% at an antibody concentration of about 0.5 to 30 µg/ml. To identify such antibodies, the SK-BR-3 assay described in U.S. Patent No. 5,677,171 can be performed. According to this assay, SK-BR-3 cells are grown in a 1:1 mixture of F12 and DMEM medium supplemented with 10% fetal bovine serum, glutamine and penicillin streptomycin. The SK-BR-3 cells are plated at 20,000 cells in a 35mm cell culture dish (2mls/35mm dish). 0.5 to 30 µg/ml of the anti-ErbB2 antibody is added per dish. After six days, the number of cells, compared to untreated cells are counted using an electronic COULTER™ cell counter. Those antibodies which inhibit growth of the SK-BR-3 cells by about 20-100% or about 50-100% may be selected as growth inhibitory antibodies.

To select for antibodies which induce cell death, loss of membrane integrity as indicated by, *e.g.*, PI, trypan blue or 7AAD uptake may be assessed relative to control. The preferred assay is the PI uptake assay using BT474 cells. According to this assay, BT474 cells (which can be obtained from the American Type Culture Collection (Rockville, MD)) are cultured in Dulbecco's Modified Eagle Medium (D-MEM):Ham's F-12 (50:50) supplemented with 10% heat-inactivated FBS (Hyclone) and 2 mM L-glutamine. (Thus, the assay is performed in the absence of complement and immune effector cells). The BT474 cells are seeded at a density of $3 \times 10^6$ per dish in 100 x 20 mm dishes and allowed to attach overnight. The medium is then removed and replaced with fresh medium alone or medium containing 10μg/ml of the appropriate monoclonal antibody. The cells are incubated for a 3 day time period. Following each treatment, monolayers are washed with PBS and detached by trypsinization. Cells are then centrifuged at 1200rpm for 5 minutes at 4°C, the pellet resuspended in 3 ml ice cold $Ca^{2+}$ binding buffer (10 mM Hepes, pH 7.4, 140 mM NaCl, 2.5 mM $CaCl_2$) and aliquoted into 35 mm strainer-capped 12 x 75 tubes (1ml per tube, 3 tubes per treatment group) for removal of cell clumps. Tubes then receive PI (10μg/ml). Samples may be analyzed using a FACSCAN™ flow cytometer and FACSCONVERT™ CellQuest software (Becton Dickinson). Those antibodies which induce statistically significant levels of cell death as determined by PI uptake may be selected as cell death-inducing antibodies.

In order to select for antibodies which induce apoptosis, an annexin binding assay using BT474 cells is available. The BT474 cells are cultured and seeded in dishes as discussed in the preceding paragraph. The medium is then removed and replaced with fresh medium alone or medium containing 10μg/ml of the monoclonal antibody. Following a three day incubation period, monolayers are washed with PBS and detached by trypsinization. Cells are then centrifuged, resuspended in $Ca^{2+}$ binding buffer and aliquoted into tubes as discussed above for the cell death assay. Tubes then receive labeled annexin (e.g. annexin V-FTIC) (1μg/ml). Samples may be analyzed using a FACSCAN™ flow cytometer and FACSCONVERT™ CellQuest software (Becton Dickinson). Those antibodies which induce statistically significant levels of annexin binding relative to control are selected as apoptosis-inducing antibodies.

In addition to the annexin binding assay, a DNA staining assay using BT474 cells is available. In order to perform this assay, BT474 cells which have been treated with the antibody of interest as described in the preceding two paragraphs are incubated with 9μg/ml HOECHST 33342™ for 2 hr at 37°C, then analyzed on an EPICS ELITE™ flow cytometer (Coulter Corporation) using MODFIT LT™ software (Verity Software House). Antibodies which induce a change in the percentage of apoptotic cells which is 2 fold or greater (and preferably 3 fold or greater) than untreated cells (up to 100% apoptotic cells) may be selected as pro-apoptotic antibodies using this assay.

To screen for antibodies which bind to an epitope on ErbB2 bound by an antibody of interest, a routine cross-blocking assay such as that described in *Antibodies, A Laboratory Manual,* Cold Spring Harbor Laboratory, Ed Harlow and David Lane (1988), can be performed. Alternatively, or additionally, epitope mapping can be performed by methods known in the art (see, e.g. Figs. 1A and 1B herein).

*(ix) Immunoconjugates*

The invention also pertains to therapy with immunoconjugates comprising an antibody conjugated to a cytotoxic agent such as a chemotherapeutic agent, toxin (e.g. a small molecule toxin or an enzymatically active toxin of bacterial, fungal, plant or animal origin, including fragments and/or variants thereof), or a radioactive isotope (i.e., a radioconjugate).

Chemotherapeutic agents useful in the generation of such immunoconjugates have been described above.

Conjugates of an antibody and one or more small molecule toxins, such as a caliche amicin, a maytansine (U.S. Patent No. 5,208,020), a trichothene, and CC1065 are also contemplated herein.

In one preferred embodiment of the invention, the antibody is conjugated to one or more maytansine molecules (e.g. about 1 to about 10 maytansine molecules per antibody molecule). Maytansine may, for example, be converted to May-SS-Me which may be reduced to May-SH3 and reacted with modified antibody (Chari et al. Cancer Research 52: 127-131 (1992)) to generate a maytansinoid-antibody immunoconjugate.

Another immunoconjugate of interest comprises an anti-ErbB2 antibody conjugated to one or more calicheamicin molecules. The calicheamicin family of antibiotics are capable of producing double-stranded DNA breaks at sub-picomolar concentrations. Structural analogues of calicheamicin which may be used include, but are not limited to, $\gamma_1^I$, $\alpha_2^I$, $\alpha_3^I$, N-acetyl-$\gamma_1^I$, PSAG and $\theta_1^I$ (Hinman et al. Cancer Research 53: 3336-3342 (1993) and Lode et al. Cancer Research 58: 2925-2928 (1998)).

Enzymatically active toxins and fragments thereof which can be used include diphtheria A chain, nonbinding active fragments of diphtheria toxin, exotoxin A chain (from *Pseudomonas aeruginosa*), ricin A chain, abrin A chain, modeccin A chain, alpha-sarcin, *Aleurites fordii* proteins, dianthin proteins, *Phytolaca americana* proteins (PAPI, PAPII, and PAP-S), momordica charantia inhibitor, curcin, crotin, sapaonaria officinalis inhibitor, gelonin, mitogellin, restrictocin, phenomycin, enomycin and the tricothecenes. See, for example, WO 93/21232 published October 28, 1993.

The present invention further contemplates an immunoconjugate formed between an antibody and a compound with nucleolytic activity (e.g. a ribonuclease or a DNA endonuclease such as a deoxyribonuclease; DNase).

A variety of radioactive isotopes are available for the production of radioconjugated anti-ErbB2 antibodies. Examples include $At^{211}$, $I^{131}$, $I^{125}$, $Y^{90}$, $Re^{186}$, $Re^{188}$, $Sm^{153}$, $Bi^{212}$, $P^{32}$ and radioactive isotopes of Lu.

Conjugates of the antibody and cytotoxic agent may be made using a variety of bifunctional protein coupling agents such as N-succinimidyl-3-(2-pyridyldithiol) propionate (SPDP), succinimidyl-4-(N-maleimidomethyl) cyclohexane-1-carboxylate, iminothiolane (IT), bifunctional derivatives of imidoesters (such as dimethyl adipimidate HCL), active esters (such as disuccinimidyl suberate), aldehydes (such as glutareldehyde), bis-azido compounds (such as bis (p-azidobenzoyl) hexanediamine), bis-diazonium derivatives (such as bis-(p-diazoniumbenzoyl)-ethylenediamine), diisocyanates (such as tolyene 2,6-diisocyanate), and bis-active fluorine compounds (such as 1,5-difluoro-2,4-dinitrobenzene). For example, a ricin immunotoxin can be prepared as described in Vitetta *et al. Science* 238: 1098 (1987). Carbon-14-labeled 1-isothiocyanatobenzyl-3-methyldiethylene triaminepentaacetic acid (MX-DTPA) is an exemplary chelating agent for conjugation of radionucleotide to the antibody. See WO94/11026. The linker may be a "cleavable linker" facilitating release of the cytotoxic drug in the cell. For example, an acid-labile linker, peptidase-sensitive linker, dimethyl linker or disulfide-containing linker (Chari *et al. Cancer Research* 52: 127-131 (1992)) may be used.

Alternatively, a fusion protein comprising the anti-ErbB2 antibody and cytotoxic agent may be made, *e.g.* by recombinant techniques or peptide synthesis.

In yet another embodiment, the antibody may be conjugated to a "receptor" (such streptavidin) for utilization in tumor pretargeting wherein the antibody-receptor conjugate is administered to the patient, followed by removal of unbound conjugate from the circulation using a clearing agent and then administration of a "ligand" (*e.g.* avidin) which is conjugated to a cytotoxic agent (*e.g.* a radionucleotide).

*(x) Antibody Dependent Enzyme Mediated Prodrug Therapy (ADEPT)*

The antibodies of the present invention may also be used in ADEPT by conjugating the antibody to a prodrug-activating enzyme which converts a prodrug (*e.g.* a peptidyl chemotherapeutic agent, see WO81/01145) to an active anti-cancer drug. See, for example, WO 88/07378 and U.S. Patent No. 4,975,278.

The enzyme component of the immunoconjugate useful for ADEPT includes any enzyme capable of acting on a prodrug in such a way so as to covert it into its more active, cytotoxic form.

Enzymes that are useful in the method of this invention include, but are not limited to, alkaline phosphatase useful for converting phosphate-containing prodrugs into free drugs; arylsulfatase useful for converting sulfate-containing prodrugs into free drugs; cytosine deaminase useful for converting non-toxic 5-fluorocytosine into the anti-cancer drug, 5-fluorouracil; proteases, such as serratia protease, thermolysin, subtilisin, carboxypeptidases and cathepsins (such as cathepsins B and L), that are useful for converting peptide-containing prodrugs into free drugs; D-alanylcarboxypeptidases, useful for converting prodrugs that contain D-amino acid substituents; carbohydrate-cleaving enzymes such as β-galactosidase and neuraminidase useful for converting glycosylated prodrugs into free drugs; β-lactamase useful for converting drugs derivatized with β-lactams into free drugs; and penicillin amidases, such as penicillin V amidase or penicillin G amidase, useful for converting drugs derivatized at their amine nitrogens with phenoxyacetyl or phenylacetyl groups, respectively, into free drugs. Alternatively, antibodies with enzymatic activity, also known in the art as "abzymes", can be used to convert the prodrugs of the invention into free active drugs (see, *e.g.,* Massey, *Nature* 328: 457-458 (1987)). Antibody-abzyme conjugates can be prepared as described herein for delivery of the abzyme to a tumor cell population.

The enzymes of this invention can be covalently bound to the anti-ErbB2 antibodies by techniques well known in the art such as the use of the heterobifunctional crosslinking reagents discussed above. Alternatively, fusion proteins comprising at least the antigen binding region of an antibody of the invention linked to at least a functionally active portion of an enzyme of the invention can be constructed using recombinant DNA techniques well known in the art (see, *e.g.,* Neuberger *et al., Nature,* 312: 604-608 (1984).

*(xi) Other antibody modifications*

Other modifications of the antibody are contemplated herein. For example, the antibody may be linked to one of a variety of nonproteinaceous polymers, *e.g.,* polyethylene glycol, polypropylene glycol, polyoxyalkylenes, or copolymers of polyethylene glycol and polypropylene glycol. The antibody also may be entrapped in microcapsules prepared, for example, by coacervation techniques or by interfacial polymerization (for example, hydroxymethylcellulose or gelatin-microcapsules and poly-(methylmethacylate) microcapsules, respectively), in colloidal drug delivery systems (for example, liposomes, albumin microspheres, microemulsions, nano-particles and nanocapsules), or in macroemulsions. Such techniques are disclosed in *Remington's Pharmaceutical Sciences,* 16th edition, Oslo, A., Ed., (1980).

The anti-ErbB2 antibodies disclosed herein may also be formulated as immunoliposomes. Liposomes containing the antibody are prepared by methods known in the art, such as described in Epstein *et al., Proc. Natl. Acad. Sci. USA,* 82:3688 (1985); Hwang *et al., Proc. Natl Acad. Sci. USA,* 77:4030 (1980); U.S. Pat. Nos. 4,485,045 and 4,544,545; and WO97/38731 published October 23, 1997. Liposomes with enhanced circulation time are disclosed in U.S. Patent No. 5,013,556.

Particularly useful liposomes can be generated by the reverse phase evaporation method with a lipid composition comprising phosphatidylcholine, cholesterol and PEG-derivatized phosphatidylethanolamine (PEG-PE). Liposomes are extruded through filters of defined pore size to yield liposomes with the desired diameter. Fab' fragments of the antibody of the present invention can be conjugated to the liposomes as described in Martin *et al. J. Biol. Chem.* 257: 286-288 (1982) via a disulfide interchange reaction. A chemotherapeutic agent is optionally contained within the liposome. See Gabizon *et al. J. National Cancer Inst.* 81(19)1484 (1989).

## III. Pharmaceutical Formulations

Therapeutic formulations of the antibodies used in accordance with the present invention are prepared for storage by mixing an antibody having the desired degree of purity with optional pharmaceutically acceptable carriers, excipients or stabilizers (*Remington's Pharmaceutical Sciences* 16th edition, Osol, A. Ed. (1980)), in the form of lyophilized formulations or aqueous solutions. Acceptable carriers, excipients, or stabilizers are nontoxic to recipients at the dosages and concentrations employed, and include buffers such as phosphate, citrate, and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (*e.g.* Zn-protein complexes); and/or nonionic surfactants such as TWEEN™, PLURONICS™ or polyethylene glycol (PEG). Preferred lyophilized anti-ErbB2 antibody formulations are described in WO 97/04801, expressly incorporated herein by reference.

The formulation herein may also contain more than one active compound as necessary for the particular indication being treated, preferably those with complementary activities that do not adversely affect each other. For example, it may be desirable to further provide antibodies which bind to EGFR, ErbB2 (*e.g.* an antibody which binds a different epitope on ErbB2), ErbB3, ErbB4, or vascular endothelial factor (VEGF) in the one formulation. Alternatively, or additionally, the composition may further comprise a chemotherapeutic agent, cytotoxic agent, cytokine, growth inhibitory agent, anti-hormonal agent, and/or cardioprotectant. Such molecules are suitably present in combination in amounts that are effective for the purpose intended.

The active ingredients may also be entrapped in microcapsules prepared, for example, by coacervation techniques or by interfacial polymerization, for example, hydroxymethylcellulose or gelatin-microcapsules and poly-(methylmethacylate) microcapsules, respectively, in colloidal drug delivery systems (for example, liposomes, albumin microspheres, microemulsions, nano-particles and nanocapsules) or in macroemulsions. Such techniques are disclosed in *Remington's Pharmaceutical Sciences* 16th edition, Osol, A. Ed. (1980).

Sustained-release preparations may be prepared. Suitable examples of sustained-release preparations include semipermeable matrices of solid hydrophobic polymers containing the antibody, which matrices are in the form of shaped articles, *e.g.* films, or microcapsules. Examples of sustained-release matrices include polyesters, hydrogels (for example, poly(2-hydroxyethyl-methacrylate), or poly(vinylalcohol)), polylactides (U.S. Pat. No. 3,773,919), copolymers of L-glutamic acid and γ ethyl-L-glutamate, non-degradable ethylene-vinyl acetate, degradable lactic acid-glycolic acid copolymers such as the LUPRON DEPOT™ (injectable microspheres composed of lactic acid-glycolic acid copolymer and leuprolide acetate), and poly-D-(-)-3-hydroxybutyric acid.

The formulations to be used for *in vivo* administration must be sterile. This is readily accomplished by filtration through sterile filtration membranes.

## IV. Treatment with the Anti-ErbB2 Antibodies

According to the present invention, the anti-ErbB2 antibody is used for the manufacture of a medicament to treat prostate cancer, such as androgen independent prostate cancer or androgen dependent prostate cancer. Where the cancer to be treated is androgen independent or dependent prostate cancer, expression of the androgen (*e.g.* andosterone or testosterone) and/or its cognate receptor in the tumor may be assessed using any of the various assays available, *e.g.* as described above. Alternatively, or additionally, a patient may be diagnosed as having androgen independent prostate cancer in that they no longer respond to anti-androgen therapy and the patient diagnosed as having androgen dependent prostate cancer may be one who responds to anti-androgen therapy. The cancer will generally comprise ErbB2-expressing cells, such that the anti-ErbB2 antibody is able to bind thereto. While the cancer may be characterized by overexpression of the ErbB2 receptor, the present application further provides a method for treating cancer which is not considered to be an ErbB2-overexpressing cancer. To determine ErbB2 expression in the cancer, various diagnostic/prognostic assays are available. In one embodiment. ErbB2 overexpression may be analyzed by IHC, *e.g.* using the HERCEPTEST® (Dako). Parrafin embedded tissue sections from a tumor biopsy may be subjected to the IHC assay and accorded a ErbB2 protein staining intensity criteria as follows:

Score 0      no staining is observed or membrane staining is observed in less than 10% of tumor cells.

Score 1+      a faint/barely perceptible membrane staining is detected in more than 10% of the tumor cells. The cells are only stained in part of their membrane.

Score 2+      a weak to moderate complete membrane staining is observed in more than 10% of the tumor cells.

Score 3+      a moderate to strong complete membrane staining is observed in more than 10% of the tumor cells.

Those tumors with 0 or 1+ scores for ErbB2 overexpression assessment may be characterized as not overexpressing ErbB2. whereas those tumors with 2+ or 3+ scores may be characterized as overexpressing ErbB2.

Alternatively, or additionally, FISH assays such as the INFORM™ (sold by Ventana, Arizona) or PATHVISION™ (Vysis, Illinois) may be carried out on formalin-fixed, paraffin-embedded tumor tissue to determine the extent (if any) of ErbB2 overexpression in the tumor.

The prostate cancer to be treated herein may be one characterized by excessive activation of an ErbB receptor, *e.g.* EGFR. Such excessive activation may be attributable to overexpression or increased production of the ErbB receptor or of an ErbB ligand. In one embodiment of the invention, a diagnostic or prognostic assay will be performed to determine whether the patient's cancer is characterized by excessive activation of an ErbB receptor. For example, ErbB gene amplification and/or overexpression of an ErbB receptor in the cancer may be determined. Various assays for determining such amplification/overexpression are available in the art and include the IHC, FISH and shed antigen assays described above. Alternatively, or additionally, levels of an ErbB ligand, such as TGF-$\alpha$, in or associated with the tumor may be determined according to known procedures. Such assays may detect protein and/or nucleic acid encoding it in the sample to be tested. In one embodiment. ErbB ligand levels in the tumor may be determined using immunohistochemistry (IHC): see, for example. Scher *et al. Clin. Cancer Research* 1:545-550(1995). Alternatively, or additionally, one may evaluate levels of ErbB ligand-encoding nucleic acid in the sample to be tested: *e.g.* via FISH, southern blotting, or PCR techniques.

Moreover, ErbB receptor or ErbB ligand overexpression or amplification may be evaluated using an *in vivo* diagnostic assay, *e.g.* by administering a molecule (such as an antibody) which binds the molecule to be detected and is tagged with a detectable label (*e.g.* a radioactive isotope) and externally scanning the patient for localization of the label.

In certain embodiments, an immunoconjugate comprising the anti-ErbB2 antibody conjugated with a cytotoxic agent is administered to the patient. Preferably, the immunoconjugate and/or ErbB2 protein to which it is bound is/are internalized by the cell, resulting in increased therapeutic efficacy of the immunoconjugate in killing the cancer cell to which it binds. In a preferred embodiment, the cytotoxic agent targets or interferes with nucleic acid in the cancer cell. Examples of such cytotoxic agents include maytansinoids, calicheamicins, ribonucleases ribonucleases and DNA endonucleases.

The anti-ErbB2 antibodies or immunoconjugates are administered to a human patient, in accord with known methods, such as intravenous administration. *e.g.,* as a bolus or by continuous infusion over a period of time, by intramuscular, intraperitoneal, intracerobrospinal, subcutaneous, intra-articular, intrasynovial, intrathecal, oral, topical, or inhalation routes. Intravenous or subcutaneous administration of the antibody is preferred.

Other therapeutic regimens may be combined with the administration of the anti-ErbB2 antibody. The combined administration includes coadministration, using separate formulations or a single pharmaceutical formulation, and consecutive administration in either order, wherein preferably there is a time period white both (or all) active agents simultaneously exert their biological activities.

In one preferred embodiment, the patient is treated with two different anti-ErbB2 antibodies. For example, the patient may be treated with a first anti-ErbB2 antibody which blocks ligand activation of an ErbB receptor 50-100% more effectively than humanised monoclonal antibody huMab4D5-8 as disclosed in US patent 5, 821, 337 or an antibody having a biological characteristic of monoclonal antibody 2C4 as well as a second anti-ErbB2 antibody which is growth inhibitory (*e.g.* HERCEPTIN®) or an anti-ErbB2 antibody which induces apoptosis of an ErbB2-overexpressing cell (*e.g.* 7C2,7F3 or humanized variants thereof). Preferably such combined therapy results in a synergistic therapeutic effect.

It may also be desirable to combine administration of the anti-ErbB2 antibody or antibodies, with administration of an antibody directed against another tumor associated antigen. The other antibody in this case may, for example, bind to EGFR, ErbB3, ErbB4, or vascular endothelial growth factor (VEGF).

In one embodiment, the present invention involves use of an anti-ErbB2 antibody (or antibodies) and one or more chemotherapeutic agents or growth inhibitory agents, including coadministration of cocktails of different chemotherapeutic agents. Preferred chemotherapeutic agents include taxanes (such as paclitaxel and docetaxel) and/or anthracycline antibiotics. Preparation and dosing schedules for such chemotherapeutic agents may be used according to manufacturers' instructions or as determined empirically by the skilled practitioner. Preparation and dosing schedules for such chemotherapy are also described in Chemotherapy Service Ed., M.C. Perry. Williams & Wilkins, Baltimore, MD (1992).

The antibody may be combined with an anti-hormonat compound; *e.g.,* an anti-estrogen compound such as tamoxifen; an anti-progesterone such as onapristone (see. EP 616 812); or an anti-androgen such as flutamide, in dosages known for such molecules. Where the cancer to be treated is androgen independent cancer, the patient may previously have been subjected to anti-androgen therapy and, after the cancer becomes androgen independent, the anti-ErbB2 antibody (and optionally other agents as described herein) may be administered to the patient.

Sometimes, it may be beneficial to also coadminister a cardioprotectant (to prevent or reduce myocardial dysfunction associated with the therapy) or one or more cytokines to the patient. In addition to the above therapeutic regimes, the patient may be subjected to surgical removal of cancer cells and/or radiation therapy. Suitable dosages for any of the above coadministered agents are those presently used and may be lowered due to the combined action (synergy) of the agent and anti-ErbB2 antibody.

For the prevention or treatment of disease, the appropriate dosage of antibody will depend on the type of disease to be treated, as defined above, the severity and course of the disease, whether the antibody is administered for preventive or therapeutic purposes, previous therapy, the patient's clinical history and response to the antibody, and the discretion of the attending physician. The antibody is suitably administered to the patient at one time or over a series of treatments. Depending on the type and severity of the disease, about 1 $\mu$g/kg to 15 mg/kg (*e.g.* 0.1-20mg/kg) of antibody is an initial candidate dosage for administration to the patient, whether, for example, by one or more separate administrations, or by continuous infusion. A typical daily dosage might range from about I $\mu$g/kg to 100 mg/kg or more, depending on the factors mentioned above. For repeated administrations over several days or longer, depending on the condition, the treatment is sustained until a desired suppression of disease symptoms occurs. A preferred dosing regimen comprises administering an initial loading dose of about 4 mg/kg, followed by a weekly maintenance dose of about 2 mg/kg of the anti-ErbB2 antibody. However, other dosage regimens may be useful. The progress of this therapy is easily monitored by conventional techniques and assays.

Antibody protein can be administered to the patient, or the antibody can be administered by gene therapy. See, for example, WO96/07321 published March 14, 1996 concerning the use of gene therapy to generate intracellular antibodies.

There are two major approaches to getting the nucleic acid (optionally contained in a vector) into the patient's cells; *in vivo* and *ex vivo.* For *in vivo* delivery the nucleic acid is injected directly into the patient, usually at the site where the antibody is required. For *ex vivo* treatment, the patient's cells are removed, the nucleic acid is introduced into these isolated cells and the modified cells are administered to the patient either directly or, for example, encapsulated within porous membranes which are implanted into the patient (see, *e.g.* U.S. Patent Nos. 4,892,538 and 5,283.187). There are a variety of techniques available for introducing nucleic acids into viable cells. The techniques vary depending upon whether the nucleic acid is transferred into cultured cells *in vitro,* or *in vivo* in the cells of the intended host. Techniques suitable for the transfer of nucleic acid into mammalian cells *in vitro* include the use of liposomes, electroporation, microinjection, cell fusion. DEAE-dextran, the calcium phosphate precipitation method, etc. A commonly used vector for *ex vivo* delivery of the gene is a retrovirus.

The currently preferred *in vivo* nucleic acid transfer techniques include transfection with viral vectors (such as adenovirus, Herpes simplex I virus, or adeno-associated virus) and lipid-based systems (useful lipids for lipid-mediated transfer of the gene are DOTMA, DOPE and DC-Chol, for example). In some situations it is desirable to provide the nucleic acid source with an agent that targets the target cells, such as an antibody specific for a cell surface membrane protein or the target cell, a ligand for a receptor on the target cell, etc. Where liposomes are employed, proteins which bind to a cell surface membrane protein associated with endocytosis may be used for targeting and/or to facilitate uptake, *e.g.* capsid proteins or fragments thereof tropic for a particular cell type, antibodies for proteins which undergo internalization in cycling, and proteins that target intracellular localization and enhance intracellular half-life. The technique of receptor-mediated endocytosis is described, for example, by Wu *et al., J. Biol. Chem.* 262:4429-4432 (1987); and Wagner *et al., Proc. Natl. Acad. Sci. USA* 87:3410-3414 (1990). For review of the currently known gene marking and gene therapy Protocols see Anderson *et al., Science* 256:808-813 (1992). See also WO 93/25673 and the references cited therein.

### V. Articles of Manufacture

In another embodiment of the invention, an article of manufacture containing materials useful for the treatment of prostate cancer is provided. The article of manufacture comprises a container and a label or package insert on or associated with the container. Suitable containers include, for example, bottles, vials, syringes, etc. The containers may be formed from a variety of materials such as glass or plastic. The container holds a composition which is effective for treating the condition and may have a sterile access port (for example the container may be an intravenous solution bag or a vial having a stopper pierceable by a hypodermic injection needle). At least one active agent in the composition is the anti-ErbB2 antibody. The label or package insert indicates that the composition is used for treating prostate cancer, androgen independent prostate cancer, or androgen dependent prostate cancer. Moreover, the article of manufacture may comprise (a) a first container with a composition contained therein, wherein the composition comprises a first antibody which binds ErbB2 and inhibits growth of cancer cells which overexpress ErbB2: and (b) a second container with a composition contained therein, wherein the composition comprises a second antibody which binds ErbB2 and blocks ligand activation of an ErbB receptor 50-100% more effectively than humanised monoclonal antibody humMab4D5-8 as disclosed in US patent 5, 821, 337. The article of manufacture in this embodiment of the invention may further comprises a package insert indicating that the first and second antibody compositions can be used to treat prostate cancer. Alternatively, or additionally, the article of manufacture may further comprise a second (or third) container comprising a pharmaceutically-acceptable buffer, such as bacteriostatic water for injection (BWFI), phosphate-buffered saline, Ringer's solution and dextrose solution. It may further include other materials desirable from a commercial and user standpoint, including other buffers, diluents, filters, needles, and syringes.

## VI. Deposit of Materials

The following hybridoma cell lines have been deposited with the American Type Culture Collection, 10801 University Boulevard, Manassas, VA 20110-2209, USA (ATCC):

| Antibody Designation | ATCC No | Deposit Date |
| --- | --- | --- |
| 7C2 | A TCC HB-12215 | October 17, 1996 |
| 7F3 | ATCC HB-1221 | October 17, 1996 |
| 4D5 | ATCC CRL 10463 | May 24.1990 |
| 2C4 | ATCC HB 12697 | April 8, 1999 |

Further details of the invention are illustrated by the following non-limiting Examples.

## Example 1

## Production and Characterization of Monoclonal Antibody 2C4

The murine monoclonal antibodies 2C4, 7F3 and 4D5 which specifically bind the extracellular domain of ErbB2 were produced as described in Fendly *et al., Cancer Research* 50:1550-1558 (1990). Briefly, NIH 3T3/HER2-3$_{400}$ cells (expressing approximately 1 x 10$^5$ ErbB2 molecules/cell) produced as described in Hudziak *et al Proc. Natl. Acad. Sci. (USA)* 84:7158-7163 (1987) were harvested with phosphate buffered saline (PBS) containing 25mM EDTA and used to immunize BALB/c mice. The mice were given injections i.p. of 10$^7$ cells in 0.5ml PBS on weeks 0, 2, 5 and 7. The mice with antisera that immunoprecipitated $^{32}$P-labeled ErbB2 were given i.p. injections of a wheat germ agglutinin-Sepharose (WGA) purified ErbB2 membrane extract on weeks 9 and 13. This was followed by an i.v. injection of 0.1 ml of the ErbB2 preparation and the splenocytes were fused with mouse myeloma line X63-Ag8.653. Hybridoma supernatants were screened for ErbB2-binding by ELISA and radioimmunoprecipitation.

The ErbB2 epitopes bound by monoclonal antibodies 4D5, 7F3 and 2C4 were determined by competitive binding analysis (Fendly *et al. Cancer Research* 50:1550-1558 (1990)). Cross-blocking studies were done on antibodies by direct fluorescence on intact cells by using the PANDEX™ Screen Machine to quantitate fluorescence. Each monoclonal antibody was conjugated with fluorescein isothiocyanate (FITC), using established procedures (Wofsy *et al. Selected Methods in Cellular Immunology,* p. 287, Mishel and Schiigi (eds.) San Francisco: W.J. Freeman Co. (1980)). Confluent monolayers of NIH 3T3/HER2-3$_{400}$ cells were trypsinized, washed once, and resuspended at 1.75 x 10$^6$ cell/ml in cold PBS containing 0.5% bovine serum albumin (BSA) and 0.1% NaN$_3$. A final concentration of 1 % latex particles (IDC, Portland, OR) was added to reduce clogging of the PANDEX™ plate membranes. Cells in suspension, 20 μl, and 20 μl of purified monoclonal antibodies (100μg/ml to 0.1 μg/ml) were added to the PANDEX™ plate wells and incubated on ice for 30 minutes. A predetermined dilution of FITC-labeled monoclonal antibodies in 20μl was added to each well, incubated for 30 minutes, washed, and the fluorescence was quantitated by the PANDEX™. Monoclonal antibodies were considered to share an epitope if each blocked binding of the other by 50% or greater in comparison to an irrelevant monoclonal antibody control. In this experiment, monoclonal antibodies 4D5, 7F3 and 2C4 were assigned epitopes I, G/F and F, respectively.

The growth inhibitory characteristics of monoclonal antibodies 2C4, 7F3 and 4D5 were evaluated using the breast tumor cell line, SK-BR-3 (see Hudziak *et al. Molec. Cell. Biol.* 9(3):1165-1172 (1989)). Briefly, SK-BR-3 cells were detached by using 0.25% (vol/vol) trypsin and suspended in complete medium at a density of 4 x 10$^5$ cells per ml. Aliquots of 100 μl (4 x 10$^4$ cells) were plated into 96-well microdilution plates, the cells were allowed to adhere, and 100 μl of media alone or media containing monoclonal antibody (final concentration 5 μg/ml) was then added. After 72 hours, plates were washed twice with PBS (pH 7.5), stained with crystal violet (0.5% in methanol), and analyzed for relative cell proliferation as described in Sugarman *et al. Science* 230:943-945 (1985). Monoclonal antibodies 2C4 and 7F3 inhibited SK-BR-3 relative cell proliferation by about 20% and about 38%, respectively, compared to about 56% inhibition achieved with monoclonal antibody 4D5.

Monoclonal antibodies 2C4, 4D5 and 7F3 were evaluated for their ability to inhibit HRG-stimulated tyrosine phosphorylation of proteins in the $M_r$ 180,000 range from whole-cell lysates of MCF7 cells (Lewis *et al. Cancer Research* 56:1457-1465 (1996)). MCF7 cells are reported to express all known ErbB receptors, but at relatively low levels. Since ErbB2, ErbB3, and ErbB4 have nearly identical molecular sizes, it is not possible to discern which protein is becoming tyrosine phosphorylated when whole-cell lysates are evaluated by Western blot analysis. However, these cells are ideal for HRG tyrosine phosphorylation assays because under the assay conditions used, in the absence of exogenously added HRG, they exhibit low to undetectable levels of tyrosine phosphorylation proteins in the $M_r$ 180,000 range.

MCF7 cells were plated in 24-well plates and monoclonal antibodies to ErbB2 were added to each well and incubated for 30 minutes at room temperature; then rHRGβ1$_{177-244}$ was added to each well to a final concentration of 0.2 nM, and the incubation was continued for 8 minutes. Media was carefully aspirated from each well, and reactions were stopped by the addition of 100 μl of SDS sample buffer (5% SDS, 25 mM DTT, and 25 mM Tris-HCl, pH 6.8). Each sample (25 μl) was electrophoresed on a 4-12% gradient gel (Novex) and then electrophoretically transferred to polyvinylidene difluoride membrane. Antiphosphotyrosine (4G10, from UBI, used at 1 μg/ml) immunoblots were developed, and the intensity of the predominant reactive band at $M_r$-180,000 was quantified by reflectance densitometry, as described previously (Holmes *et al. Science* 256:1205-1210 (1992); Sliwkowski *et al. J. Biol. Chem.* 269:14661-14665 (1994))

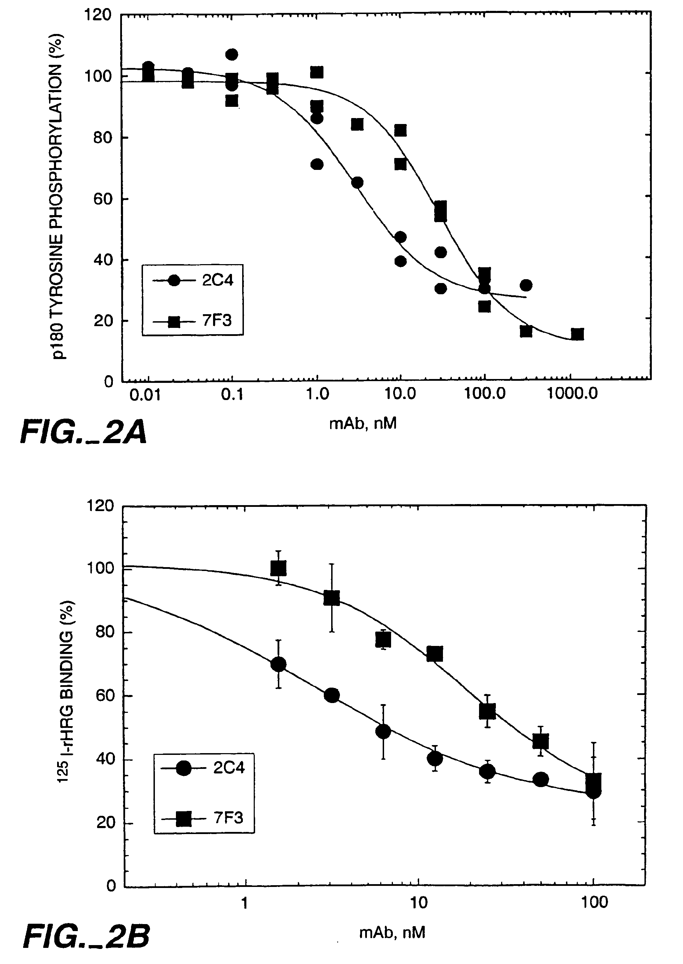

Monoclonal antibodies 2C4, 7F3, and 4D5, significantly inhibited the generation of a HRG-induced tyrosine phosphorylation signal at $M_r$ 180,000. In the absence of HRG, none of these antibodies were able to stimulate tyrosine phosphorylation of proteins in the $M_r$ 180,000 range. Also, these antibodies do not cross-react with EGFR (Fendly *et al. Cancer Research* 50:1550-1558 (1990)), ErbB3, or ErbB4. Antibodies 2C4 and 7F3 significantly inhibited HRG stimulation of p180 tyrosine phosphorylation to <25% of control. Monoclonal antibody 4D5 was able to block HRG stimulation of tyrosine phosphorylation by -50%. Fig. 2A shows dose-response curves for 2C4 or 7F3 inhibition of HRG stimulation of p 180 tyrosine phosphorylation as determined by reflectance densitometry. Evaluation of these inhibition curves using a 4-parameter fit yielded an IC$_{50}$ of 2.8 ± 0.7 nM and 29.0 ± 4.1 nM for 2C4 and 7F3, respectively.

Inhibition of HRG.binding to MCF7 breast tumor cell lines by anti-ErbB2 antibodies was performed with monolayer cultures on ice in a 24-well-plate format (Lewis *et al. Cancer Research* 56:1457-1465 (1996)). Anti-ErbB2 monoclonal antibodies were added to each well and incubated for 30 minutes. $^{125}$I-labeled rHRGβ1$_{177-224}$ (25 pm) was added, and the incubation was continued for 4 to 16 hours. Fig. 2B provides dose-response curves for 2C4 or 7F3 inhibition of HRG binding to MCF7 cells. Varying concentrations of 2C4 or 7F3 were incubated with MCF7 cells in the presence of $^{125}$I-labeled rHRGβ1, and the inhibition curves are shown in Fig. 2B. Analysis of these data yielded an IC$_{50}$ of 2.4 ± 0.3 nM and 19.0 ± 7.3 nM for 2C4 and 7F3, respectively. A maximum inhibition of -74% for 2C4 and 7F3 were in agreement with the tyrosine phosphorylation data.

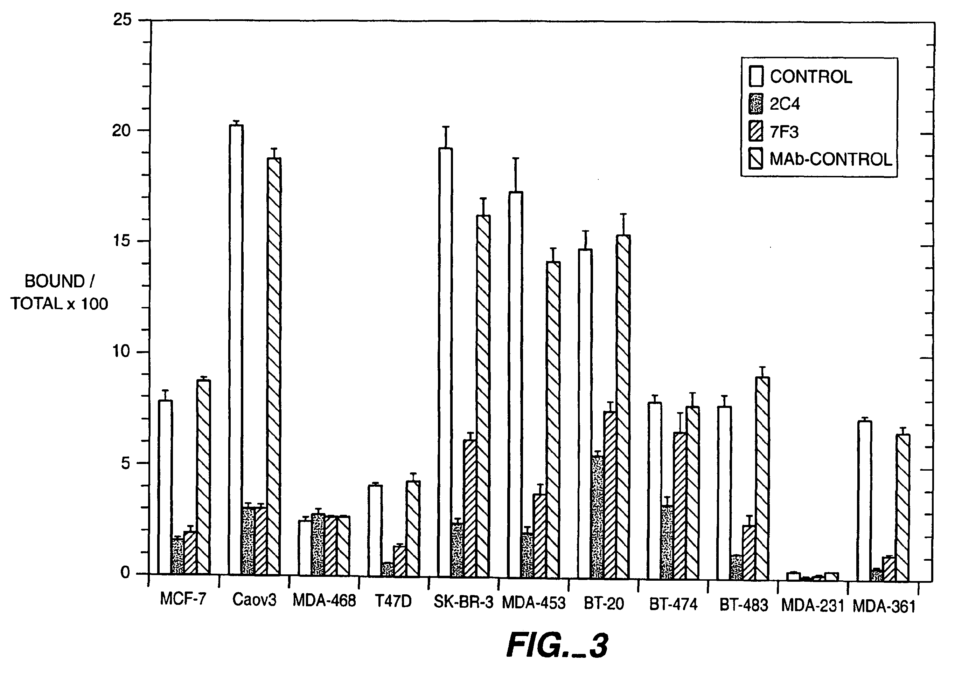

To determine whether the effect of the anti-ErbB2 antibodies observed on MCF7 cells was a general phenomenon, human tumor cell lines were incubated with 2C4 or 7F3 and the degree of specific $^{125}$I-labeled rHRGβ1 binding was determined (Lewis *et al. Cancer Research* 56:1457 1465 (1996)). The results from this study are shown in Fig. 3. Binding of $^{121}$I-labeled rHRGβ1 could be significantly inhibited by either 2C4 or 7F3 in all cell lines, with the exception of the breast cancer cell line MDA-MB-468, which has been reported to express little or no ErbB2. The remaining cell lines are reported to express ErbB2, with the level of ErbB2 expression varying widely among these cell lines. In fact, the range of ErbB2 expression in the cell lines tested varies by more than 2 orders of magnitude. For example, BT-20, MCF7, and Caov3 express ~ $10^4$ ErbB2 receptors/cell, whereas BT-474 and SK-BR-3 express ~$10^6$ ErbB2 receptors/cell. Given the wide range of ErbB2 expression in these cells and the data above, it was concluded that the interaction between ErbB2 and ErbB3 or ErbB4, was itself a high-affinity interaction that takes place on the surface of the plasma membrane.

The growth inhibitory effects of monoclonal antibodies 2C4 and 4D5 on MDA-MB-175 and SK-BR-3 cells in the presence or absence of exogenous rHRGβ1 was assessed (Schaefer *et al. Oncogene* 15:1385-1394 (1997)). ErbB2 levels in MDA-MB-175 cells are 4-6 times higher than the level found in normal breast epithelial cells and the ErbB2-ErbB4 receptor is constitutively tyrosine phosphorylated in MDA-MB-175 cells. MDA-MB-175 cells were treated with an anti-ErbB2 monoclonal antibodies 2C4 and 4D5 (10μg/mL) for 4 days. In a crystal violet staining assay, incubation with 2C4 showed a strong growth inhibitory effect on this cell line (Fig. 4A). Exogenous HRG did not significantly reverse this inhibition. On the other hand 2C4 revealed no inhibitory effect on the ErbB2 overexpressing cell line SK-BR-3 (Fig. 4B). Monoclonal antibody 2C4 was able to inhibit cell proliferation of MDA-MB-175 cells to a greater extent than monoclonal antibody 4D5, both in the presence and absence of exogenous HRG. Inhibition of cell proliferation by 4D5 is dependent on the ErbB2 expression level (Lewis *et al. Cancer Immunol. Immunother.* 37:255-263 (1993)). A maximum inhibition of 66% in SK-BR-3 cells could be detected (Fig.4B). However this effect could be overcome by exogenous HRG.

### Example 2

**HRG Dependent Association of ErbB2 with ErbB3 is Blocked by Monoclonal Antibody 2C4**

The ability of ErbB3 to associate with ErbB2 was tested in a co-immunoprecipition experiment. $1.0 \times 10^6$ MCF7 or SK-BR-3 cells were seeded in six well tissue culture plates in 50:50 DMEM/Ham's F12 medium containing 10% fetal bovine serum (FBS) and 10 mM HEPES, pH 7.2 (growth medium), and allowed to attach overnight. The cells were starved for two hours in growth medium without serum prior to beginning the experiment

The cells were washed briefly with phosphate buffered saline (PBS) and then incubated with either 100 nM of the indicated antibody diluted in 0.2% w/v bovine serum albumin (BSA), RPMI medium, with 10 mM HEPES, pH 7.2 (binding buffer), or with binding buffer alone (control). After one hour at room temperature, HRG was added to a final concentration of 5 nM to half the wells (+). A similar volume of binding buffer was added to the other wells (-). The incubation was continued for approximately 10 minutes.

Supernatants were removed by aspiration and the cells were lysed in RPMI, 10 mM HEPES, pH 7.2, 1.0% v/v TRITON X-100™, 1.0% w/v CHAPS (lysis buffer), containing 0.2 mM PMSF, 10 $\mu$g/ml leupeptin, and 10 TU/ml aprotinin. The lysates were cleared of insoluble material by centrifugation.

ErbB2 was immunoprecipitated using a monoclonal antibody covalently coupled to an affinity gel (Affi-Prep 10, Bio-Rad). This antibody (Ab-3, Oncogene Sciences) recognizes a cytoplasmic domain epitope. Immunoprecipitation was performed by adding 10 $\mu$l of gel slurry containing approximately 8.5 $\mu$g of immobilized antibody to each lysate, and the samples were allowed to mix at room temperature for two hours. The gels were then collected by centrifugation. The gels were washed batchwise three times with lysis buffer to remove unbound material. SDS sample buffer was then added and the samples were heated briefly in a boiling water bath.

Supernatants were run on 4-12% polyacrylamide gels and electroblotted onto nitrocellulose membranes. The presence of ErbB3 was assessed by probing the blots with a polyclonal antibody against a cytoplasmic domain epitope thereof (c-17, Santa Cruz Biotech). The blots were visualized using a chemiluminescent substrate (ECL, Amersham).

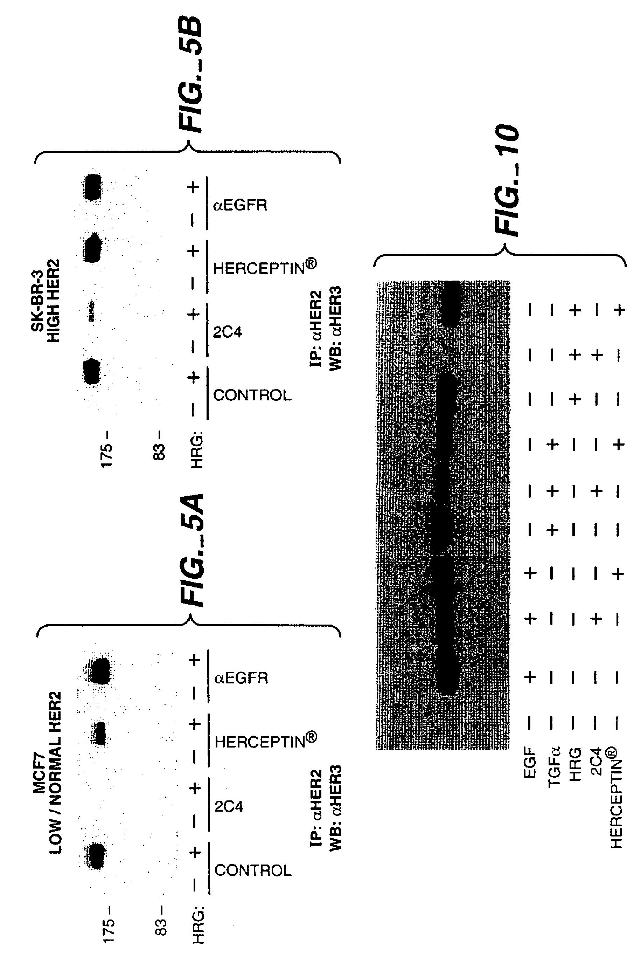

As shown in the control lanes of Figs. 5A and 5B, for MCF7 and SK-BR-3 cells, respectively, ErbB3 was present in an ErbB2 immunoprecipitate only when the cells were stimulated with HRG. If the cells were first incubated with monoclonal antibody 2C4, the ErbB3 signal was abolished in MCF7 cells (Fig. 5A, lane 2C4 +) or substantially reduced in SK-BR-3 cells (Fig. 5B, lane 2C4+). As shown in Figs 5A-B, monoclonal antibody 2C4 blocks heregulin dependent association of ErbB3 with ErbB2 in both MCF7 and SK-BR-3 cells substantially more effectively than HERCEPTIN®. Preincubation with HERCEPTIN® decreased the ErbB3 signal in MCF7 lysates but had little or no effect on the amount of ErbB3 co-precipitated from SK-BR-3 lysates. Preincubation with an antibody against the EGF receptor (Ab-1, Oncogene Sciences) had no effect on the ability of ErbB3 to co-immunoprecipitate with ErbB2 in either cell line.

### Example 3

**Humanized 2C4 Antibodies and Affinity Matured 2C4 Antibody Variants**

The variable domains of murine monoclonal antibody 2C4 were first cloned into a vector which allows production of a mouse/human chimeric Fab fragment. Total RNA was isolated from the hybridoma cells using a Stratagene RNA extraction kit following manufacturer's protocols. The variable domains were amplified by RT-PCR, gel purified, and inserted into a derivative of a pUC1 19-based plasmid containing a human kappa constant domain and human $C_H1$ domain as previously described (Carter *et al. PNAS (USA)* 89:4285 (1992); and U.S. Patent No. 5,821,337). The resultant plasmid was transformed into *E. coli* strain 16C9 for expression of the Fab fragment. Growth of cultures, induction of protein expression, and purification of Fab fragment were as previously described (Werther *et al. J. Immunol.* 157: 4986-4995 (1996); Presta *et al. Cancer Research* 57: 4593-4599 (1997)). Purified chimeric 2C4 Fab fragment was compared to the murine parent antibody 2C4 with respect to its ability to inhibit $^{125}$I-HRG binding to MCF7 cells and inhibit rHRG activation of p180 tyrosine phosphorylation in MCF7 cells. As shown in Fig. 6A, the chimeric 2C4 Fab fragment is very effective in disrupting the formation of the high affinity ErbB2-ErbB3 binding site on the human breast cancer cell line, MCF7. The relative $IC_{50}$ value calculated for intact murine 2C4 is $4.0 \pm 0.4$nM, whereas the value for the Fab fragment is $7.7 \pm 1.1$nM. As illustrated in Fig. 6B, the monovalent chimeric 2C4 Fab fragment is very effective in disrupting HRG-dependent ErbB2-ErbB3 activation. The $IC_{50}$ value calculated for intact murine monoclonal antibody 2C4 is $6.0 \pm 2$nM, whereas the value for the Fab fragment is $15.0 \pm 2$nM.

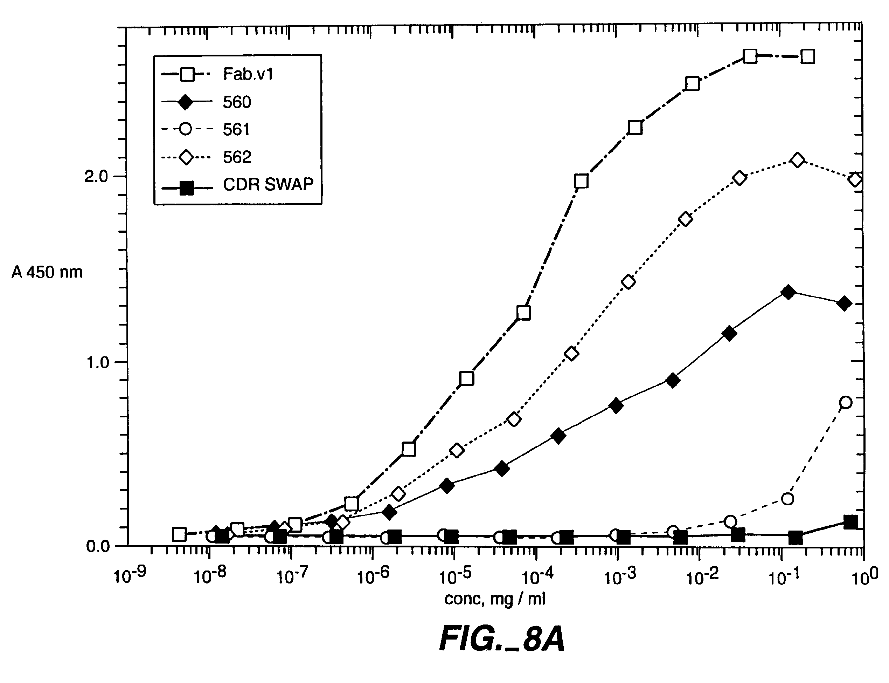

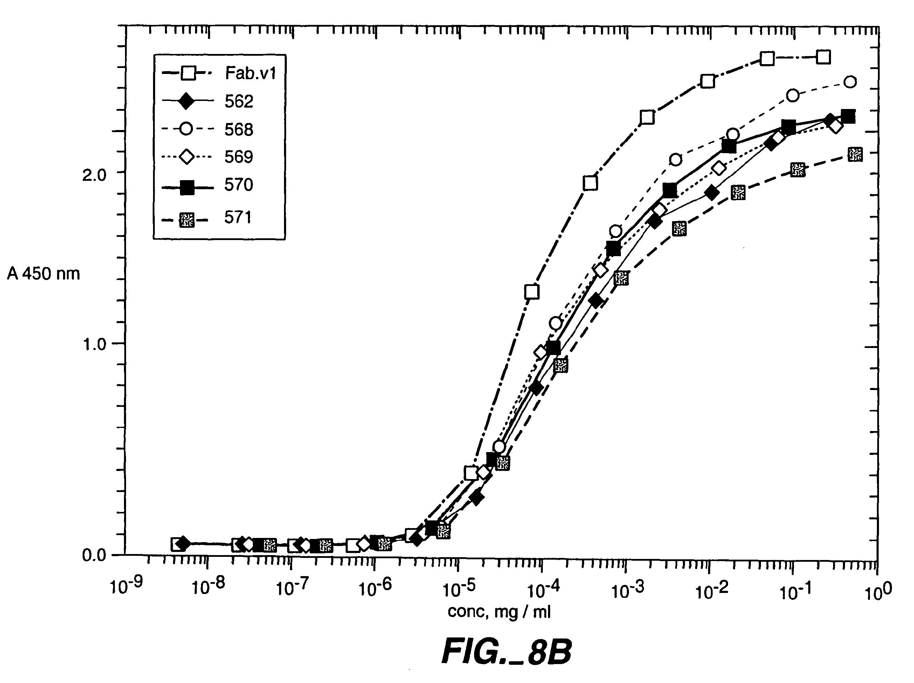

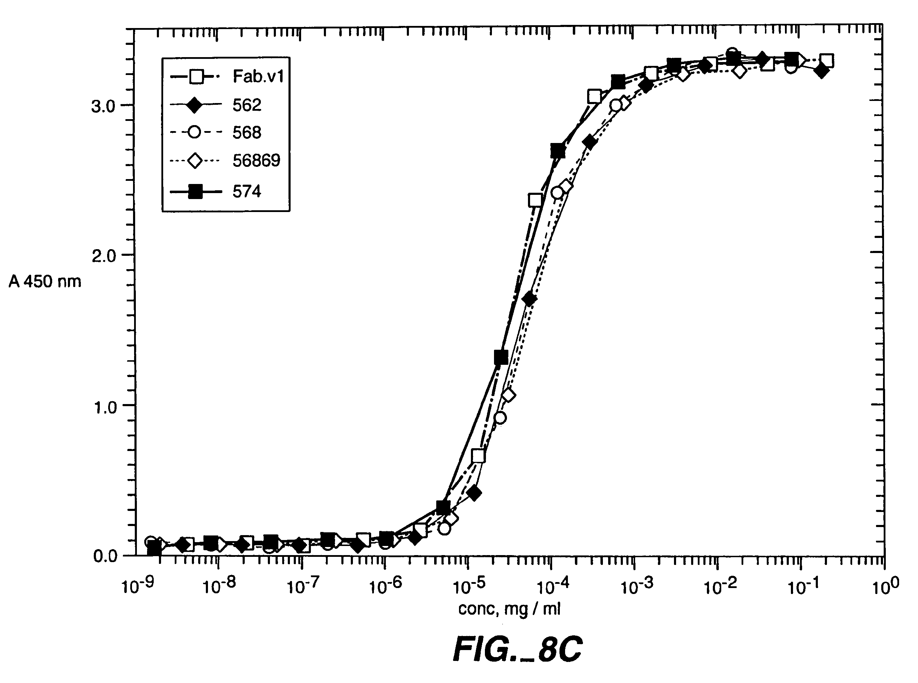

DNA sequencing of the chimeric clone allowed identification of the CDR residues (Kabat *et al., Sequences of Proteins of Immunological Interest,* 5th Ed. Public Health Service, National Institutes of Health, Bethesda, MD (1991)) (Figs. 7A and B). Using oligonucleotide site-directed mutagenesis, all six of these CDR regions were introduced into a complete human framework ($V_L$ kappa subgroup I and $V_H$ subgroup III) contained on plasmid VX4 as previously described (Presta *et al., Cancer Research* 57: 4593-4599 (1997)). Protein from the resultant "CDR-swap" was expressed and purified as above. Binding studies were performed to compare the two versions. Briefly, a NUNC MAXISORP™ plate was coated with 1 microgram per ml of ErbB2 extracellular domain (ECD; produced as described in WO 90/14357) in 50 mM carbonate buffer, pH 9.6, overnight at 4°C, and then blocked with ELISA diluent (0.5% BSA, 0.05% polysorbate 20, PBS) at room temperature for 1 hour. Serial dilutions of samples in ELISA diluent were incubated on the plates for 2 hours. After washing, bound Fab fragment was detected with biotinylated murine anti-human kappa antibody (ICN 634771) followed by streptavidin-conjugated horseradish peroxidase (Sigma) and using 3,3',5,5'-tetramethyl benzidine (Kirkegaard & Perry Laboratories, Gaithersburg, MD) as substrate. Absorbance was read at 450 nm. As shown in Fig. 8A, all binding was lost on construction of the CDR-swap human Fab fragment.

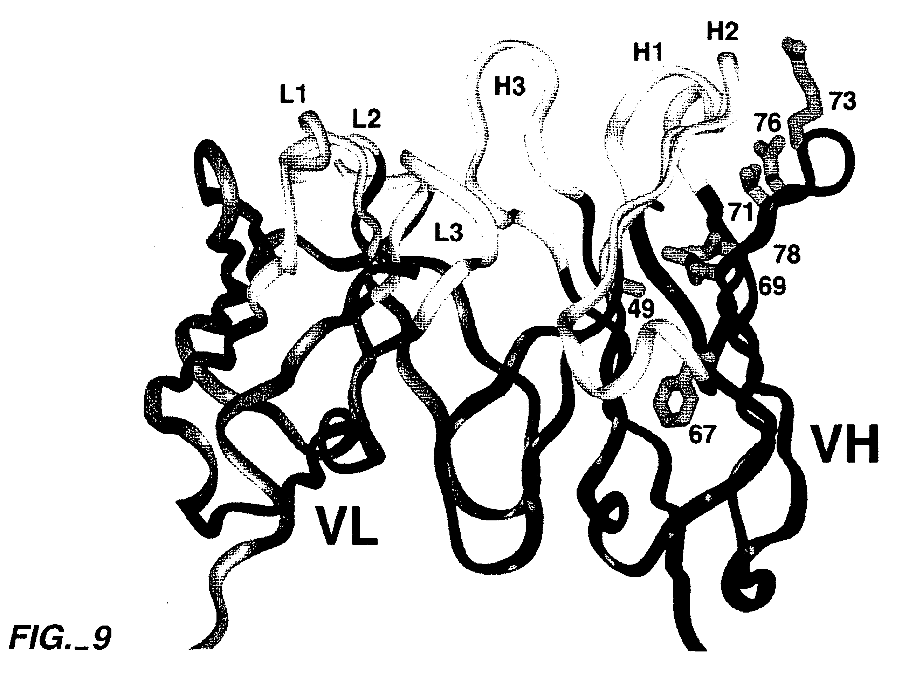

To restore binding of the humanized Fab, mutants were constructed using DNA from the CDR-swap as template. Using a computer generated model (Fig. 9), these mutations were designed to change human framework region residues to their murine counterparts at positions where the change might affect CDR conformations or the antibody-antigen interface. Mutants are shown in Table 2.

### Table 2

| Designation of Humanized 2C4 FR Mutations | |
| --- | --- |
| Mutant no. | Framework region (FR) substitutions |
| 560 | ArgH71Val |
| 561 | AspH73Arg |
| 562 | ArgH71Val, AspH73Arg |
| 568 | ArgH71Val, AspH73Arg, AlaH49Gly |
| 569 | ArgH71Val, AspH73Arg, PheH67Ala |
| 570 | ArgH71Val, AspH73Arg, AsnH76Arg |
| 571 | ArgH71Val, AspH73Arg, LeuH78Val |
| 574 | ArgH71Val, AspH73Arg, IleH69Leu |
| 56869 | ArgH71Val, AspH73Arg, AlaH49Gly, PheH67Ala |

Binding curves for the various mutants are shown in Figs. 8A-C. Humanized Fab version 574, with the changes ArgH71 Val, AspH73Arg and IleH69Leu, appears to have binding restored to that of the original chimeric 2C4 Fab fragment. Additional FR and/or CDR residues, such as L2, L54, L55, L56, H35 and/or H48, may be modified (*e.g.* substituted as follows - IleL2Thr; ArgL54Leu; TyrL55Glu; ThrL56Ser; AspH35Ser; and ValH48Ile) in order to further refine or enhance binding of the humanized antibody. Alternatively, or additionally, the humanized antibody may be affinity matured (see above) in order to further improve or refine its affinity and/or other biological activities.

Humanized 2C4 version 574 was affinity matured using a phage-display method. Briefly, humanized 2C4.574 Fab was cloned into a phage display vector as a geneIII fusion. When phage particles are induced by infection with M13KO7 helper phage, this fusion allows the Fab to be displayed on the N-terminus of the phage tail-fiber protein, geneIII (Baca *et al. J Biol Chem.* 272:10678 (1997)).

Individual libraries were constructed for each of the 6 CDRs identified above. In these libraries, the amino acids in the CDRs which were identified using a computer generated model (Fig. 9) as being potentially significant in binding to ErbB2 were randomized using oligos containing "NNS" as their codons. The libraries were then panned against ErbB2 ECD coated on NUNC MAXISORP™ plates with 3% dry milk in PBS with 0.2% TWEEN 20® (MPBST) used in place of all blocking solutions. In order to select for phage with affinities higher than that of 2C4.574, in panning rounds 3, 4, and 5, soluble ErbB2 ECD or soluble Fab 2C4.574 was added during the wash steps as competitor. Wash times were extended to 1 hour at room temperature.

After 5 rounds of panning, individual clones were again analyzed by phage-ELISA. Individual clones were grown in Costar 96-well U-bottomed tissue culture plates, and phage were induced by addition of helper phage. After overnight growth, *E. coli* cells were pelleted, and the phage-containing supernates were transfered to 96-well plates where the phage were blocked with MPBST for 1 hr at room temperature. NUNC MAXISORP™ plates coated with ErbB2 ECD were also blocked with MPBST as above. Blocked phage were incubated on the plates for 2 hours. After washing, bound phage were detected using horseradish-peroxidase-conjugated anti-M13 monoclonal antibody (Amersham Pharmacia Biotech, Inc. 27-9421-01) diluted 1:5000 in MPBST, followed by 3,3',5,5',-tetramethyl benzidine as substrate. Absorbance was read at 450 nm.

The 48 clones from each library which gave the highest signals were DNA sequenced. Those clones whose sequences occurred the most frequently were subcloned into the vector described above which allows expression of soluble Fabs. These Fabs were induced, proteins purified and the purified Fabs were analyzed for binding by ELISA as described above and the binding was compared to that of the starting humanized 2C4.574 version.

After interesting mutations in individual CDRs were identified, additional mutants which were various combinations of these were constructed and tested as above. Mutants which gave improved binding relative to 574 are described in Table 3.

**Table 3**
**Designation of mutants derived from affinity maturation of 2C4.574**

| Mutant Name | Change from 574 | Mutant/574* |
|---|---|---|
| H3.A1 | serH99trp, metH34leu | 0.380 |
| L2.F5 | serL50trp, tyrL53gly, metH34leu | 0.087 |
| H1.3.B3 | thrH28gln,thrH30ser, metH34leu | 0.572 |
| L3.G6 | tyrL92pro, ileL93lys, metH34leu | 0.569 |
| L3.G11 | tyrL92ser, ileL93arg, tyrL94gly, metH34leu | 0.561 |
| L3.29 | tyrL92phe, tyrL96asn, metH34leu | 0.552 |
| L3.36 | tyrL92phe, tyrL94leu, tyrL96pro, metH34leu | 0.215 |
| 654 | serL50trp, metH34leu | 0.176 |
| 655 | metH34ser | 0.542 |
| 659 | serL50trp, metH34ser | 0.076 |
| L2.F5.H3.A | serL50trp, tyrL53gly, metH34leu, serH99trp | 0.175 |
| L3G6.H3.Al | tyrL92pro, ileL93lys, metH34leu, serH99trp | 0.218 |
| H1.3.B3.H3.A1 | thrH28gln, thrH30ser, metH34leu, serH99trp | 0.306 |
| L3.G11.H3.Al | tyrL92ser, ileL93arg, tyrL94gly, metH34leu, serH99trp | 0.248 |
| 654.H3.Al | serL50trp, metH34leu, serH99trp | 0.133 |
| 654.L3.G6 | serL50trp, metH34leu, tyrL92pro, ileL93lys | 0.213 |
| 654.L3.29 | serL50trp, metH34leu, tyrL92phe, tyrL96asn | 0.236 |
| 654.L3.36 | serL50trp, metH351eu, tyrL92phe, tyrL941eu, tyrL96pro | 0.141 |

*Ratio of the amount of mutant needed to give the mid-OD of the standard curve to the amount of 574 needed to give the mid-OD of the standard curve in an Erb2-ECD ELISA. A number less than 1.0 indicates that the mutant binds Erb2 better than 574 binds.

The following mutants have also been constructed, and are currently under evaluation:

| | |
|---|---|
| 659.L3.G6 | serL50trp, metH34ser, tyrL92pro, ileL93lys |
| 659.L3.G11 | serL50trp, metH34ser, tyrL92ser, ileL93arg, tyrL94gly |
| 659.L3.29 | serL50trp, metH34ser, tyrL92phe, tyrL96asn |
| 659.L3.36 | serL50trp, metH34ser, tyrL92phe, tyrL94leu, tyrL96pro |
| L2F5.L3G6 | serL50trp, tyrL53gly, metH34leu, tyrL92pro, ileL93lys |
| L2F5.L3G11 | serL50trp, tyrL53gly, metH34leu, tyrL92ser, ileL93arg, tyrL94gly |
| L2F5.L29 | serL50trp, tyrL53gly, metH34leu, tyrL92phe, tyrL96asn |
| L2F5.L36 | serL50trp, tyrL53gly, metH34leu, tyrL92phe, tyrL94leu, tyrL96pro |
| L2F5.L3G6.655 | serL50trp, tyrL53gly, metH35ser, tyrL92pro, ileL93lys |
| L2F5.L3G11.655 | serL50trp, tyrL53gly, metH34ser, tyrL92ser, ileL93arg, tyrL94gly |
| L2F5.L29.655 | serL50trp, tyrL53gly, metH34ser, tyrL92phe, tyrL96asn |
| L2F5.L36.655 | serL50trp, tyrL53gly, metH34ser, tyrL92phe, tyrL94leu, tyrL96pro |

The following mutants, suggested by a homology scan, are currently being constructed:

| | |
|---|---|
| 678 | thrH30ala |
| 679 | thrH30ser |
| 680 | lysH64arg |

(continued)

| 681 | leuH96val |
|-----|-----------|
| 682 | thrL97ala |
| 683 | thrL97ser |
| 684 | tyrL96phe |
| 685 | tyrL96ala |
| 686 | tyrL91phe |
| 687 | thrL56ala |
| 688 | glnL28ala |
| 689 | glnL28glu |

The preferred amino acid at H34 would be methionine. A change to leucine might be made if there were found to be oxidation at this position.

AsnH52 and asnH53 were found to be strongly preferred for binding. Changing these residues to alanine or aspartic acid dramatically decreased binding.

An intact antibody comprising humanized Fab version 574 with a human IgG1 heavy chain constant region has been prepared (see U.S. Patent No. 5,821,337). The intact antibody is produced by Chinese Hamster Ovary (CHO) cells.

## Example 4

### Monoclonal Antibody 2C4 Blocks EGF, TGF-α or HRG Mediated Activation of MAPK

Many growth factor receptors signal through the mitogen-activated protein kinase (MAPK) pathway. These dual specificity kinases are one of the key endpoints in signal transduction pathways that ultimately triggers cancer cells to divide. The ability of monoclonal antibody 2C4 or HERCEPTIN® to inhibit EGF, TGF-α or HRG activation of MAPK was assessed in the following way.

MCF7 cells ($10^5$ cells/well) were plated in serum containing media in 12-well cell culture plates. The next day, the cell media was removed and fresh media containing 0.1% serum was added to each well. This procedure was then repeated the following day and prior to assay the media was replaced with serum-free binding buffer (Jones *et al. J. Biol. Chem.* 273:11667-74 (1998); and Schaefer *et al. J. Biol. Chem.* 274:859-66 (1999)). Cells were allowed to equilibrate to room temperature and then incubated for 30 minutes with 0.5 mL of 200 nM HERCEPTIN® or monoclonal antibody 2C4. Cells were then treated with 1 nM EGF, 1 nM TGF-α or 0.2 nM HRG for 15 minutes. The reaction was stopped by aspirating the cell medium and then adding 0.2 mL SDS-PAGE sample buffer containing 1% DTT. MAPK activation was assessed by Western blotting using an anti-active MAPK antibody (Promega) as described previously (Jones *et al. J. Biol. Chem.* 273:11667-74 (1998)).

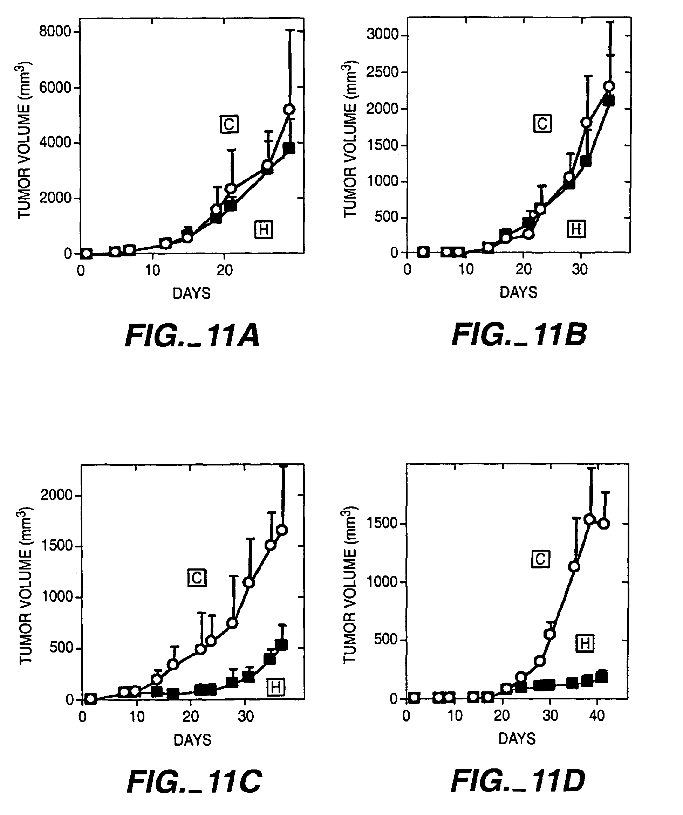

As shown in Fig. 10, monoclonal antibody 2C4 significantly blocks EGF, TGF-α and HRG mediated activation of MAPK to a greater extent than HERCEPTIN®. These data suggest that monoclonal antibody 2C4 binds to a surface of ErbB2 that is used for its association with either EGFR or ErbB3 and thus prevents the formation of the signaling receptor complex.

## Example 5

### Effect of HERCEPTIN® on the Growth of Androgen Dependent and Androgen Independent Human Prostate Cancer

The effect of HERCEPTIN® monotherapy in androgen dependent and androgen independent prostate cancer xenograft models and the combination of HERCEPTIN® with paclitaxel were studied in preclinical models of human prostate cancer. The androgen dependent CWR22 and LNCaP human prostate cancer xenograft models and androgen independent sublines of CWR22 were used (Nagabhushan *et al. Cancer Res.* 56:3042-3046 (1996); Wainstein *et al. Cancer Res.* 54:6049-6052 (1994); and Stearns *et al. Prostate* 36:56-58 (1998)).

MATERIALS AND METHODS

*Animal studies.* Four to six week old nude athymic BALB/c male and female mice were obtained from the National Cancer Institute-Frederick Cancer Center and maintained in pressurized ventilated caging at the Sloan-Kettering Institute. Male animals were inoculated s.c. with 1 x 10⁶ LNCaP cells or minced tumor tissue from the androgen dependent CWR22, and females received the androgen independent sublines CWR22R, or CWR22SA 1, CWRSA4, CWRSA6 which were obtained by selecting tumors for regrowth and increased serum PSA after androgen withdrawal. All lines were injected together with reconstituted basement membrane (Matrigel; Collaborative Research, Bedford, MA) as described previously (Nagabhushan *et al. Cancer Res.* 56:3042-3046 (1996); Wainstein *et al. Cancer Res.* 54:6049-6052 (1994); and Sato *et al. Cancer Res.* 57:1584-1589 (1997)). To maintain serum testosterone levels, male mice were implanted with 12.5-mg sustained release testosterone pellets (Innovative Research of America, Sarasota, FL) s.c. before receiving the tumor cell inoculation. Treatments consisted of twice weekly i.p. injection of 20 mg/kg HERCEPTIN® in PBS for no less than 3 weeks and/or paclitaxel (TAXOL®, Bristol Myers-Squibb Company, Princeton, NJ) s.c. low dose (6.25mg/kg s.c., 5x/week x 3 weeks) or high dose (12.5 mg/kg s.c., 5x/week x 2 weeks) in sterile saline. Control mice were given vehicle alone. Tumors were measured every 3-4 days with vernier calipers, and tumor volumes were calculated by the formula: p/6 x larger diameter x (smaller diameter)². Animals with palpably established tumors of at least 65mm³ in volume were designated to treatment groups.

*Determination of the ErbB2 status of the xenografts.* Xenografts were assayed for ErbB2 expression by immunohistochemistry using the DAKO ErbB2 kits (HERCEPTEST®, DAKO Corporation, Carpinteria, CA). The samples were scored blindly by comparison with standard controls in the DAKO kit standards and scored as follows: 0 (no staining, or membrane staining in less than 10% of the tumor cells), 1⁺ (faint membrane staining in more than 10% of the tumor cells), 2⁺ (weak to moderate complete membrane stain in >10% of cells), or 3⁺ (moderate to strong complete membrane staining in >10% of cells). A score of 0 or 1⁺ was considered negative for ErbB2 overexpression, whereas 2⁺ or 3⁺ indicated ErbB2 overexpression. FISH analysis was done using the Oncor kits (INFORM® ErbB2 gene detection system, Oncor Inc., Gaithersburg, MD). A minimum of 100 tumor cells in each tumor was evaluated for nuclear ErbB2 gene copy number (Ross *et al. Hum. Pathol.* 28:827-833 (1997)).

*Determination of Serum PSA Values.* Blood samples (-50 ml) from male mice collected in microtainer serum separator tubes (Becton Dickinson, Franklin Lakes, NJ) by superficial incision of the dorsal tail vein were taken prior to therapy, and on days 9 and 21 of treatment. PSA values were then determined from serum using the Tandem-R PSA immunoradiometric assay (Hybritech, San Diego, CA).

*Statistical Analysis.* Pairwise differences between the tumor volumes of the treatment groups were compared over time using a permutation test. The null hypothesis for this test is that treatment has no differential effect on the tumor volumes over time. The statistic used to test the hypothesis was the sum of the squared differences between mean tumor volume summed over all time points.

$$\text{SS\_DEV} = \sum_{i=1}^{k}\left(\bar{x_i} - \bar{y_i}\right)^2$$

SS_Dev was used in order to capture average differences between treatment groups at each time point. This statistic reflects the amount by which the trajectories of average tumor volume of the two treatment groups are different.

RESULTS

*ErbB2 immunohistochemical staining and ErbB2 gene copy number of the prostate xenografts.* TheErbB2 expression patterns of the androgen dependent and androgen independent prostate xenografts were examined by immunohistochemistry (IHC) and FISH. The parental androgen dependent CWR22 tumors demonstrated 2⁺ ErbB2 staining and the LNCaP tumors 3⁺ ErbB2 staining. The androgen independent sublines of CWR22 demonstrate 2⁺ (CWRSA1), 3⁺ (CWRSA4), 2⁺ (CWRSA6) and 1⁺ (CWR22R) staining for ErbB2. All tumors had a 2-4 ErbB2 gene copy (normal range) number by FISH.

*Effects of HERCEPTIN® on established prostate cancer xenografts.* Animal experiments were preformed to evaluate the efficacy of HERCEPTIN® in well-established androgen dependent and androgen independent prostate cancer xenografts. The CWR22, LNCaP, CWR22R and CWRSA6 models were used for these experiments because they provided reproducible growth curves. HERCEPTIN® was administered intraperitoneally (i.p.) at a dose of 20 mg/kg twice weekly after the xenograft had been established. No effect of HERCEPTIN® on tumor growth was observed in any of the androgen independent tumors when compared to controls (CWR22R, p=0.60, n=10, Fig. 11 A; CWRSA6, p=0.63, n=10, Fig.11B). The murine anti-ErbB2 antibody, 4D5, also had no effect on tumor growth in the CWR22R androgen independent line (p=0.21, n=10). In contrast, HERCEPTIN® did show significant growth inhibition in both of the androgen dependent xenograft models, CWR22 (68% growth inhibition; p<0.03, n=12, Fig. 11C) and LNCaP (89% growth inhibition; p=0.002, n=12, Fig. 11D).

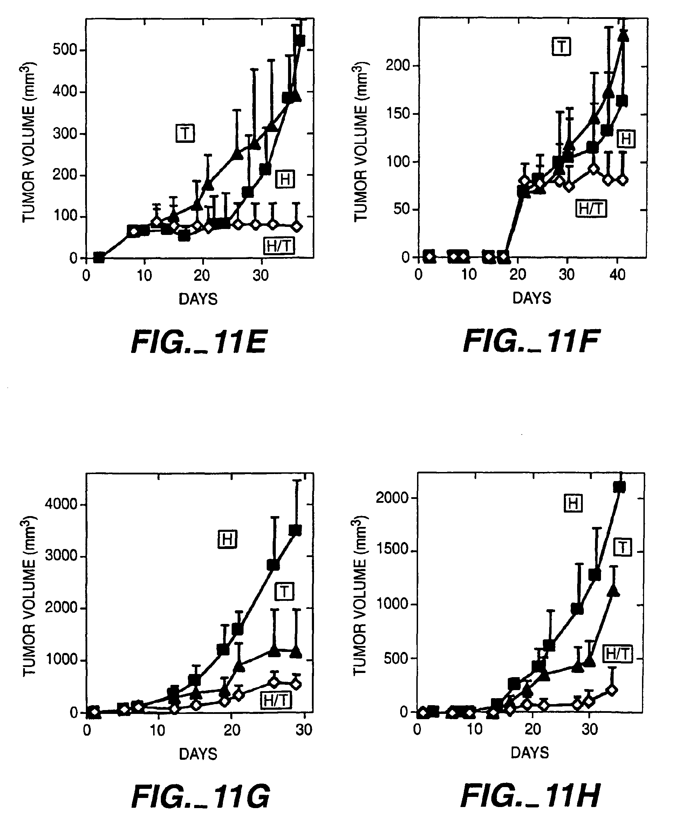

*Effects of HERCEPTIN® combined with TAXOL® on established tumor xenografts.* When paclitaxel and HERCEPTIN® were co-administered to animals there was a marked reduction in tumor volume versus control for both androgen dependent and androgen independent tumors (CWR22 98% growth inhibition, p<0.01, Fig. 11E; CWR22R 92% growth inhibition, p<0.01, Fig. 11G; LNCaP 94% growth inhibition, p=0.006, Fig. 11 F; CWRSA6 77% growth inhibition, p<0.01, Fig. 11H). Increased growth inhibition was observed with the combination of HERCEPTIN® and paclitaxel as compared to each agent alone at the end of the treatment period in the animals with androgen dependent xenografts (Figs. 11E-H): the CWR22 group (mean tumor volumes, n=6 in each group, paclitaxel 408 mm$^3$, HERCEPTIN® 520 mm$^3$, paclitaxel and HERCEPTIN® 76 mm$^3$; p<0.03 paclitaxel versus paclitaxel and HERCEPTIN®) and the LNCaP group (mean tumor volumes, n=6 in each group, paclitaxel 233 mm$^3$, HERCEPTIN® 163 mm$^3$, paclitaxel and HERCEPTIN® 82 mm$^3$; p<0.03 paclitaxel versus paclitaxel and HERCEPTIN®). In addition, there was increased growth inhibition with the combination of HERCEPTIN® and paclitaxel versus each agent alone at the end of the treatment period in the animals with androgen independent xenografts (Figs. 11E-H): the CWRSA6 group (mean tumor volumes, n=5 in each group, paclitaxel 1,496 mm$^3$, HERCEPTIN® 2,941 mm$^3$, paclitaxel and HERCEPTIN® 687 mm$^3$; p<0.001 paclitaxel versus paclitaxel and HERCEPTIN®) and the CWR22R group (mean tumor volumes, n=5 in each group, paclitaxel 1,273 mm$^3$, HERCEPTIN® 3,811 mm$^3$, paclitaxel and HERCEPTIN® 592 mm$^3$; p=0.095 paclitaxel versus paclitaxel and HERCEPTIN®).

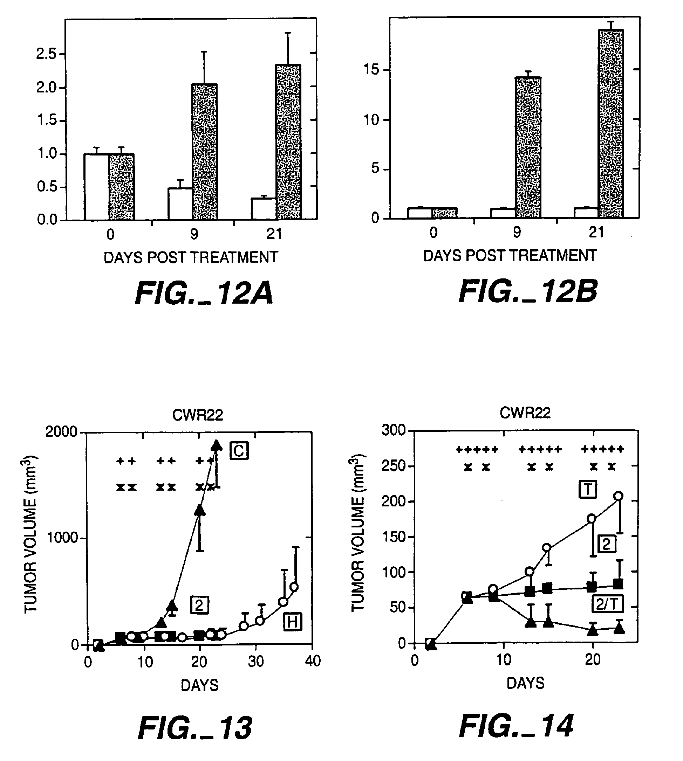

*Effects of HERCEPTIN® on PSA index in the treated animals with androgen dependent xenografts.* As shown in Figs. 12A and B, there was a significant increase in prostate specific antigen (PSA) index (ng PSA/ml serum/mm$^3$ tumor) in HERCEPTIN®-treated androgen dependent groups compared with control (CWR22, 1864% versus -4%, p<0.0001, Fig. 12A; LNCaP, 232% versus -68%, p<0.0001, Fig. 12B). There was also an increase in the PSA index after combination treatment with HERCEPTIN® and paclitaxel when compared with pretreatment values.

## CONCLUSIONS

In these prostate cancer model systems, HERCEPTIN® alone has clinical activity only in the androgen dependent tumors and has at least an additive effect on growth, in combination with paclitaxel, in both androgen dependent and androgen independent tumors. Response to HERCEPTIN® did not correlate with the PSA levels, as the PSA index markedly increased in the HERCEPTIN®-treated group, while remaining constant in the control group.

## **Example 6**

## **Effect of Monoclonal Antibody 2C4 on the Growth of Androgen Dependent and Androgen Independent Human Prostate Cancer**

The effect of an antibody, which blocks ligand activation of an ErbB receptor, on human prostate cancer was assessed. In particular, response of xenograft tumors to HERCEPTIN®, monoclonal antibody 2C4, paclitaxel and combination 2C4/paclitaxel treatment was determined using the androgen dependent tumor CWR22 and androgen independent tumors CWR22R and CWRSA6 described in Example 5 above. The antibodies and paclitaxel were administered as described in Example 5.

The response of the androgen dependent tumor CWR22 to therapy is shown in Figs. 13 and 14. Results are given as mean tumor volume $\pm$ SE. The tumor volumes of the animals depicted in Fig. 13 demonstrate that HERCEPTIN® has clinical activity in this androgen dependent model, as does monoclonal antibody 2C4. The combination of monoclonal antibody 2C4 and TAXOL® demonstrates increased growth inhibition when compared with either 2C4 or TAXOL® alone (Fig. 14; p=0.003).

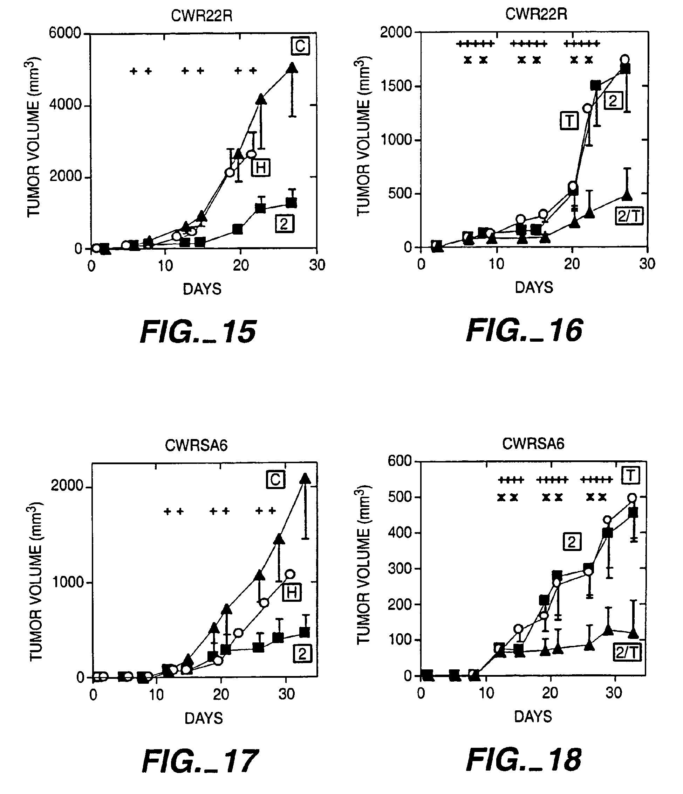

The response of the androgen independent tumors CWR22R and CWRSA6 to therapy with HERCEPTIN®, monoclonal antibody 2C4, paclitaxel or combination 2C4/paclitaxel treatment is shown in Figs. 15-18. Results are given as mean tumor volume $\pm$ SE. The tumor volumes of the animals depicted in Figs. 15 and 17 demonstrate that HERCEPTIN® has little or no clinical activity in these androgen independent models, while monoclonal antibody 2C4 has clinical activity in these models. The combination of monoclonal antibody 2C4 and TAXOL® demonstrates increased growth inhibition when compared with either monoclonal antibody 2C4 or TAXOL® alone (Figs. 16 and 18; p=0.002).

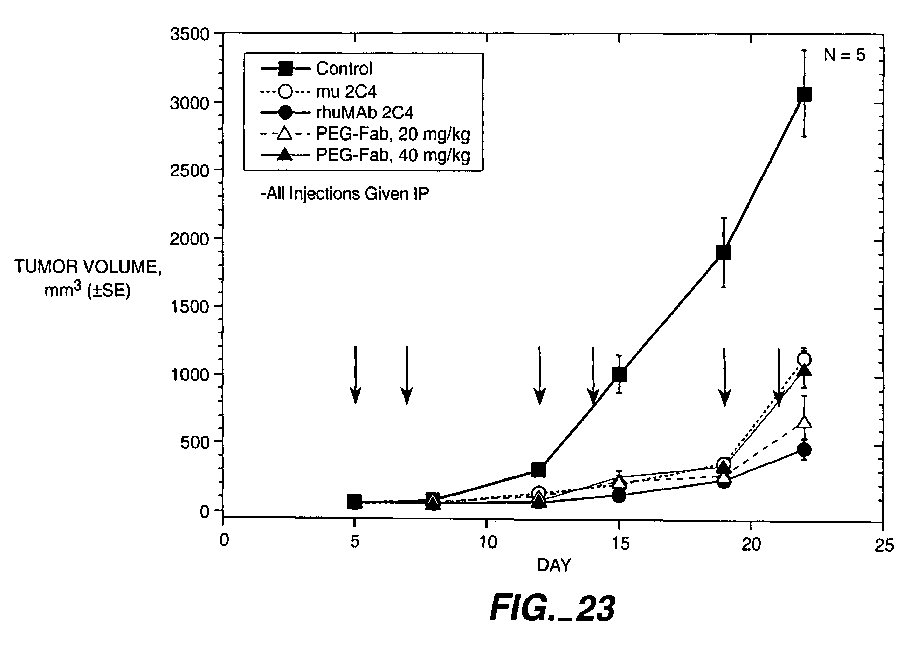

A Fab' fragment of rhuMAb 2C4 was expressed in *E. coli* and conjugated to 20kD branched polyethylene glycol (PEG) as described in WO98/37200. The ability of the murine 2C4 antibody (20mg/kg), rhuMAb 2C4 (20mg/kg), and the pegylated Fab fragment (PEG-Fab; 20 or 40mg/kg) to treat androgen independent prostate cancer *in vivo* was assessed using the above CWR22R xenograft. All injections were given IP (N=5). The results of these studies are shown in Fig. 23. These data demonstrate that the tumor inhibition seen with 2C4 in the CWR22R model does not require an intact, bivalent antibody. Since these Fab fragments do not contain Fc, an immunological mechanism such as ADCC can likely be ruled out. These results are consistent with that shown in Fig. 6 utilizing an *in vitro* system and chimeric versions of the 2C4 Fab. The observation that 2C4 inhibits tumor growth as a monovalent fragment also lends credence to the notion that this inhibition is a result of blocking ErbB2 ability to heterodimerize with other ErbB family members and thus inhibits initiation of downstream signaling events.

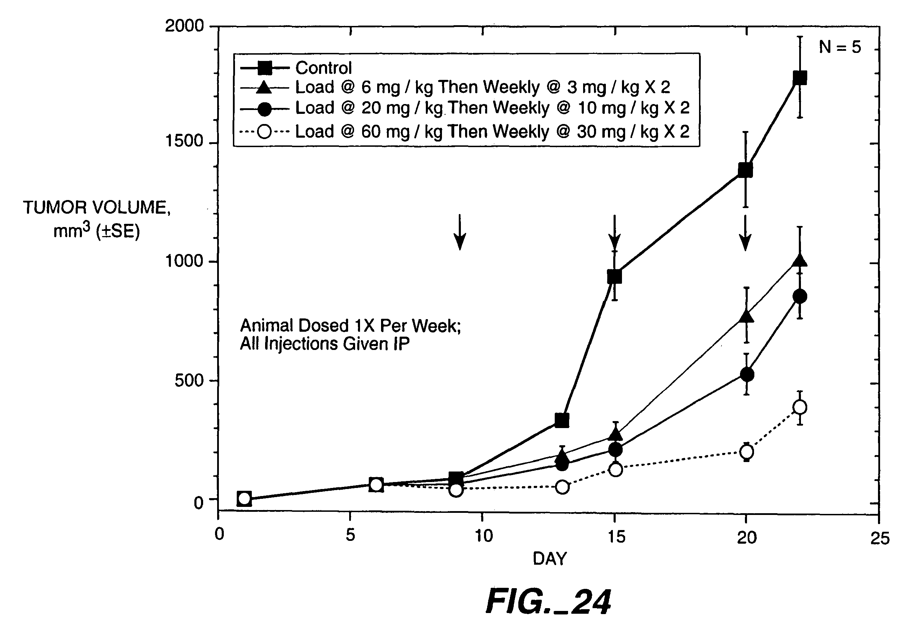

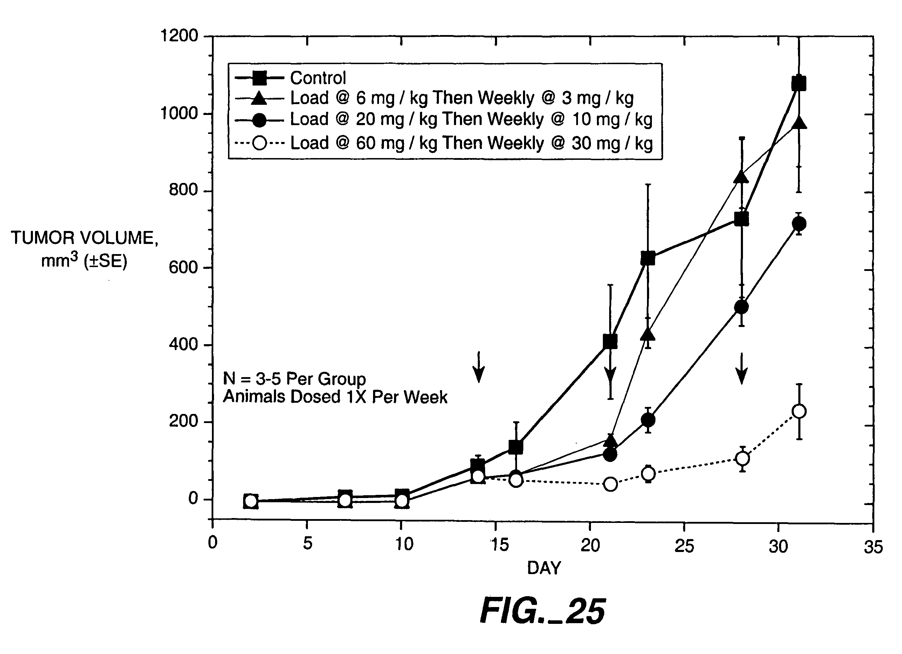

Dose response studies were carried out using rhuMAb 4D5 in the CWR22R and MSKPC6 (Agus *et al. Cancer Research 59:* 4761-4764 (1999)) androgen independent prostate xenografts. Animals were dosed IP with: control; 6mg/kg loading dose then 3mg/kg twice weekly; 20mg/kg loading dose then 10mg/kg twice weekly; or 60mg/kg loading dose then 30mg/kg twice weekly. The results of these studies are shown in Figs. 24 and 25. These data demonstrate that 2C4 suppresses the growth of androgen-independent tumor xenografts in a dose dependent manner. Furthermore, these results further confirm that this inhibition of tumor growth is due to 2C4 treatment and not an experimental artifact.

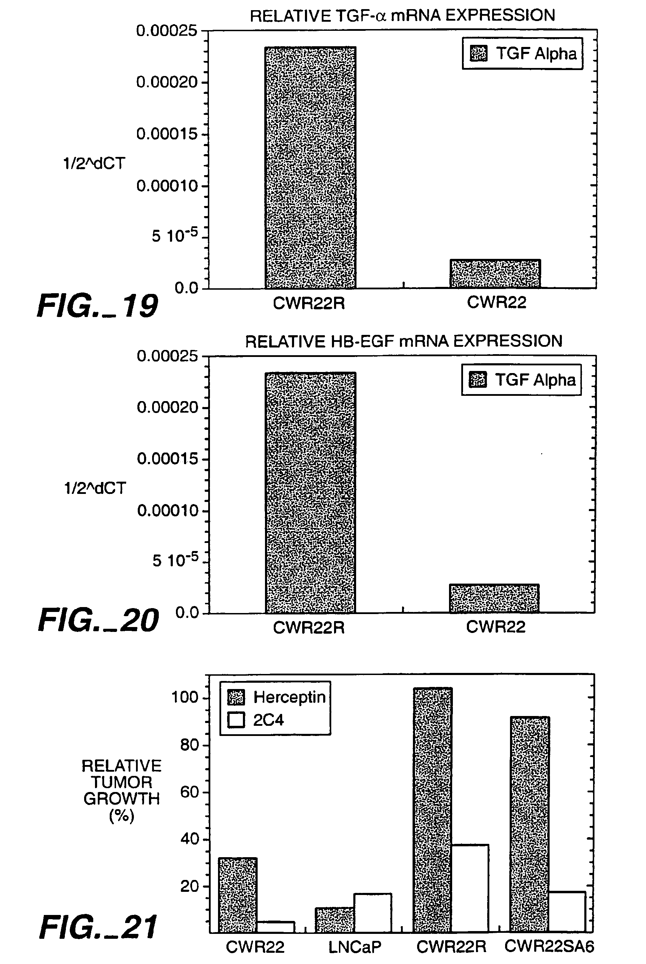

A summary of typical results from the studies in Examples 5 and 6 is shown in Fig. 21.

## Example 7

### TGF-$\alpha$ and HB-EGF levels in Androgen Dependent and Androgen Independent Human Prostate Cancer

TGF-$\alpha$ and HB-EGF mRNA levels in CWR22 cells (androgen dependent) and CWR22R cells (androgen independent) were evaluated in this example.

MATERIALS AND METHODS

*mRNA Preparation.* Frozen tumor tissue was processed according to the Qiagen protocol (Qiagen Maxi Kit #75163). Briefly, homogenization of tissue was accomplished with a Brinkman Polytron (Pt-3000) homogenizer equipped with the PT-DA 3012/2 TS generator using 15 second pulses and then pausing for 30 seconds. This process was repeated three times and the extract was loaded on to a Qiagen column and washed according to the manufacturer's specifications. Columns were eluted with 1 mL of RNAse-free water and RNA content was determined by absorbance at 260 nm. Since TGF-$\alpha$ and HB-EGF are expressed in the cell line MDA-MB-231, total RNA from these cells was used as a standard for TGF-$\alpha$ and HB-EGF quantification.

*Real Time Quantitative PCR.* TGF-$\alpha$ and HB-EGF mRNA was quantified using real time quantitative PCR or TaqMan technique as previously described (Gibson *et al. Genome Research* 6: 986-994 (1996); and Heid *et al. Genomic Research* 6:986-994 (1996)). The sequence of the primer/probe sets used for this analysis are shown below:

TGF-$\alpha$
F 5'-GGACAGCACTGCCAGAGA -3' (SEQ ID NO:14)
R 5'-CAGGTGATTACAGGCCAAGTAG -3' (SEQ ID NO:15)
P 5'FAM-CCTGGGTGTGCCACAGACCTTCA-TAMRA-p-3' (SEQ ID NO:16)
HB-EGF:
F 5'-TGAAGTTACCTCCAGGTTGGT-3' (SEQ ID NO:17)
R 5'-AGACACATTCTGTCCATTTTCAA-3' (SEQ ID NO:18)
P 5'-FAM-CAAGCTGCAAAGTGCCTTGCTCAT-TAMRA-p-3' (SEQ ID NO:19)

where F and R are the forward and reverse primers respectively, and P is the flourescent labeled probe. β-actin was used as a housekeeping gene. Primer/probe sets for β-actin are:

β-actin
F 5'-ATGTATCACAGCCTGTACCTG-3' (SEQ ID NO:20)
R 5'-TTCTTGGTCTCTTCCTCCTTG-3' (SEQ ID NO:21)
P 5'FAM-AGGTCTAAGACCAAGGAAGCACGCAA-TAMRA-p-3' (SEQ ID NO: 22)

TaqMan analysis was performed in a standard 96-well plate format. Standard curves were constructed using 0.6-150 ng of mRNA for TGF-$\alpha$ and HB-EGF analysis and 9.4-150 ng for β-actin. Each dilution was run in duplicate. For tumor samples, 100ng was used for all genes analyzed.

RESULTS

As shown in Figs. 19-20, the androgen independent prostate tumor line, CWR22R, expressed significantly greater levels of the EGFR ligands TGF-$\alpha$ and HB-EGF in comparison to the androgen dependent cell line, CWR22. Specifically, TGF-$\alpha$ was expressed at levels 8-9 higher in the CWR22R tumor relative to the CWR22 tumor. In a similar fashion, HB-EGF was expressed ~19 fold higher in CWR22R versus CWR22.

## Example 8

### Effect of 2C4 or HERCEPTIN® on PSA Index in Animals With Androgen-Dependent Xenografts

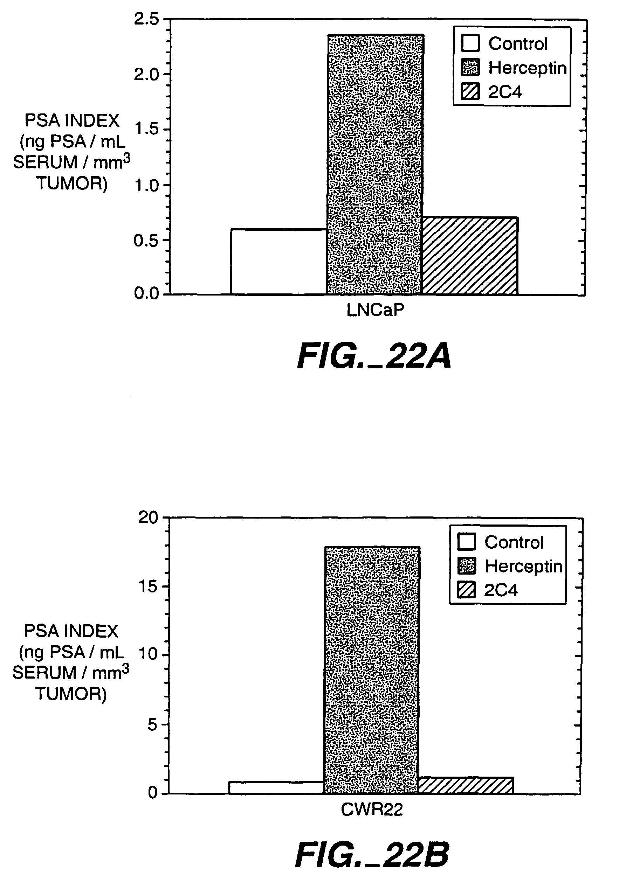

As shown in Fig. 22, the PSA index (defined as ng PSA/mL serum/mm$^3$ tumor) was measured in the androgen-dependent animals at day 21 near the end of treatment. There was a significant increase in the PSA index in HERCEPTIN®-treated, androgen-dependent animals, while the control animals showed a decrease in the PSA index (LNCaP: control=0.6 relative to pretreatment value, HERCEPTIN® group=2.35 relative to pretreatment value at day 21; CWR22: control=1.0 relative to pretreatment value, HERCEPTIN® group=18 relative to pretreatment value at day 21). Relative PSA index decreased in the LNCaP untreated group, presumably secondary to increased necrosis with increasing tumor size. In contrast, there was no significant effect of 2C4 on the PSA index of treated tumors compared with controls. Without being limited to any one theory, a possible explanation for this phenomenon might be related to the degree of ErbB2 activation in prostate cancer cells. ErbB2 activation may cause androgen-independent growth by crosstalk with the androgen receptor signaling pathway (Craft *et al. Nature Med.* 5:280-285 (1999)). In our model systems, HERCEPTIN® binding to ErbB2 led to increased cellular secretion of PSA in an androgen-independent fashion (Agus *et al. Cancer Res.* 59:4761-4764 (1999)). This result further supports the notion of crosstalk between the ErbB2 and androgen receptor signaling pathways.

## Example 9

### Effect of 7C2 anti-ErbB2 Antibody on Androgen Dependent and Independent Xenografts

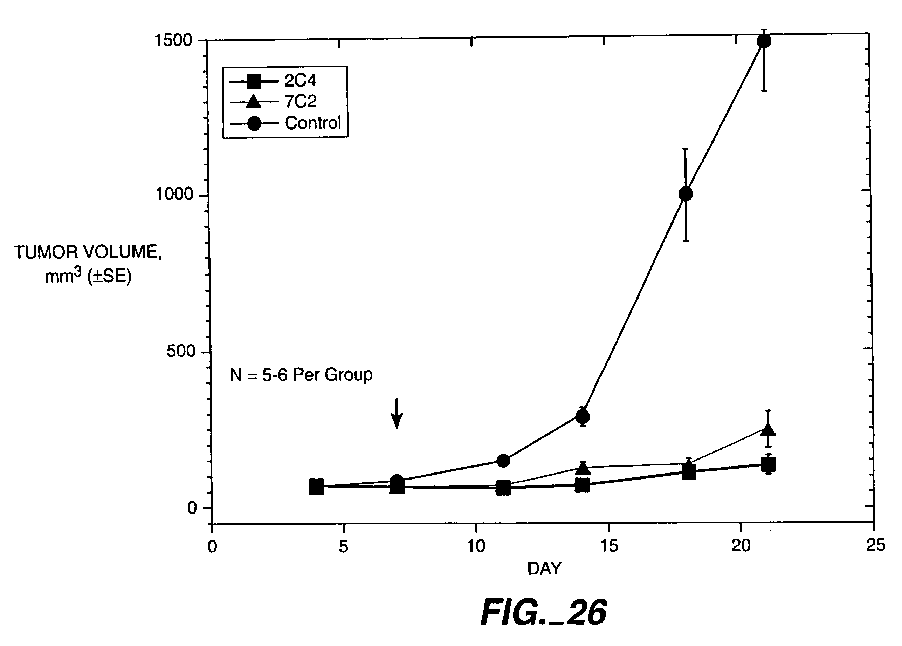

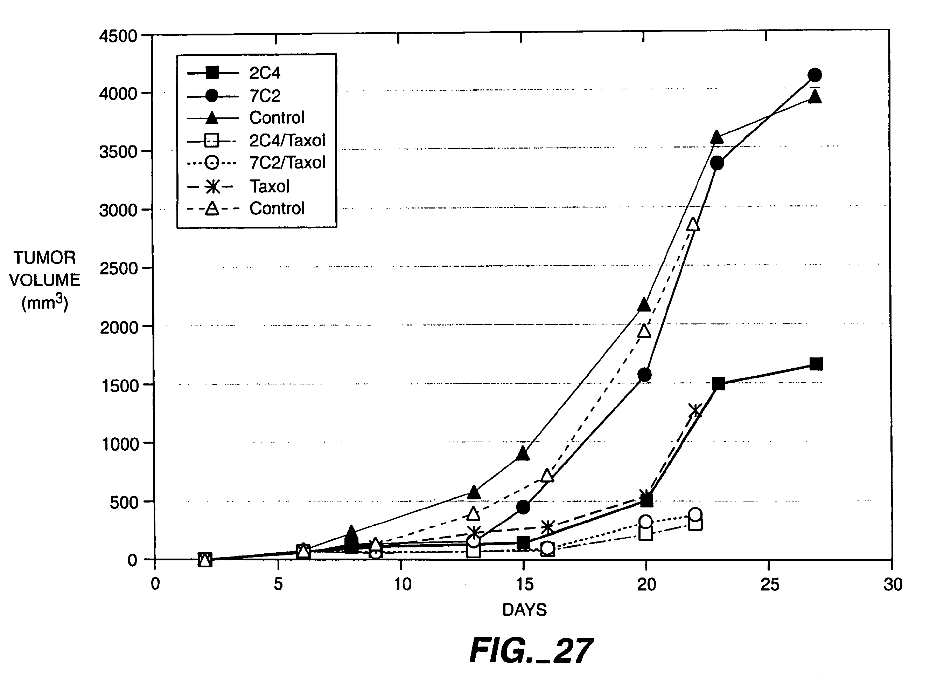

The effect of monoclonal antibody 7C2 (ATCC HB-12215) which induces apoptosis of ErbB2 overexpressing cells was compared to that of monoclonal antibody 2C4 in the androgen dependent CWR22 xenograft. Both antibodies were dosed at 20mg/kg twice per week. As shown in Fig. 26, like 2C4 and HERCEPTIN®, 7C2 is also effective in treating androgen dependent prostate cancer. The effect of 7C2 on androgen independent prostate cancer was also assessed using the CWR22R xenograft. Fig. 27 shows that 7C2 alone was not effective in this model, but was effective when combined with TAXOL®.

## Example 10

### Therapy of Relapsed or Refractory Metastatic Prostate Cancer

RhuMAb 2C4 is a full-length, humanized monoclonal antibody (produced in CHO cells) directed against ErbB2. RhuMAb 2C4 blocks the associated of ErbB2 with other ErbB family members thereby inhibiting intracellular signaling through the ErbB pathway. In contrast to HERCEPTIN®, rhuMAb 2C4 not only inhibits the growth of ErbB2 overexpressing tumors but also blocks growth of tumors that require ErbB ligand-dependent signaling.

RhuMAb 2C4 is indicated as a single agent for treatment of hormone-refractory (androgen independent) prostate cancer patients. Primary endpoints for efficacy include overall survival compared to best available care (Mitoxantrone/Prednisone), when used as a single agent, and safety. Secondary efficacy endpoints include: time to disease progression, response rate, quality of life, pain and/or duration of response. RhuMAb 2C4 is administered intravenously (IV) weekly or every three weeks at 2 or 4 mg/kg, respectively, until disease progression. The antibody is supplied as a multi-dose liquid formulation (20mL fill at a concentration of 20mg/mL or higher concentration).

RhuMAb 2C4 is also indicated in combination with chemotherapy for treatment of hormone-refractory (androgen independent) prostate cancer patients. Primary endpoints for efficacy include overall survival compared to chemotherapy, and safety. Secondary efficacy endpoints include: time to disease progression, response rate, quality of life, pain and/or duration of response. RhuMAb 2C4 is administered intravenously (IV) weekly or every three weeks at 2 or 4 mg/kg, respectively, until disease progression. The antibody is supplied as a multi-dose liquid formulation (20mL fill at a concentration of 20mg/mL or higher concentration).

Examples of drugs that can be combined with the anti-ErbB2 antibody (which blocks ligand activation of an ErbB2 receptor) to treat prostate cancer (*e.g.* androgen independent prostate cancer) include a farnesyl transferase inhibitor; an anti-angiogenic agent (*e.g.* an anti-VEGF antibody); an EGFR-targeted drug *(e.g.* C225 or ZD1839); another anti-ErbB2 antibody (*e.g.* a growth inhibitory anti-ErbB2 antibody such as HERCEPTIN®, or an anti-ErbB2 antibody which induces apoptosis such as 7C2 or 7F3, including humanized and/or affinity matured variants thereof); a cytokine (*e.g.* IL-2, IL-12, G-CSF or GM-CSF); an anti-androgen (such as flutamide or cyproterone acetate); leuprolide; suramin; a chemotherapeutic agent such as vinblastine, estramustine, mitoxantrone, liarozole (a retinoic acid metabolism-blocking agent), cyclophosphamide, anthracycline antibiotics such as doxorubicin, a taxane (*e.g.* paclitaxel or docetaxel), or methotrexate, or any combination of the above, such as vinblastine/estramustine or cyclophosphamide/doxorubicin/methotrexate; prednisone; hydrocortizone; or combinations thereof. Standard doses for these various drugs can be administered, *e.g.* 40 mg/m$^2$/wk docetaxel (TAXOTERE®); 6 (AUC) carboplatin; and 200mg/m$^2$ paclitaxel (TAXOL®).

**Claims**

1. Use of an antibody which binds ErbB2 and blocks ligand activation of an ErbB receptor 50-100% more effectively than humanized monoclonal antibody huMAb4D5-8 as disclosed in US patent 5,821,337 for the manufacture of a medicament for treating prostate cancer in a human, wherein the antibody blocks binding of monoclonal antibody 2C4 obtainable from deposit ATCC HB 12697 to ErbB2.

2. Use according to claim 1 wherein the prostate cancer is androgen independent prostate cancer.

3. Use according to claim 1 wherein the antibody blocks TGF-$\alpha$ activation of mitogen-activated protein kinase (MAPK).

4. Use according to claim 1 wherein the antibody blocks formation of an ErbB hetero-oligomer.

5. Use according to claim 4 wherein the antibody comprises monoclonal antibody 2C4 obtainable from deposit ATCC HB12697 or humanized 2C4.

6. Use according to claim 1 wherein the antibody is an antibody fragment.

7. Use according to claim 6 wherein the antibody fragment is a Fab fragment.

8. Use according to claim 1 wherein the antibody is not conjugated with a cytotoxic agent.

9. Use according to claim 6 wherein the antibody fragment is not conjugated with a cytotoxic agent.

10. Use according to claim 1 wherein the antibody is conjugated with a cytotoxic agent.

48

11. Use according to claim 1 wherein the prostate cancer is androgen dependent prostate cancer.

12. Use according to claim 11 wherein the medicament further comprises a taxane.

13. Use according to claim 11 wherein administering the medicament results in an increased prostate specific antigen (PSA) index in the human.

14. Use according to claim 11 wherein the antibody comprises monoclonal antibody 2C4 obtainable from deposit ATCC HB 12697 or humanized 2C4.

15. Use of a chemotherapeutic agent and of an antibody which binds ErbB2 and blocks ligand activation of an ErbB receptor 50-100% more effectively than humanized monoclonal antibody huMAb4D5-8 as disclosed in US patent 5,821,337 for the manufacture of a medicament for treating prostate cancer in a human, wherein the antibody blocks binding of monoclonal antibody 2C4 obtainable from deposit ATCC HB 12697 to ErbB2.

16. Use according to claim 15 wherein the chemotherapeutic agent is a taxane.

17. An article of manufacture comprising a container and a composition contained therein, wherein the composition comprises an antibody which binds ErbB2 and blocks ligand activation of an ErbB receptor 50-100% more effectively than humanized monoclonal antibody huMAb4D5-8 as disclosed in US patent 5,821,337, and further comprising a package insert indicating that the composition can be used to treat prostate cancer, wherein the antibody blocks binding of monoclonal antibody 2C4 obtainable from deposit ATCC HB 12697 to ErbB2.

18. The article of manufacture of claim 17 wherein the prostate cancer is androgen independent prostate cancer.

19. The article of manufacture of claim 17 wherein the package insert further indicates treating the patient with a chemotherapeutic agent.

20. The article of manufacture of claim 19 wherein the chemotherapeutic agent is a taxane.

21. The article of manufacture according to claim 17 wherein the prostate cancer is androgen dependent prostate cancer.


**Patentansprüche**

1. Verwendung eines Antikörpers, der ErbB2 bindet und Ligandenaktivierung eines ErbB-Rezeptors 50-100 % wirksamer blockiert als ein humanisierter monoklonaler Antikörper huMAb4D5-8, wie im US-Patent 5.821.337 geoffenbart ist, zur Herstellung eines Medikaments zur Behandlung von Prostatakrebs beim Menschen, worin der Antikörper die Bindung eines monoklonalen Antikörpers 2C4, der bei der ATCC unter der Hinterlegungsnummer HB12697 erhältlich ist, an ErbB2 blockiert.

2. Verwendung nach Anspruch 1, worin der Prostatakrebs ein androgenunabhängiger Prostatakrebs ist.

3. Verwendung nach Anspruch 1, worin der Antikörper TGF-α-Aktivierung von mitogenaktivierter Proteinkinase (MAPK) blockiert.

4. Verwendung nach Anspruch 1, worin der Antikörper die Bildung eines ErbB-Heterooligomers blockiert.

5. Verwendung nach Anspruch 4, worin der Antikörper einen monoklonalen Antikörper 2C4, der bei der ATCC unter der Hinterlegungsnummer HB12697 erhältlich ist, oder einen humanisierten 2C4 umfasst.

6. Verwendung nach Anspruch 1, worin der Antikörper ein Antikörperfragment ist.

7. Verwendung nach Anspruch 6, worin das Antikörperfragment ein Fab-Fragment ist.

8. Verwendung nach Anspruch 1, worin der Antikörper nicht mit einem zytotoxischen Mittel konjugiert ist.

9. Verwendung nach Anspruch 6, worin das Antikörperfragment nicht mit einem zytotoxischen Mittel konjugiert ist.

**10.** Verwendung nach Anspruch 1, worin der Antikörper mit einem zytotoxischen Mittel konjugiert ist.

**11.** Verwendung nach Anspruch 1, worin der Prostatakrebs ein androgenabhängiger Prostatakrebs ist.

**12.** Verwendung nach Anspruch 11, worin das Medikament weiters ein Taxan enthält.

**13.** Verwendung nach Anspruch 11, worin die Verabreichung des Medikaments zu einem erhöhten prostataspezfischen Antigen- (PSA-) Index im Menschen führt.

**14.** Verwendung nach Anspruch 11, worin der Antikörper einen monoklonalen Antikörper 2C4, der bei der ATCC unter der Hinterlegungsnummer HB12697 erhältlich ist, oder einen humanisierten 2C4 umfasst.

**15.** Verwendung eines Chemotherapeutikums und eines Antikörpers, der ErbB2 bindet und Ligandenaktivierung eines ErbB-Rezeptors 50-100 % wirksamer blockiert als ein humanisierter monoklonaler Antikörper huMAb4D5-8, wie im US-Patent 5.821.337 geoffenbart ist, zur Herstellung eines Medikaments zur Behandlung von Prostatakrebs beim Menschen, worin der Antikörper die Bindung eines monoklonalen Antikörpers 2C4, der bei der ATCC unter der Hinterlegungsnummer HB12697 erhältlich ist, an ErbB2 blockiert.

**16.** Verwendung nach Anspruch 15, worin das Chemotherapeutikum ein Taxan ist.

**17.** Fabrikat, einen Behälter und eine darin enthaltene Zusammensetzung umfassend, worin die Zusammensetzung einen Antikörper umfasst, der ErbB2 bindet und Ligandenaktivierung eines ErbB-Rezeptors 50-100 % wirksamer blockiert als ein humanisierter monoklonaler Antikörper huMAb4D5-8, wie im US-Patent 5.821.337 geoffenbart ist, und weiters eine Packungsbeilage umfassend, in der angegeben ist, dass die Zusammensetzung zur Behandlung von Prostatakrebs eingesetzt werden kann, worin der Antikörper die Bindung eines monoklonalen Antikörpers 2C4, der bei der ATCC unter der Hinterlegungsnummer HB12697 erhältlich ist, an ErbB2 blockiert.

**18.** Fabrikat nach Anspruch 17, worin der Prostatakrebs ein androgenunabhängiger Prostatakrebs ist.

**19.** Fabrikat nach Anspruch 17, worin die Packungsbeilage weiters die Behandlung des Patienten mit einem Chemotherapeutikum angibt.

**20.** Fabrikat nach Anspruch 19, worin das Chemotherapeutikum ein Taxan ist.

**21.** Fabrikat nach Anspruch 17, worin der Prostatakrebs ein androgenabhängiger Prostatakrebs ist.


**Revendications**

**1.** Utilisation d'un anticorps qui se lie à ErbB2 et qui bloque l'activation par un ligand d'un récepteur de ErbB 50-100 % plus efficacement que l'anticorps monoclonal humanisé huMAb4D5-8 décrit dans le brevet des E.U.A. n° 5 821 337 pour la production d'un médicament destiné au traitement du cancer de la prostate chez un être humain, dans laquelle l'anticorps bloque la liaison de l'anticorps monoclonal 2C4 pouvant être obtenu auprès du dépôt ATCC HB12697 à ErbB2.

**2.** Utilisation suivant la revendication 1, dans laquelle le cancer de la prostate est un cancer de la prostate indépendant des androgènes.

**3.** Utilisation suivant la revendication 1, dans laquelle l'anticorps bloque l'activation par le TGF-$\alpha$ de la protéine-kinase activée par un agent mitogène (MAPK).

**4.** Utilisation suivant la revendication 1, dans laquelle l'anticorps bloque la formation d'un hétéro-oligomère de ErbB.

**5.** Utilisation suivant la revendication 4, dans laquelle l'anticorps comprend l'anticorps monoclonal 2C4 pouvant être obtenu auprès du dépôt ATCC HB12697 ou l'anticorps 2C4 humanisé.

**6.** Utilisation suivant la revendication 1, dans laquelle l'anticorps est un fragment d'anticorps.

7. Utilisation suivant la revendication 6, dans laquelle le fragment d'anticorps est le fragment Fab.

8. Utilisation suivant la revendication 1, dans laquelle l'anticorps n'est pas conjugué avec un agent cytotoxique.

9. Utilisation suivant la revendication 6, dans laquelle le fragment d'anticorps n'est pas conjugué avec un agent cytotoxique.

10. Utilisation suivant la revendication 1, dans laquelle l'anticorps est conjugué avec un agent cytotoxique.

11. Utilisation suivant la revendication 1, dans laquelle le cancer de la prostate est un cancer de la prostate dépendant des androgènes.

12. Utilisation suivant la revendication 11, dans laquelle le médicament comprend en outre un taxane.

13. Utilisation suivant la revendication 11, dans laquelle l'administration du médicament a pour résultat un indice d'antigène spécifique de la prostate (PSA) augmenté chez l'être humain.

14. Utilisation suivant la revendication 11, dans laquelle l'anticorps comprend l'anticorps monoclonal 2C4 pouvant être obtenu auprès du dépôt ATCC HB12697 ou l'anticorps 2C4 humanisé.

15. Utilisation d'un agent chimiothérapeutique et d'un anticorps qui se lie à ErbB2 et qui bloque l'activation par un ligand d'un récepteur de ErbB 50-100 % plus efficacement que l'anticorps monoclonal humanisé huMAb4D5-8 décrit dans le brevet des E.U.A. n° 5 821 337 pour la production d'un médicament destiné au traitement du cancer de la prostate chez un être humain, dans laquelle l'anticorps bloque la liaison de l'anticorps monoclonal 2C4 pouvant être obtenu auprès du dépôt ATCC HB12697 à ErbB2.

16. Utilisation suivant la revendication 15, dans laquelle l'agent chimiothérapeutique est un taxane.

17. Article manufacturé comprenant un récipient et une composition présente dans ce récipient, dans lequel la composition comprend un anticorps qui se lie à ErbB2 et qui bloque l'activation par un ligand d'un récepteur de ErbB 50-100 % plus efficacement que l'anticorps monoclonal humanisé huMAb4D5-8 décrit dans le brevet des E.U.A. n° 5 821 337, et comprenant en outre un encart d'emballage indiquant que la composition peut être utilisée pour traiter le cancer de la prostate, l'anticorps bloquant la liaison de l'anticorps monoclonal 2C4 pouvant être obtenu auprès du dépôt ATCC HB12697 à ErbB2.

18. Article manufacturé suivant la revendication 17, dans lequel le cancer de la prostate est un cancer de la prostate indépendant des androgènes.

19. Article manufacturé suivant la revendication 17, dans lequel l'encart d'emballage indique en outre le traitement du patient avec un agent chimiothérapeutique.

20. Article manufacturé suivant la revendication 19, dans lequel l'agent chimiothérapeutique est un taxane.

21. Article manufacturé suivant la revendication 17, dans lequel le cancer de la prostate est un cancer de la prostate dépendant des androgènes.

1 MELAALCRWG LLLALLPPGA ASTQVCTGTD MKLRLPASPE THLDMLRHLY QGCQVVQGNL ELTYLPTNAS LSFLQDIQEV QGYVLIAHNQ VRQVPLQRLR

101 IVRGTQLFED NYALAVLDNG DPLNNTTPVT GASPGGLREL QLRSLTEILK GGVLIQRNPQ LCYQDTILWK DIFHKNNQLA LTLIDTNRSR ACHPCSPMCK

201 GSRCWGESSE DCQSLTRTVC AGGCARCKGP LPTDCCHEQC AAGCTGPKHS DCLACLHFNH SGICELHCPA LVTYNTDTFE SMPNPEGRYT FGASCVTACP

301 YNYLSTDVGS CTLVCPLHNQ EVTAEDGTQR CEKCSKPCAR VCYGLGMEHL REVRAVTSAN IQEFAGCKKI FGSLAFLPES FDGDPASNTA PLQPEQLQVF

401 ETLEEITGYL YISAWPDSLP DLSVFQNLQV IRGRILHNGA YSLTLQGLGI SWLGLRSLRE LGSGLALIHH NTHLCFVHTV PWDQLFRNPH QALLHTANRP

501 EDECVGEGLA CHQLCARGHC WGPGPTQCVN CSQFLRGQEC VEECRVLQGL PREYVNARHC LPCHPECQPQ NGSVTCFGPE ADQCVACAHY KDPPFCVARC

601 PSGVKPDLSY MPIWKFPDEE GACQPCPINC THSCVDLDDK GCPAE  (SEQ ID NO: 13)

## FIG._1A

| | | | |
|---|---|---|---|
| 7C2 | aa | 22-53 | (31 RESIDUES) |
| 7F3 | aa | 22-53 | (31 RESIDUES) |
| 2C4 | aa | 22-584 | (562 RESIDUES) |
| 7D3 | aa | 22-584 | (562 RESIDUES) |
| 3E8 | aa | 512-625 | (113 RESIDUES) |
| 4D5 | aa | 529-625 | (96 RESIDUES) |
| 2H11 | aa | 529-645 | (116 RESIDUES) |
| 3H4 | aa | 541-599 | (58 RESIDUES) |

FIG._1B

**FIG._2A**

**FIG._2B**

*FIG._3*

EP 1 189 634 B1

FIG._4A

FIG._4B

FIG._5B

SK-BR-3
HIGH HER2

175 –

83 –

HRG: | – | + | – | + | – | + | – | + |

CONTROL | 2C4 | HERCEPTIN® | αEGFR

IP: αHER2
WB: αHER3

FIG._5A

MCF7
LOW / NORMAL HER2

175 –

83 –

HRG: | – | + | – | + | – | + | – | + |

CONTROL | 2C4 | HERCEPTIN® | αEGFR

IP: αHER2
WB: αHER3

FIG._10

| EGF | – | + | + | – | – | – | – | – | – | – |
| TGFα | – | – | – | + | + | + | + | – | – | – |
| HRG | – | – | – | – | – | – | + | + | + | + |
| 2C4 | – | – | + | – | + | – | – | – | + | + |
| HERCEPTIN® | – | + | – | + | – | – | + | – | – | + |

FIG._6A

FIG._6B

VARIABLE LIGHT

```
                  10        20         30         40
2C4     DTVMTQSHKIMSTSVGDRVSITC  [KASQDVSIGVA]  WYQQRP
        **      **** *          *                     *
574     DIQMTQSPSSLSASVGDRVTITC  [KASQDVSIGVA]  WYQQKP
                                  *   ** ***

hum κI  DIQMTQSPSSLSASVGDRVTITC  [RASQSISNYLA]  WYQQKP
```

```
                  50        60         70         80
2C4     GQSPKLLIY  [SASYRYT]  GVPDRFTGSGSGTDFTFTISSVQA
        **                    *   *        *      *  *
574     GKAPKLLIY  [SASYRYT]  GVPSRFSGSGSGTDFTLTISSLQP
         *  *****

hum κI  GKAPKLLIY  [AASSLES]  GVPSRFSGSGSGTDFTLTISSLQP
```

```
                  90        100
2C4     EDLAVYYC  [QQYYIYPYT]  FGGGTKLEIK  (SEQ ID NO:1)
         *  *                  *   *
574     EDFATYYC  [QQYYIYPYT]  FGQGTKVEIK  (SEQ ID NO:3)
                   *** *

hum κI  EDFATYYC  [QQYNSLPWT]  FGQGTKVEIK  (SEQ ID NO:5)
```

# FIG._7A

VARIABLE HEAVY

```
                  10        20         30         40
2C4     EVQLQQSGPELVKPGTSVKISCKAS  [GFTFTDYTMD]  WVKQS
         **   **  *   * ***   *                   *  *
574     EVQLVESGGGLVQPGGSLRLSCAAS  [GFTFTDYTMD]  WVRQA
                                    ** * *
hum III EVQLVESGGGLVQPGGSLRLSCAAS  [GFTFSSYAMS]  WVRQA
```

```
                  50 a      60         70         80
2C4     HGKSLEWIG  [DVNPNSGGSIYNQRFKG]  KASLTVDRSSRIVYM
         *  *   **                      *** *   **** *
574     PGKGLEWVA  [DVNPNSGGSIYNQRFKG]  RFTLSVDRSKNTLYL
                    ****** *** ****       * *
hum III PGKGLEWVA  [VISGDGGSTYYADSVKG]  RFTISRDNSKNTLYL
```

```
                  abc      90        100ab      110
2C4     ELRSLTFEDTAVYYCAR  [NLGPSFYFDY]  WGQGTTLTVSS  (SEQ ID NO:2)
        ***   **                                  **
574     QMNSLRAEDTAVYYCAR  [NLGPSFYFDY]  WGQGTLVTVSS  (SEQ ID NO:4)
                            ********
hum III QMNSLRAEDTAVYYCAR  [GRVGYSLYDY]  WGQGTLVTVSS  (SEQ ID NO:6)
```

# FIG._7B

FIG._8A

FIG._8B

EP 1 189 634 B1

FIG._8C

EP 1 189 634 B1

FIG._9

EP 1 189 634 B1

FIG._11A

FIG._11B

FIG._11C

FIG._11D

**FIG._11E**

**FIG._11F**

**FIG._11G**

**FIG._11H**

DAYS POST TREATMENT

*FIG._12A*

DAYS POST TREATMENT

*FIG._12B*

*FIG._13*

*FIG._14*

**FIG._15**

**FIG._16**

**FIG._17**

**FIG._18**

RELATIVE TGF-α mRNA EXPRESSION

FIG._19

RELATIVE HB-EGF mRNA EXPRESSION

FIG._20

FIG._21

*FIG._22A*

*FIG._22B*

*FIG._23*

EP 1 189 634 B1

FIG._24

EP 1 189 634 B1

FIG._25

FIG._26

FIG._27

EP 1 189 634 B1

Sequence Listing

<110> Genentech, Inc. et al.

<120> TREATING PROSTATE CANCER WITH ANTI-ErbB2 ANTIBODIES

<130> P1760R1PCT

<141> 2000-06-23

<150> US 60/141,315
<151> 1999-06-25

<160> 22

<210> 1
<211> 107
<212> PRT
<213> Mus Musculus

<400> 1

```
        Asp Thr Val Met Thr Gln Ser His Lys Ile Met Ser Thr Ser Val
         1               5                  10                  15

        Gly Asp Arg Val Ser Ile Thr Cys Lys Ala Ser Gln Asp Val Ser
                        20                  25                  30

        Ile Gly Val Ala Trp Tyr Gln Gln Arg Pro Gly Gln Ser Pro Lys
                        35                  40                  45

        Leu Leu Ile Tyr Ser Ala Ser Tyr Arg Tyr Thr Gly Val Pro Asp
                        50                  55                  60

        Arg Phe Thr Gly Ser Gly Ser Gly Thr Asp Phe Thr Phe Thr Ile
                        65                  70                  75

        Ser Ser Val Gln Ala Glu Asp Leu Ala Val Tyr Tyr Cys Gln Gln
                        80                  85                  90

        Tyr Tyr Ile Tyr Pro Tyr Thr Phe Gly Gly Gly Thr Lys Leu Glu
                        95                  100                 105

        Ile Lys
```

<210> 2
<211> 119
<212> PRT
<213> Mus musculus

<400> 2

```
Glu Val Gln Leu Gln Gln Ser Gly Pro Glu Leu Val Lys Pro Gly
 1               5                  10                  15

Thr Ser Val Lys Ile Ser Cys Lys Ala Ser Gly Phe Thr Phe Thr
             20                  25                  30

Asp Tyr Thr Met Asp Trp Val Lys Gln Ser His Gly Lys Ser Leu
             35                  40                  45

Glu Trp Ile Gly Asp Val Asn Pro Asn Ser Gly Gly Ser Ile Tyr
             50                  55                  60

Asn Gln Arg Phe Lys Gly Lys Ala Ser Leu Thr Val Asp Arg Ser
```

```
             65                  70                  75

Ser Arg Ile Val Tyr Met Glu Leu Arg Ser Leu Thr Phe Glu Asp
             80                  85                  90

Thr Ala Val Tyr Tyr Cys Ala Arg Asn Leu Gly Pro Ser Phe Tyr
             95                  100                 105

Phe Asp Tyr Trp Gly Gln Gly Thr Thr Leu Thr Val Ser Ser
             110                 115
```

<210> 3
<211> 107
<212> PRT
<213> artificial

<400> 3

```
Asp Ile Gln Met Thr Gln Ser Pro Ser Ser Leu Ser Ala Ser Val
 1               5                  10                  15

Gly Asp Arg Val Thr Ile Thr Cys Lys Ala Ser Gln Asp Val Ser
             20                  25                  30

Ile Gly Val Ala Trp Tyr Gln Gln Lys Pro Gly Lys Ala Pro Lys
             35                  40                  45

Leu Leu Ile Tyr Ser Ala Ser Tyr Arg Tyr Thr Gly Val Pro Ser
             50                  55                  60

Arg Phe Ser Gly Ser Gly Ser Gly Thr Asp Phe Thr Leu Thr Ile
             65                  70                  75

Ser Ser Leu Gln Pro Glu Asp Phe Ala Thr Tyr Tyr Cys Gln Gln
             80                  85                  90

Tyr Tyr Ile Tyr Pro Tyr Thr Phe Gly Gln Gly Thr Lys Val Glu
             95                  100                 105

Ile Lys
```

<210> 4
<211> 119
<212> PRT

<213> artificial

<220>
<221> artificial
<222> 1-119
<223> Fab 574 VH

<400> 4

```
Glu Val Gln Leu Val Glu Ser Gly Gly Gly Leu Val Gln Pro Gly
 1               5                  10                      15

Gly Ser Leu Arg Leu Ser Cys Ala Ala Ser Gly Phe Thr Phe Thr
                20                  25                      30

Asp Tyr Thr Met Asp Trp Val Arg Gln Ala Pro Gly Lys Gly Leu
                35                  40                      45

Glu Trp Val Ala Asp Val Asn Pro Asn Ser Gly Gly Ser Ile Tyr
                50                  55                      60


Asn Gln Arg Phe Lys Gly Arg Phe Thr Leu Ser Val Asp Arg Ser
                65                  70                      75

Lys Asn Thr Leu Tyr Leu Gln Met Asn Ser Leu Arg Ala Glu Asp
                80                  85                      90

Thr Ala Val Tyr Tyr Cys Ala Arg Asn Leu Gly Pro Ser Phe Tyr
                95                  100                     105

Phe Asp Tyr Trp Gly Gln Gly Thr Leu Val Thr Val Ser Ser
                110                 115
```

<210> 5
<211> 107
<212> PRT
<213> artificial

<400> 5

```
Asp Ile Gln Met Thr Gln Ser Pro Ser Ser Leu Ser Ala Ser Val
1               5                   10                  15

Gly Asp Arg Val Thr Ile Thr Cys Arg Ala Ser Gln Ser Ile Ser
                20                  25                  30

Asn Tyr Leu Ala Trp Tyr Gln Gln Lys Pro Gly Lys Ala Pro Lys
                35                  40                  45

Leu Leu Ile Tyr Ala Ala Ser Ser Leu Glu Ser Gly Val Pro Ser
                50                  55                  60

Arg Phe Ser Gly Ser Gly Ser Gly Thr Asp Phe Thr Leu Thr Ile
                65                  70                  75

Ser Ser Leu Gln Pro Glu Asp Phe Ala Thr Tyr Tyr Cys Gln Gln
                80                  85                  90

Tyr Asn Ser Leu Pro Trp Thr Phe Gly Gln Gly Thr Lys Val Glu
                95                  100                 105

Ile Lys
```

<210> 6
<211> 119
<212> PRT
<213> artificial

<400> 6

```
Glu Val Gln Leu Val Glu Ser Gly Gly Gly Leu Val Gln Pro Gly
1               5                   10                  15

Gly Ser Leu Arg Leu Ser Cys Ala Ala Ser Gly Phe Thr Phe Ser
                20                  25                  30

Ser Tyr Ala Met Ser Trp Val Arg Gln Ala Pro Gly Lys Gly Leu
                35                  40                  45

Glu Trp Val Ala Val Ile Ser Gly Asp Gly Gly Ser Thr Tyr Tyr
                50                  55                  60

Ala Asp Ser Val Lys Gly Arg Phe Thr Ile Ser Arg Asp Asn Ser
                65                  70                  75

Lys Asn Thr Leu Tyr Leu Gln Met Asn Ser Leu Arg Ala Glu Asp
                80                  85                  90

Thr Ala Val Tyr Tyr Cys Ala Arg Gly Arg Val Gly Tyr Ser Leu
                95                  100                 105

Tyr Asp Tyr Trp Gly Gln Gly Thr Leu Val Thr Val Ser Ser
                110                 115
```

<210> 7
<211> 10
<212> PRT

42

<213> Mus musculus

<220>
<221> unsure
<222> 10
<223> unknown amino acid

<400> 7

```
Gly Phe Thr Phe Thr Asp Tyr Thr Met Xaa
1               5               10
```

<210> 8
<211> 17
<212> PRT
<213> Mus musculus

<400> 8

```
Asp Val Asn Pro Asn Ser Gly Gly Ser Ile Tyr Asn Gln Arg Phe
1               5                   10                  15
```

<210> 9
<211> 10
<212> PRT
<213> Mus musculus

<400> 9

```
Asn Leu Gly Pro Ser Phe Tyr Phe Asp Tyr
1               5               10
```

<210> 10
<211> 11
<212> PRT
<213> Mus musculus

<400> 10

```
Lys Ala Ser Gln Asp Val Ser Ile Gly Val Ala
1               5               10
```

<210> 11
<211> 7
<212> PRT
<213> Mus musculus

<220>
<221> unsure
<222> 5-7
<223> unknown amino acid

<400> 11

```
Ser Ala Ser Tyr Xaa Xaa Xaa
                 1               5
```

<210> 12
<211> 9
<212> PRT
<213> Mus musculus

<400> 12

```
Gln Gln Tyr Tyr Ile Tyr Pro Tyr Thr
 1               5
```

<210> 13
<211> 645
<212> PRT
<213> human

<400> 13

```
Met Glu Leu Ala Ala Leu Cys Arg Trp Gly Leu Leu Leu Ala Leu
 1               5                  10                  15

Leu Pro Pro Gly Ala Ala Ser Thr Gln Val Cys Thr Gly Thr Asp
                20              25                  30

Met Lys Leu Arg Leu Pro Ala Ser Pro Glu Thr His Leu Asp Met
                35              40                  45

Leu Arg His Leu Tyr Gln Gly Cys Gln Val Val Gln Gly Asn Leu
                50              55                  60

Glu Leu Thr Tyr Leu Pro Thr Asn Ala Ser Leu Ser Phe Leu Gln
                65              70                  75

Asp Ile Gln Glu Val Gln Gly Tyr Val Leu Ile Ala His Asn Gln
                80              85                  90

Val Arg Gln Val Pro Leu Gln Arg Leu Arg Ile Val Arg Gly Thr
                95              100                 105

Gln Leu Phe Glu Asp Asn Tyr Ala Leu Ala Val Leu Asp Asn Gly
                110             115                 120

Asp Pro Leu Asn Asn Thr Thr Pro Val Thr Gly Ala Ser Pro Gly
                125             130                 135

Gly Leu Arg Glu Leu Gln Leu Arg Ser Leu Thr Glu Ile Leu Lys
                140             145                 150

Gly Gly Val Leu Ile Gln Arg Asn Pro Gln Leu Cys Tyr Gln Asp
                155             160                 165

Thr Ile Leu Trp Lys Asp Ile Phe His Lys Asn Asn Gln Leu Ala
                170             175                 180

Leu Thr Leu Ile Asp Thr Asn Arg Ser Arg Ala Cys His Pro Cys
                185             190                 195

Ser Pro Met Cys Lys Gly Ser Arg Cys Trp Gly Glu Ser Ser Glu
                200             205                 210

Asp Cys Gln Ser Leu Thr Arg Thr Val Cys Ala Gly Gly Cys Ala
                215             220                 225

Arg Cys Lys Gly Pro Leu Pro Thr Asp Cys Cys His Glu Gln Cys
                230             235                 240
```

Ala Ala Gly Cys Thr Gly Pro Lys His Ser Asp Cys Leu Ala Cys
            245               250               255

Leu His Phe Asn His Ser Gly Ile Cys Glu Leu His Cys Pro Ala
            260               265               270

Leu Val Thr Tyr Asn Thr Asp Thr Phe Glu Ser Met Pro Asn Pro
            275               280               285

Glu Gly Arg Tyr Thr Phe Gly Ala Ser Cys Val Thr Ala Cys Pro
            290               295               300

Tyr Asn Tyr Leu Ser Thr Asp Val Gly Ser Cys Thr Leu Val Cys
            305               310               315

Pro Leu His Asn Gln Glu Val Thr Ala Glu Asp Gly Thr Gln Arg
            320               325               330

Cys Glu Lys Cys Ser Lys Pro Cys Ala Arg Val Cys Tyr Gly Leu
            335               340               345

Gly Met Glu His Leu Arg Glu Val Arg Ala Val Thr Ser Ala Asn
            350               355               360

Ile Gln Glu Phe Ala Gly Cys Lys Lys Ile Phe Gly Ser Leu Ala
            365               370               375

Phe Leu Pro Glu Ser Phe Asp Gly Asp Pro Ala Ser Asn Thr Ala
            380               385               390

Pro Leu Gln Pro Glu Gln Leu Gln Val Phe Glu Thr Leu Glu Glu
            395               400               405

Ile Thr Gly Tyr Leu Tyr Ile Ser Ala Trp Pro Asp Ser Leu Pro
            410               415               420

Asp Leu Ser Val Phe Gln Asn Leu Gln Val Ile Arg Gly Arg Ile
            425               430               435

Leu His Asn Gly Ala Tyr Ser Leu Thr Leu Gln Gly Leu Gly Ile
            440               445               450

Ser Trp Leu Gly Leu Arg Ser Leu Arg Glu Leu Gly Ser Gly Leu
            455               460               465

Ala Leu Ile His His Asn Thr His Leu Cys Phe Val His Thr Val
            470               475               480

Pro Trp Asp Gln Leu Phe Arg Asn Pro His Gln Ala Leu Leu His
            485               490               495

Thr Ala Asn Arg Pro Glu Asp Glu Cys Val Gly Glu Gly Leu Ala
            500               505               510

Cys His Gln Leu Cys Ala Arg Gly His Cys Trp Gly Pro Gly Pro
            515               520               525

Thr Gln Cys Val Asn Cys Ser Gln Phe Leu Arg Gly Gln Glu Cys
            530               535               540

Val Glu Glu Cys Arg Val Leu Gln Gly Leu Pro Arg Glu Tyr Val
            545               550               555

Asn Ala Arg His Cys Leu Pro Cys His Pro Glu Cys Gln Pro Gln

46

```
                          560                    565                    570
        Asn Gly Ser Val Thr Cys Phe Gly Pro Glu Ala Asp Gln Cys Val
                          575                    580                    585
        Ala Cys Ala His Tyr Lys Asp Pro Pro Phe Cys Val Ala Arg Cys
                          590                    595                    600
        Pro Ser Gly Val Lys Pro Asp Leu Ser Tyr Met Pro Ile Trp Lys
                          605                    610                    615
        Phe Pro Asp Glu Glu Gly Ala Cys Gln Pro Cys Pro Ile Asn Cys
                          620                    625                    630
        Thr His Ser Cys Val Asp Leu Asp Asp Lys Gly Cys Pro Ala Glu
                          635                    640                    645
```

<210> 14
<211> 18
<212> DNA
<213> artificial

<400> 14
ggacagcact gccagaga          18

<210> 15
<211> 22
<212> DNA
<213> artificial

<400> 15
caggtgatta caggccaagt ag          22

<210> 16
<211> 23
<212> DNA
<213> artificial

<400> 16
cctgggtgtg ccacagacct tca          23

<210> 17
<211> 21
<212> DNA
<213> artificial

<400> 17
tgaagttacc tccaggttgg t          21

<210> 18
<211> 23
<212> DNA
<213> artificial

<400> 18
agacacattc tgtccatttt caa          23

<210> 19

EP 1 189 634 B1

<211> 24
<212> DNA
<213> artificial

<400> 19
caagctgcaa agtgccttgc tcat        24

<210> 20
<211> 21
<212> DNA
<213> artificial

<400> 20
atgtatcaca gcctgtacct g        21

<210> 21
<211> 21
<212> DNA
<213> artificial

<400> 21
ttcttggtct cttcctcctt g        21

<210> 22
<211> 26
<212> DNA
<213> artificial

<400> 22
aggtctaaga ccaaggaagc acgcaa        26